(19) Europäisches Patentamt
European Patent Office
Office européen des brevets

(11) EP 1 828 146 B1

# (12) EUROPÄISCHE PATENTSCHRIFT

(45) Veröffentlichungstag und Bekanntmachung des Hinweises auf die Patenterteilung:
**05.11.2014 Patentblatt 2014/45**

(21) Anmeldenummer: **05821982.5**

(22) Anmeldetag: **20.12.2005**

(51) Int Cl.:
*C07D 239/54* (2006.01) *A61K 31/513* (2006.01)
*C07D 239/36* (2006.01) *C07D 401/06* (2006.01)
*C07D 401/12* (2006.01) *C07D 403/12* (2006.01)
*C07D 405/12* (2006.01) *C07D 409/12* (2006.01)

(86) Internationale Anmeldenummer:
**PCT/EP2005/013737**

(87) Internationale Veröffentlichungsnummer:
**WO 2006/066885 (29.06.2006 Gazette 2006/26)**

## (54) SUBSTITUIERTE N-HETEROCYCLISCHE VERBINDUNGEN UND IHRE VERWENDUNG ALS DOPAMIN D3 REZEPTORLIGANDEN

SUBSTITUTED N-HETEROCYCLIC COMPOUNDS AND THEIR USE AS DOPAMINE D3 RECEPTOR LIGANDS

COMPOSES N-HETEROCYCLIQUES SUBSTITUES ET LEUR UTILISATION COMME LIGANDS DU RECEPTEUR DE LA DOPAMINE D3

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**HR**

(30) Priorität: **21.12.2004 DE 102004061593**

(43) Veröffentlichungstag der Anmeldung:
**05.09.2007 Patentblatt 2007/36**

(73) Patentinhaber: **AbbVie Deutschland GmbH & Co KG**
**65205 Wiesbaden (DE)**

(72) Erfinder:
- **GENESTE, Hervé**
  **67141 Neuhofen (DE)**
- **SAUER, Daryl, R.**
  **Trevor, WI 53179 (US)**

(74) Vertreter: **Reitstötter Kinzebach**
**Patentanwälte**
**Im Zollhof 1**
**67061 Ludwigshafen (DE)**

(56) Entgegenhaltungen:
**EP-A- 0 711 763** **WO-A-00/53596**
**WO-A-91/09594** **WO-A-95/00131**
**WO-A-97/23216** **WO-A-98/08842**
**WO-A-2004/080981** **GB-A- 2 369 616**

- **CHA, MI YOUNG ET AL: "QSAR studies on piperazinylalkylisoxazole analogues selectively acting on dopamine D3 receptor by HQSAR and CoMFA" BIOORGANIC & MEDICINAL CHEMISTRY , 11(7), 1293-1298 CODEN: BMECEP; ISSN: 0968-0896, 2003, XP002373147**
- **DATABASE WPI Section Ch, Week 199202 Derwent Publications Ltd., London, GB; Class B03, AN 1992-013607 XP002373184 -& JP 03 264579 A (DAIICHI PHARM CO LTD) 25. November 1991 (1991-11-25)**
- **GUZIKOWSKI, ANTHONY P. ET AL: "Synthesis of N-Substituted 4-(4-Hydroxyphenyl)piperidines, 4-(4-Hydroxybenzyl)piperidines, and (.+-.)-3-(4-Hydroxyphenyl)pyrrolidines: Selective Antagonists at the 1A/2B NMDA Receptor Subtype" JOURNAL OF MEDICINAL CHEMISTRY , 43(5), 984-994 CODEN: JMCMAR; ISSN: 0022-2623, 2000, XP002373148**
- **ISMAIEL A M ET AL: "KETANSERIN ANALOGUES: THE EFFECT OF STRUCTURAL MODIFICATION ON 5-HT2 SEROTONIN RECEPTOR BINDING" JOURNAL OF MEDICINAL CHEMISTRY, AMERICAN CHEMICAL SOCIETY. WASHINGTON, US, Bd. 38, Nr. 7, 1995, Seiten 1196-1202, XP002166697 ISSN: 0022-2623**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

• BAZIARD-MOUYSSET G ET AL: "Synthesis and structure-activity relationships of novel 2-amino alkyl chromones and related derivatives as sigma site-selective ligands" EUROPEAN JOURNAL OF MEDICINAL CHEMISTRY, EDITIONS SCIENTIFIQUE ELSEVIER, PARIS, FR, Bd. 33, Nr. 5, Juni 1998 (1998-06), Seiten 339-347, XP004127366 ISSN: 0223-5234

## Beschreibung

Die vorliegende Erfindung betrifft neue substituierte N-heterocyclische Verbindungen. Diese Verbindungen besitzen wertvolle therapeutische Eigenschaften und sind insbesondere zur Behandlung von Erkrankungen geeignet, die auf die Modulation des Dopamin-$D_3$-Rezeptors ansprechen.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin. Es liegen gesicherte Erkenntnisse über die Anwesenheit von Dopamin und dessen physiologische Funktion als Neurotransmitter vor. Störungen im dopaminergen Transmittersystem resultieren in Erkrankungen des zentralen Nervensystems, zu denen z. B. Schizophrenie, Depression oder Parkinson-Krankheit zählen. Die Behandlung dieser und anderer Erkrankungen erfolgt mit Arzneimitteln, die mit den Dopaminrezeptoren in Wechselwirkung treten.

Bis 1990 waren zwei Subtypen von Dopaminrezeptoren pharmakologisch klar definiert, nämlich die $D_1$- und $D_2$-Rezeptoren. In jüngerer Zeit wurde ein dritter Subtyp gefunden, nämlich der $D_3$-Rezeptor, der einige Effekte der Antipsychotika und Anti-Parkinsonmittel zu vermitteln scheint (J.C. Schwartz et al., The Dopamine D3 Receptor as a Target for Antipsychotics, in Novel Antipsychotic Drugs, H.Y. Meltzer, Ed. Raven Press, New York 1992, Seiten 135-144; M. Dooley et al., Drugs and Aging 1998, 12, 495-514, J.N. Joyce, Pharmacology and Therapeutics 2001, 90, S. 231-59 "The Dopamine D3 Receptor as a Therapeutic Target for Antipsychotic and Antiparkinsonian Drugs").

Mittlerweile teilt man die Dopamin-Rezeptoren in zwei Familien ein. Einerseits die $D_2$-Gruppe bestehend aus $D_2$-, $D_3$- und $D_4$-Rezeptoren, andererseits die $D_1$-Gruppe bestehend aus $D_1$- und $D_5$-Rezeptoren. Während $D_1$- und $D_2$-Rezeptoren weit verbreitet sind, scheinen $D_3$-Rezeptoren hingegen regioselektiv exprimiert zu werden. So findet man diese Rezeptoren vorzugsweise im limbischen System, den Projektionsbereichen des mesolimbischen Dopaminsystems, vor allem im Nucleus accumbens, aber auch in anderen Bereichen, wie der Amygdala. Wegen dieser vergleichsweise regioselektiven Expression gelten $D_3$-Rezeptoren als nebenwirkungsarmes Target, und es wird angenommen, dass ein selektiver $D_3$-Ligand wohl die Eigenschaften bekannter Antipsychotika, nicht aber ihre Dopamin-$D_2$-Rezeptor-vermittelten neurologischen Nebenwirkungen haben sollte (P. Sokoloff et al., Localization and Function of the D3 Dopamine Receptor, Arzneim. Forsch./Drug Res. 42(1), 224 (1992); P. Sokoloff et al. Molecular Cloning and Characterization of a Novel Dopamine Receptor (D3) as a Target for Neuroleptics, Nature, 347, 146 (1990)).

Pyrimidinverbindungen mit Dopamin-$D_3$-Rezeptoraffinität sind aus der WO 03/002543 und WO 96/02519 bekannt. Diese Verbindungen weisen teilweise hohe Affinitäten zum $D_3$-Rezeptor auf. Sie werden daher zur Behandlung von Erkrankungen des zentralen Nervensystems vorgeschlagen. Die Selektivität bezüglich anderer Rezeptoren ist jedoch nicht zufriedenstellend.

Die PCT/EP04/002609 betrifft Pyrimidinone und die frühere Patentanmeldung DE 102004027359.6 betrifft Pyridinone, die jeweils mit hoher Selektivität an den Dopamin-$D_3$-Rezeptor binden.

Es besteht ein Bedarf nach weiteren Verbindungen, die eine hohe Affinität zum $D_3$-Rezeptor aufweisen und dabei mit hoher Selektivität an diesen Rezeptor binden. Der Erfindung liegt daher die Aufgabe zu Grunde, Verbindungen zur Verfügung zu stellen, die als selektive Dopamin-$D_3$-Rezeptor-Liganden wirken.

Diese Aufgabe wird gelöst durch substituierte N-heterocyclische Verbindungen der allgemeinen Formel (I.A)

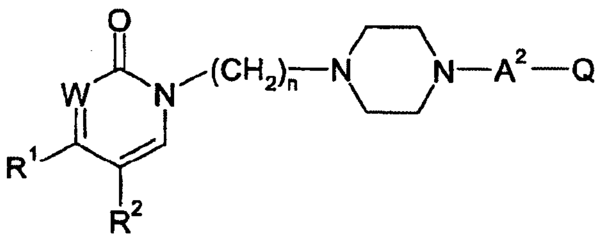

(I), (I.A)

worin

W für CH oder N steht;

$R^1$ für Wasserstoff oder eine Gruppe $R^a$ oder $R^{a1}$ steht;

$R^2$ für Wasserstoff oder eine Gruppe $R^a$ steht;

jedes $R^a$ unabhängig unter CN, $NO_2$, Halogen, $OR^3$, $NR^4R^5$, $C(O)NR^4R^5$, $O-C(O)NR^4R^5$, $SR^6$, $SOR^6$, $SO_2R^6$, $SO_2NR^4R^5$, $COOR^7$, $O-C(O)R^8$, $COR^8$, $C_1$-$C_6$-Alkyl, $C_2$-$C_6$-Alkenyl, $C_2$-$C_6$-Alkinyl, $C_3$-$C_6$-Cycloalkyl ausgewählt sind; wobei $C_1$-$C_6$-Alkyl und $C_2$-$C_6$-Alkenyl gegebenenfalls durch 1, 2 oder 3 Reste substituiert sind, die unabhängig

voneinander unter Halogen, $OR^3$, $NR^4R^5$, $C(O)NR^4R^5$, $O-C(O)NR^4R^5$, $SR^6$, $SOR^6$, $SO_2R^6$, $SO_2NR^4R^5$, $COOR^7$, $O-C(O)R^8$, $COR^8$, $C_3$-$C_6$-Cycloalkyl, Phenyl und 4- bis 6-gliedrigem Heterocyclyl mit 1, 2 oder 3 unter O, S und N ausgewählten Heteroatomen, ausgewählt sind; wobei Phenyl und Heterocyclyl ihrerseits durch ein oder zwei Reste substituiert sein können, die unabhängig voneinander ausgewählt sind unter $C_1$-$C_4$-Alkyl, $C_1$-$C_4$-Alkoxy, $NR^4R^5$, OH, CN, $C_1$-$C_2$-Fluoralkyl und Halogen;

$R^{a1}$ für einen Phenylrest oder einen 4- bis 6-gliedrigen Heterocyclylrest, der 1, 2, 3 oder 4 unabhängig voneinander unter O, S und N ausgewählte Heteroatome als Ringglieder aufweist, steht; wobei der Phenylrest und der Heterocyclylrest gegebenenfalls durch 1, 2, 3 oder 4 unabhängig voneinander ausgewählte Gruppen $R^a$ substituiert ist;

n für 4, 5 oder 6, insbesondere für 4 steht;

$A^2$ für eine 1- bis 2-gliedrige Kohlenwasserstoffkette steht, die 1 oder 2 Methylgruppen als Substituenten aufweisen kann, worin 1 Kohlenstoffatom durch eine Carbonylgruppe ersetzt sein kann;

Q für 5- oder 6-gliedriges Carbocyclyl oder Heterocyclyl mit 1, 2 oder 3 Heteroatomen, ausgewählt unter O, N und S, steht;

wobei Carbocyclyl und Heterocyclyl jeweils vollständig gesättigt, teilweise ungesättigt oder aromatisch sein kann und 1, 2 oder 3 Substituenten aufweisen kann, die unabhängig voneinander ausgewählt sind unter

$C_1$-$C_6$-Alkyl, das gegebenenfalls ein- oder mehrfach durch OH, $C_1$-$C_4$-Alkoxy, Halogen oder Phenyl substituiert ist, das seinerseits 1, 2 oder 3 Substituenten, ausgewählt unter $C_1$-$C_4$-Alkyl, $C_1$-$C_4$-Alkoxy, $NR^4R^5$, OH, CN, $C_1$-$C_2$-Fluoralkyl und Halogen, tragen kann;

$C_2$-$C_6$-Alkenyl, $C_2$-$C_6$-Alkinyl, $C_3$-$C_6$-Cycloalkyl, $C_4$-$C_{10}$-Bicycloalkyl, $C_6$-$C_{10}$-Tricycloalkyl, wobei die fünf letztgenannten Gruppen gegebenenfalls durch Halogen oder $C_1$-$C_4$-Alkyl substituiert sein können;

Halogen, CN, $OR^3$, $NR^4R^5$, $NO_2$, $SR^6$, $SO_2R^6$, $SO_2NR^4R^5$, $COOR^7$, $COR^8$; Phenyl, 5- oder 6-gliedrigem Heterocyclyl mit 1, 2 oder 3 Heteroatomen, ausgewählt unter O, S und N, wobei Phenyl und Heterocyclyl gegebenenfalls 1 oder 2 Substituenten tragen, die unabhängig voneinander ausgewählt sind unter $C_1$-$C_4$-Alkyl, $C_1$-$C_4$-Alkoxy, $NR^4R^5$, OH, CN, $C_1$-$C_2$-Fluoralkyl und Halogen; und wobei 2 an benachbarte C-Atome des 5- oder 6-gliedrigen Carbocyclyls oder Heterocyclyls gebundene Substituenten gemeinsam für $C_3$- oder $C_4$-Alkylen stehen können oder gemeinsam mit den C-Atomen, an die sie gebunden sind, für einen anellierten, ungesättigten 4-, 5- oder 6-gliedrigen Carbocyclus oder für einen 4-, 5- oder 6-gliedrigen Heterocyclus mit 1 oder 2 Heteroatomen, ausgewählt unter O, N und S, als Ringglieder stehen können;

$R^3$, $R^4$, $R^5$, $R^6$, $R^7$, $R^8$ unabhängig voneinander für H, $C_1$-$C_6$-Alkyl, das gegebenenfalls durch OH, $C_1$-$C_4$-Alkoxy oder Phenyl substituiert ist, das seinerseits 1, 2 oder 3 Substituenten, ausgewählt unter $C_1$-$C_4$-Alkyl, $C_1$-$C_4$-Alkoxy, $NR^4R^5$, OH, CN, $C_1$-$C_2$-Fluoralkyl oder Halogen, tragen kann, $COR^{11}$, $C_1$-$C_6$-Halogenalkyl oder Phenyl, das seinerseits 1, 2 oder 3 Substituenten, ausgewählt unter $C_1$-$C_4$-Alkyl, $C_1$-$C_4$-Alkoxy, $NR^4R^5$, OH, CN, $C_1$-$C_2$-Fluoralkyl oder Halogen, tragen kann, stehen; wobei

$R^5$ auch eine Gruppe $COR^9$ bedeuten kann; und wobei

$R^4$ mit $R^5$ auch gemeinsam mit dem Stickstoffatom, an das sie gebunden sind, einen 4-, 5- oder 6-gliedrigen, gesättigten oder ungesättigten Heterocyclus bilden können, der ein weiteres Heteroatom, ausgewählt unter O, S und $NR^{10}$ als Ringglied aufweisen kann, wobei der Heterocyclus unsubstituiert ist oder ein oder zwei $C_1$-$C_4$-Alkylgruppen trägt;

$R^9$ für Wasserstoff, $C_1$-$C_4$-Alkyl oder Phenyl, das gegebenenfalls durch 1, 2 oder 3 Reste substituiert ist, die unabhängig voneinander ausgewählt sind unter $C_1$-$C_4$-Alkyl, $C_1$-$C_4$-Alkoxy, $NR^4R^5$, CN, $C_1$-$C_2$-Fluoralkyl oder Halogen, steht;

$R^{10}$ für Wasserstoff oder $C_1$-$C_4$-Alkyl steht, und

$R^{11}$ für H, $C_1$-$C_6$-Alkyl, das gegebenenfalls durch OH, $C_1$-$C_4$-Alkoxy oder Phenyl substituiert ist, das seinerseits 1, 2 oder 3 Substituenten, ausgewählt unter $C_1$-$C_4$-Alkyl, $C_1$-$C_4$-Alkoxy, $NR^4R^5$, OH, CN, $C_1$-$C_2$-Fluoralkyl oder Halogen, tragen kann, $C_1$-$C_6$-Halogenalkyl oder Phenyl, das seinerseits 1, 2 oder 3 Substituenten, ausgewählt unter $C_1$-$C_4$-Alkyl, $C_1$-$C_4$-Alkoxy, $NR^4R^5$, OH, CN, $C_1$-$C_2$-Fluoralkyl oder Halogen, tragen kann, steht;

sowie durch die Tautomere der Verbindungen I.A, die physiologisch akzeptablen Salze der Verbindungen I.A und die physiologisch akzeptablen Salze der Tautomere der Verbindungen I.A.

Gegenstand der vorliegenden Erfindung sind daher die Verbindungen der allgemeinen Formel I.A, ihre Tautomere sowie die physiologisch verträglichen Salze der Verbindun gen I.A und die physiologisch akzeptablen Salze der Tautomere von I.A.

Gegenstand der vorliegenden Verbindung ist außerdem ein pharmazeutisches Mittel, enthaltend wenigstens eine Pyri(mi)dinon-Verbindung der Formel I.A, deren Tautomere, deren physiologisch akzeptable Säureadditionssalze und/oder die physiologisch akzeptablen Säureadditionssalze der Tautomeren und gegebenenfalls einen oder mehrere physiologisch akzeptable Träger.

Gegenstand der vorliegenden Erfindung ist auch die Verwendung einer substituierten N-heterocyclischen Verbindung der Formel I.A und von deren Tautomeren sowie von deren Salzen oder den Salzen ihrer Tautomeren zur Herstellung eines pharmazeuti schen Mittels zur Behandlung von Erkrankungen, die auf die Modulation durch Dopa min-$D_3$-Rezeptorliganden ansprechen.

Zu den Erkrankungen, die auf die Modulation durch Dopamin-$D_3$-Rezeptorliganden ansprechen, zählen beispielsweise Störungen und Erkrankungen des zentralen Nervensystems, insbesondere Schizophrenie und Depression, Parkinson und Epilepsie, weiterhin Suchterkrankungen sowie Nierenfunktionsstörungen.

Erfindungsgemäß verwendet man zur Behandlung der vorstehend genannten Indikationen wenigstens eine Verbindung der allgemeinen Formel I.A mit den eingangs genannten Bedeutungen. Sofern die Verbindungen der Formel I.A ein oder mehrere Asymmetriezentren aufweisen, können auch Enantiomerengemische, insbesondere Racemate, Diastereomerengemische, Tautomerengemische, vorzugsweise jedoch die jeweiligen im Wesentlichen reinen Enantiomere, Diastereomere und Tautomere eingesetzt werden.

Ebenfalls brauchbar sind physiologisch verträgliche Salze der Verbindungen der Formel I.A, vor allem Säureadditionssalze mit physiologisch verträglichen Säuren. Als physiologisch verträgliche organische und anorganische Säuren kommen beispielsweise Chlorwasserstoffsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in Fortschritte der Arzneimittelforschung, Band 10, Seiten 224 ff., Birkhäuser Verlag, Basel und Stuttgart, 1966, beschrieben.

Halogen steht hier und im folgenden für Fluor, Chlor, Brom oder Iod, insbesondere Fluor oder Chlor.

$C_n$-$C_m$-Alkyl (auch in Resten wie Alkoxy, Alkylthio, Alkylamino etc.) bedeutet eine geradkettige oder verzweigte Alkylgruppe mit n bis m Kohlenstoffatomen, z.B. 1 bis 6 Kohlenstoffatomen und insbesondere 1 bis 4 Kohlenstoffatomen. Beispiele für eine Alkylgruppe sind Methyl, Ethyl, n-Propyl, iso-Propyl, n-Butyl, 2-Butyl, iso-Butyl, tert-Butyl, n-Pentyl, 2-Pentyl, Neopentyl, n-Hexyl und dergleichen.

Die Alkylgruppe kann einen oder mehrere Substituenten aufweisen, die unabhängig voneinander ausgewählt sind unter Halogen, $OR^3$, $NR^4R^5$, $C(O)NR^4R^5$, $O$-$C(O)NR^4R^5$, $SR^6$, $SOR^6$, $SO_2R^6$, $SO_2NR^4R^5$, $COOR^7$, $O$-$C(O)R^8$, $COR^8$, $C_3$-$C_6$-Cycloalkyl, 4-, 5- oder 6-gliedrigem Heterocyclyl mit 1, 2 oder 3 Heteroatomen, ausgewählt unter O, S und N, und Phenyl, wobei Phenyl und Heterocyclyl durch ein oder zwei Reste substituiert sein können, die unabhängig voneinander ausgewählt sind unter $C_1$-$C_4$-Alkyl, $C_1$-$C_4$-Alkoxy, $NR^4R^5$, CN, OH, $C_1$-$C_2$-Fluoralkyl oder Halogen.

Im Falle eines Halogensubstituenten kann die Alkylgruppe insbesondere 1, 2, 3 oder 4 Halogenatome, speziell Fluor oder Chlor, umfassen, die sich an einem oder mehreren C-Atomen befinden können, vorzugsweise in $\alpha$- oder $\omega$-Position. Derartige Gruppen werden im Folgenden auch als Halogenalkyl bezeichnet. Bevorzugtes Halogenalkyl ist $C_1$-$C_2$-Fluoralkyl oder $C_1$-$C_2$-Fluorchloralkyl, insbesondere $CF_3$, $CHF_2$, $CF_2Cl$, $CH_2F$, $CH_2CF_3$.

Im Falle von Hydroxy-substituiertem Alkyl weist die Alkylgruppe insbesondere eine Hydroxy-Gruppe auf, z.B. Hydroxymethyl, 2-Hydroxyeth-1-yl, 2-Hydroxyprop-1-yl, 3-Hydroxyprop-1-yl, 1-Hydroxyprop-2-yl, 2-Hydroxybut-1-yl, 3-Hydroxybut-1-yl, 4-Hydroxybut-1-yl, 1-Hydroxybut-2-yl, 1-Hydroxybut-3-yl, 2-Hydroxybut-3-yl, 1-Hydroxy-2-methylprop-3-yl, 2-Hydroxy-2-methylprop-3-yl oder 2-Hydroxymethylprop-2-yl, insbesondere 2-Hydroxyethyl.

Im Falle von Alkoxy-substituiertem Alkyl weist die Alkylgruppe insbesondere einen Alkoxysubstituenten auf. Diese Reste werden, abhängig von der Anzahl der Kohlenstoffatome, auch als $C_n$-$C_m$-Alkoxy-$C_n$-$C_m$-alkyl bezeichnet und stehen z.B. für Methoxymethyl, Ethoxymethyl, 2-Methoxyethyl, 1-Methoxyethyl, 2-Ethoxyethyl, 1-Ethoxyethyl, n-Propoxymethyl, Isopropoxymethyl, n-Butoxymethyl, (1-Methylpropoxy)methyl, (2-Methylpropoxy)methyl, $CH_2$-$OC(CH_3)_3$, 2-(Methoxy)ethyl, 2-(Ethoxy)ethyl, 2-(n-Prop-oxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)propyl, 2-(1-Methyl-propoxy)propyl, 2-(2-Methylpropoxy)propyl, 2-(1,1-Dimethylethoxy)propyl, 3-(Methoxy)-propyl, 3-(Ethoxy)propyl, 3-(n-Propoxy)propyl, 3-(1-Methylethoxy)propyl, 3-(n-Butoxy)/-propyl, 3-(1-Methylpropoxy)propyl, 3-(2-Methylpropoxy)propyl, 3-(1,1-Dimethylethoxy)-propyl, 2-(Methoxy)butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethyl-ethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, vorzugsweise Methoxymethyl, Ethoxymethyl, 2-Methoxyethyl, 2-Ethoxyethyl, 2-(Methoxy)propyl, 2-(Ethoxy)propyl oder 3-(Methoxy)propyl, 3-(Ethoxy)propyl.

Cycloalkyl steht insbesondere für $C_3$-$C_6$-Cycloalkyl, wie Cyclopropyl, Cyclobutyl, Cyclopentyl und Cyclohexyl.

Der Begriff "Alkylen" umfasst grundsätzlich geradkettige oder verzweigte Reste mit vorzugsweise mit 3 bis 10 und besonders bevorzugt 3 bis 8 Kohlenstoffatomen wie Prop-1,2-ylen, Prop-1,3-ylen, But-1,2-ylen, But-1,3-ylen, But-1,4-ylen, 2-Methylprop-1,3-ylen, Pent-1,2-ylen, Pent-1,3-ylen, Pent-1,4-ylen, Pent-1,5-ylen, Pent-2,3-ylen, Pent-2,4-ylen, 1-Methylbut-1,4-ylen, 2-Methylbut-1,4-ylen, Hex-1,3-ylen, Hex-2,4-ylen, Hex-1,4-ylen, Hex-1,5-ylen, Hex-1,6-ylen und dergleichen. $C_0$-Alkylen steht für eine Einfachbindung, $C_1$-Alkylen für Methylen und $C_2$-Alkylen für 1,1-Ethylen oder 1,2-Ethylen.

Der Begriff "1- bis 2-gliedrige Kohlenwasserstoffkette" umfasst eine Kette mit 1 oder 2 Kohlenstoffatomen mit je einer freien Valenz an den endständigen Atomen der Kohlenwasserstoffkette. Wenn in der 1-bis 2-gliedrigen Kohlenwasserstoffkette ein Kohlenstoffatom durch eine Carbonylgruppe ersetzt ist, so sind Beispiele hierfür -C(O)-, $-CH_2C(O)-$ oder $-C(O)CH_2-$. Die Kohlenwasserstoffkette kann außerdem ein oder zwei Methylgruppen tragen. Beispiele hierfür sind $-C(CH_3)H-$, $-CH(CH_3)CH_2-$, $-CH_2C(CH_3)H-$, $-CH(CH_3)CH(CH_3)_2-$, $-CH(CH_3)C(O)-$, $-C(O)CH(CH_3)-$ und dergleichen. 4-, 5- oder 6-gliedriges Heterocyclyl umfasst sowohl aromatisches Heterocyclyl (Hetaryl bzw. Heteroaryl) als auch vollständig gesättigte oder teilweise ungesättigte heterocyclische Reste. Heterocyclyl weist 1, 2 oder 3 Heteroatome, ausgewählt unter O, S und N auf, z. B. 1, 2 oder 3 Stickstoffatome, 1 oder 2 Sauerstoffatome, oder 1 Sauerstoffatom und 1 oder 2 Stickstoffatome oder 1 Schwefelatom und 1 oder 2 Stickstoffatome.

Heterocyclyl kann unsubstituiert sein oder 1 oder 2 Substituenten ausgewählt unter $C_1$-$C_4$-Alkyl, $C_1$-$C_4$-Alkoxy, OH, CN, $NR^4R^5$, $C_1$-$C_2$-Fluoralkyl und Halogen aufweisen. Außerdem kann Heterocyclyl einen anellierten (kondensierten) 5- oder 6-gliedrigen Carbocyclus, z. B. ein Benzol-, Cyclopentan- oder Cyclohexen-Ring oder einen anellierten Heterocyclus aufweisen, z. B. einen anellierten Pyrrolyl-, Furan-, Thiophen-, Thiazol-, Pyridin-, Pyrimidin- oder Pyridazin-Ring.

Beispiele für gesättigtes Heterocyclyl sind Azetidin-1-yl, Azetidin-2-yl, Azetidin-3-yl, Oxetan-2-yl, Oxetan-3-yl, Thietan-2-yl, Thietan-3-yl, Pyrrolidinyl, Piperidyl, Piperazinyl, Morpholinyl, Oxolanyl, 1,3-Dioxolanyl, 1,3- und 1,4-Dioxanyl, 1,3-Oxothiolanyl, Oxazolidinyl und dergleichen.

Beispiele für 5- oder 6-gliedrige aromatische heterocyclische Reste (5- oder 6-gliedriges aromatisches Heterocyclyl), die 1, 2 oder 3 Heteroatome aufweisen, die ausgewählt sind unter O, S und N, sind vor allem Pyridinyl, Pyrimidinyl, Pyrazinyl, Triazinyl, Imidazolyl, Pyrrolyl, Pyrazolyl, Thienyl, Furyl, Oxazolyl, Thiazolyl, Isoxazolyl, Tetrazolyl, Thiadiazolyl und Triazolyl. Diese können an den Stickstoffatomen sowie an den Kohlenstoffatomen 1 oder 2 der vorgenannten Substituenten aufweisen. Sofern einer der Substituenten Hydroxy ist, können die Reste auch in einer tautomeren Form mit Carbonylgruppe vorliegen. Beispiele für 5- oder 6-gliedrige heterocyclische Reste, die einen anellierten Carbocyclus aufweisen, bzw. mit diesem kondensiert sind, umfassen Benzofuranyl, Benzthienyl, Indolyl, Benzothiazolyl, Benzoxazolyl, Benzimidazolyl, Benzopyrazolyl, 1,3-Benzodioxolyl, Chinolinyl, Isochinolinyl, Chinoxalinyl, Chinazolinyl, Cinnolinyl, sowie entsprechende teilhydrierte Gruppen.

Beispiele für einen anellierten 5 oder 6-gliedrigen Carbocyclus sind Cyclopenten, Cyclopentadien, Cyclohexen, Cyclohexadien und Benzol. Beispiele für einen anellierten 5- oder 6-gliedrigen Heterocyclus mit 1 oder 2 Stickstoffatomen als Ringglieder sind Pyridin, 1,2,3,4-und 1,2,5,6-Tetrahydropyridin, 1,2- und 1,4-Dihydropyridin, Pyrimidin, Pyrazin und Pyridazin.

Sofern es sich bei der Gruppe $A^2$ um Ethylen handelt, befinden sich die Bindungsstellen nicht an dem gleichen Atom, sondern bilden eine zweigliedrige Kette, die den Ring der Gruppe Q von dem N-Atom des zentralen gesättigten N-Heterocyclus der Formel (I.A) durch 3 Bindungen voneinander trennt.

Die Variablen W, $A^2$, $R^1$, $R^2$ und Q weisen unabhängig voneinander vorzugsweise die nachstehend angegebenen Bedeutungen auf:

$$W = N.$$

$A^2$ steht für $CH_2$, $CH_2CH_2$, CO, $CH_2CO$ oder $COCH_2$, insbesondere für $CH_2$.

$R^1$ steht für eine Gruppe Halogen, $OR^3$, $NR^4R^5$, $SR^6$, $C_3$-$C_6$-Cycloalkyl, $C_1$-$C_6$-Alkyl, das gegebenenfalls durch 1, 2 oder 3 unabhängig voneinander ausgewählte Reste OH, $C_1$-$C_4$-Alkoxy, Halogen oder Phenyl substituiert ist, für Phenyl oder 5- oder 6-gliedriges aromatisches Heterocyclyl mit 1, 2 oder 3 Heteroatomen, ausgewählt unter O, S und N, wobei Heterocyclyl und Phenyl durch ein oder zwei Reste substituiert sein können, die unabhängig voneinander ausgewählt sind unter $C_1$-$C_4$-Alkyl, $C_1$-$C_4$-Alkoxy, $NR^4R^5$, OH, CN, $C_1$-$C_2$-Fluoralkyl oder Halogen.
Insbesondere steht $R^1$ für $C_1$-$C_6$-Alkyl, speziell $C_1$-$C_4$-Alkyl, Halogen, gegebenen falls substituiertes Phenyl oder 2-Furyl, $C_1$-$C_2$-Fluoralkyl, insbesondere Trifluormethyl, $C_4$-$C_6$-Cycloalkyl, eine Gruppe $OR^3$, eine Gruppe $SR^6$ oder einen Rest $NR^4R^5$. Hierbei steht $R^3$ insbesondere für Wasserstoff, $C_1$-$C_4$-Alkyl, Phenyl oder Benzyl und speziell für Wasserstoff. $R^4$ steht vorzugsweise für Wasserstoff oder Alkyl. $R^5$ steht vorzugsweise für Wasserstoff, $C_1$-$C_4$-Alkyl, Phenyl oder Benzyl oder bildet zusammen mit dem Stickstoffatom und dem Rest $R^4$ einen 4-, 5- oder 6-gliedrigen

gesättigten Heterocyclus wie Azetidinyl, Pyrrolidinyl, Piperidinyl, Morpholinyl oder Piperazinyl. $R^6$ steht hierbei vorzugsweise für Wasserstoff, $C_1$-$C_4$-Alkyl, Phenyl oder Benzyl und insbesondere für Wasserstoff. Substituiertes Phenyl bedeutet hier, dass der Phenylrest durch ein oder zwei Reste substituiert sein kann, z.B. durch $C_1$-$C_4$-Alkyl, $C_1$-$C_4$-Alkoxy, $NR^4R^5$, OH, CN, $C_1$-$C_2$-Fluoralkyl und/oder Halogen.
In einer besonders bevorzugten Ausführungsform der Erfindung steht $R^1$ für $C_1$-$C_6$-Alkyl, insbesondere $C_1$-$C_4$-Alkyl und speziell Methyl, iso-Propyl oder tert-Butyl, $C_1$-$C_2$-Fluoralkyl, insbesondere $CF_3$, $C_4$-$C_6$-Cycloalkyl, insbesondere Cyclobutyl oder Cyclohexyl, 2-Furyl, Phenyl, das durch ein oder zwei Reste substituiert sein kann, die unabhängig voneinander ausgewählt sind unter $C_1$-$C_4$-Alkyl, $C_1$-$C_4$-Alkoxy, $NR^4R^5$, OH, CN, $C_1$-$C_2$-Fluoralkyl oder Halogen, insbesondere für p-Fluorphenyl, m-Fluorphenyl, o-Fluorphenyl, p-Methylphenyl, m-Methylphenyl, o-Methylphenyl, oder für einen Rest $OR^3$. Hierin hat $R^3$ die zuvor genannten Bedeutungen und steht insbesondere für H, $C_1$-$C_4$-Alkyl, Phenyl oder Benzyl und speziell für H. Hierbei kann der Phenylring in Phenyl sowie in Benzyl durch ein oder zwei Reste substituiert sein, die unabhängig voneinander ausgewählt sind unter $C_1$-$C_4$-Alkyl, $C_1$-$C_4$-Alkoxy, $NR^4R^5$, OH, CN, $C_1$-$C_2$-Fluoralkyl oder Halogen.

$R^2$ ist vorzugsweise ausgewählt unter H, $C_1$-$C_4$-Alkyl, insbesondere Methyl, $C_1$-$C_2$-Fluoralkyl, insbesondere Trifluormethyl, Halogen, insbesondere Fluor, und CN. In einer besonders bevorzugten Ausführungsform steht $R^2$ für $C_1$-$C_4$-Alkyl, speziell für Methyl. In einer anderen besonders bevorzugten Ausführungsform steht $R^2$ für Wasserstoff.

Eine ganz besonders bevorzugte Ausführungsform der Erfindung betrifft Verbindungen der Formel I, worin $R^1$ für $OR^3$ und insbesondere für OH, oder für Methyl, iso-Propyl, tert-Butyl, $CF_3$, Cyclobutyl, Cyclohexyl, Phenyl, p-Fluorphenyl, m-Fluorphenyl, o-Fluorphenyl, p-Methylphenyl, m-Methylphenyl, o-Methylphenyl oder 2-Furyl steht und $R^2$ insbesondere ausgewählt ist unter H, Halogen, CN, $CF_3$ und $C_1$-$C_4$-Alkyl und speziell Wasserstoff, Methyl, Fluor oder Chlor.

Eine weitere ganz besonders bevorzugte Ausführungsform der Erfindung betrifft Verbindungen der Formel I, worin $R^1$ für OH, Phenyl, p-Fluorphenyl, m-Fluorphenyl, o-Fluorphenyl, p-Methylphenyl, m-Methylphenyl, o-Methylphenyl, insbesondere OH steht und $R^2$ insbesondere ausgewählt ist unter H, Fluor, Chlor und $C_1$-$C_4$-Alkyl und speziell für Wasserstoff, Fluor oder Methyl steht.

Q steht vorzugsweise für Phenyl, das gegebenenfalls 1, 2 oder 3 Substituenten $R^Q$ aufweist, die unabhängig voneinander ausgewählt sind unter OH, $C_1$-$C_6$-Alkyl, das gegebenenfalls vollständig oder teilweise durch Halogen substituiert ist, Halogen, Cyano, $C_1$-$C_4$-Alkoxy, $C_1$-$C_2$-Halogenalkoxy, $NR^4R^5$, $C_3$-$C_6$-Cycloalkyl, oder wobei 2 an benachbarte C-Atome des Phenyls gebundene Substituenten gemeinsam mit den C-Atomen, an die sie gebunden sind, für einen anellierten, ungesättigten 4-, 5- oder 6-gliedrigen Carbocyclus oder für einen 4-, 5- oder 6-gliedrigen Heterocyclus mit 1 oder 2 Heteroatomen, ausgewählt unter O, N und S, als Ringglieder stehen. $R^Q$ kann auch für $COOR^7$ stehen.

In einer besonders bevorzugten Ausführungsform steht Q für Phenyl, das 1, 2 oder 3 Substituenten $R^Q$ aufweist, die unabhängig voneinander ausgewählt sind unter Methyl, Ethyl, n-Propyl, iso-Propyl, tert-Butyl, Cyclopropyl, Cyclobutyl, Hydroxyl, Chlor, Fluor, Trifluormethyl, $OCF_3$, $OCHF_2$, CN, Dimethylamino, Methoxy oder Ethoxy.

In dieser Ausführungsform steht Q z.B. für 2-Chlorphenyl, 2-Fluorphenyl, 3-Chlorphenyl, 3-Fluorphenyl, 4-Chlorphenyl, 4-Fluorphenyl, 2-Chlor-6-fluorphenyl, 2-Methylphenyl, 3-Methylphenyl, 4-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 2-Trifluormethoxyphenyl, 3-Trifluormethoxyphenyl, 2-Methoxycarbonylphenyl, 3-Methoxycarbonylphenyl, 4-Methoxycarbonylphenyl, 2-Ethylphenyl, 3-Ethylphenyl, 4-Ethylphenyl, 2-Ethoxyphenyl, 3-Ethoxyphenyl, 4-Ethoxyphenyl, 2-n-Propylphenyl, 3-n-Propylphenyl, 4-n-Propylphenyl, 2-Isopropylphenyl, 3-Isopropylphenyl, 4-Isopropylphenyl, 2-Cyclopropylphenyl, 3-Cyclopropylphenyl, 4-Cyclopropylphenyl, 2-Cyclobutylphenyl, 3-Cyclobutylphenyl, 4-Cyclobutylphenyl, 2-tert-Butylphenyl, 3-tert-Butylphenyl, 4-tert-Butylphenyl, 4-Trifluormethoxyphenyl, 2-Hydroxyphenyl, 3-Hydroxyphenyl, 4-Hydroxyphenyl, 2-Cyanophenyl, 3-Cyanophenyl, 4-Cyanophenyl, 2-Dimethylaminophenyl, 3-Dimethylaminophenyl, 4-Dimethylaminophenyl, 2,4,6-Trimethylphenyl, 2,3,4-Trimethoxyphenyl, 2,4,5-Trimethoxyphenyl,.

Wenn Q für Phenyl steht, weist es insbesondere zwei Substituenten $R^Q$ auf. Die zwei Substituenten $R^Q$ befinden sich dann besonders bevorzugt in 2,3-; 2,4- oder 3,4-Position am Phenylring. Beispiele hierfür sind 2,3-Dichlorphenyl, 2,4-Dichlorphenyl, 3,4-Dichlorphenyl, 2,3-Difluorphenyl, 2,4-Difluorphenyl, 3,4-Difluorphenyl, 2-Chlor-3-fluorphenyl, 2-Chlor-4-fluorphenyl, 3-Chlor-4-fluorphenyl, 3-Chlor-2-fluorphenyl, 4-Chlor-2-fluorphenyl, 4-Chlor-3-fluorphenyl, 2-Chlor-3-methylphenyl, 2-Chlor-4-methylphenyl, 3-Chlor-4-methylphenyl, 3-Chlor-2-methylphenyl, 4-Chlor-2-methylphenyl, 4-Chlor-3-methylphenyl, 2-Chlor-3-methoxyphenyl, 2-Chlor-4-methoxyphenyl, 3-Chlor-4-methoxyphenyl, 3-Chlor-2-methoxyphenyl, 4-Chlor-2-methoxyphenyl, 4-Chlor-3-methoxyphenyl, 2-Chlor-3-trifluormethoxyphenyl, 2-Chlor-4-trifluormethoxyphenyl, 3-Chlor-4-trifluormethoxyphenyl, 3-Chlor-2-trifluormethoxyphenyl, 4-Chlor-2-trifluormethoxyphenyl, 4-Chlor-3-trifluormethoxyphenyl, 2,3-Dimethylphenyl, 2,4-Dimethylphenyl, 3,4-Dimethylphenyl, 2,3-Dimethoxyphenyl, 2,4-Dimethoxyphenyl und 3,4-Dimethoxyphenyl.

In einer weiteren besonders bevorzugten Ausführungsform steht Q für Phenyl, das 1 oder insbesondere 2 unter Halogen, speziell Fluor und Chlor, ausgewählte Substituenten aufweist, wobei vorzugsweise 1 Halogenatom in der para-Position zur Bindungsstelle an $A^2$ angeordnet ist. Insbesondere steht Q bevorzugt für 2,4-Dichlorphenyl oder 3,4-Dichlorphenyl.

In einer anderen Ausführungsform steht Q für Naphthyl oder Phenyl, das einen anellierten 5-gliedrigen Heterocyclus mit 1 oder 2 O-Atomen als Ringglieder trägt. In dieser Ausführungsform steht Q z.B. für Naphth-1-yl, Naphth-2-yl, 2-Methyl-$\alpha$-naphthyl, 1,3-Benzodioxol-4-yl oder 1,3-Benzodioxol-5-yl.

In einer anderen Ausführungsform steht Q für 5- oder 6-gliedriges aromatisches Heterocyclyl mit 1 oder 2 unter O, N und S ausgewählten Heteroatomen, das 1 oder 2 Substituenten aufweisen kann. Die Substituenten sind vorzugsweise unter Chlor und Methyl ausgewählt. In dieser Ausführungsform steht Q z.B. für 2-Pyridinyl, 3-Pyridinyl, 4-Pyridinyl, 4,6-Dimethoxypyrimidin-2-yl, 2-Thienyl, 3-Thienyl, 4-Thienyl, 2-Furyl, 3-Furyl, 4-Furyl, 2-Chlorthiazol-5-yl, 6-Chlorpyridin-2-yl,1-Methylimidazol-2-yl, 2-Methylthiazol-5-yl.

In einer anderen Ausführungsform steht Q für 5- oder 6-gliedriges aromatisches Heterocyclyl mit 1 oder 2 unter O, N und S ausgewählten Heteroatomen, das einen anellierten, ungesättigten sechsgliedrigen Carbocyclus und insbesondere einen anellierten Benzolring trägt. In dieser Ausführungsform steht Q z. B. für Benzimidazol-2-yl, Benzoxazol-2-yl oder Benzothiazol-2-yl.

Im übrigen weisen die Gruppen $R^3$, $R^4$, $R^5$, $R^6$, $R^7$ und $R^8$ vorzugsweise die nachfolgend angegebenen Bedeutungen auf:

$R^3$ steht vorzugsweise für H, $C_1$-$C_4$-Alkyl, phenylsubstituiertes $C_1$-$C_4$-Alkyl oder $COR^{11}$. Hierin weist $R^{11}$ die für $R^8$ angegebenen Bedeutungen auf und steht insbesondere für $C_1$-$C_4$-Alkyl. In Gruppen $NR^3$ steht $R^3$ vorzugsweise für H, $C_1$-$C_4$-Alkyl, phenylsubstituiertes $C_1$-$C_4$-Alkyl oder $COR^{11}$. Insbesondere bevorzugt steht $NR^3$ für NH, $NCH_3$, $NCOCH_3$ oder $NCH_2$-Phenyl. In den Gruppen $C(O)NR^3$ und $NR^3C(O)$ steht $R^3$ vorzugsweise für H, $C_1$-$C_4$-Alkyl, phenylsubstituiertes $C_1$-$C_4$-Alkyl oder $COR^{11}$. Insbesondere bevorzugt steht $C(O)NR^3$ für CONH, $CONCH_3$ oder $CONCH_2$-Phenyl. Insbesondere bevorzugt steht $NR^3C(O)$ für NHCO, $NCH_3CO$ oder $N(CH_2$-Phenyl)CO.

$R^3$ steht vorzugsweise für H, $C_1$-$C_4$-Alkyl, $CF_3$, $CHF_2$ oder Phenyl. Insbesondere bevorzugt steht $OR^3$ für Methoxy, Trifluormethoxy oder Phenoxy.

$R^4$ steht vorzugsweise für Wasserstoff oder $C_1$-$C_4$-Alkyl. $R^5$ steht vorzugsweise für Wasserstoff, $C_1$-$C_4$-Alkyl, Phenyl, Benzyl oder eine Gruppe $COR^{11}$. In Substituenten $CONR^4R^5$ steht $R^4$ vorzugsweise für H oder $C_1$-$C_4$-Alkyl und $R^5$ vorzugsweise für H, $C_1$-$C_4$-Alkyl oder $COR^{11}$. Insbesondere bevorzugt steht $CONR^4R^5$ für $CONH_2$, $CONHCH_3$, $CON(CH_3)_2$ oder $C(O)NHC(O)CH_3$. In Substituenten $NR^4R^5$ steht $R^4$ vorzugsweise für H, $C_1$-$C_4$-Alkyl oder phenylsubstituiertes $C_1$-$C_4$-Alkyl und $R^5$ für H, $C_1$-$C_4$-Alkyl oder $COR^{11}$. Insbesondere bevorzugt steht $NR^4R^5$ für $NH_2$, $NHCH_3$, $N(CH_3)_2$, NH-Benzyl oder $NHCOCH_3$. In Substituenten $SO_2NR^4R^5$ steht $R^4$ vorzugsweise für H oder $C_1$-$C_4$-Alkyl und $R^5$ vorzugsweise für H, $C_1$-$C_4$-Alkyl oder $COR^{11}$. Insbesondere bevorzugt steht $SO_2NR^4R^5$ für Sulfamoyl. In den vorgenannten Gruppen können $R^4$ und $R^5$ auch gemeinsam mit dem Stickstoffatom, an das sie gebunden sind, einen gesättigten oder ungesättigten 4-, 5- oder 6-gliedrigen, vorzugsweise gesättigten Stickstoffheterocyclus bilden, der ein weiteres Heteroatom wie N, S oder O aufweisen kann und der durch 1, 2, 3 oder 4 Alkylgruppen substituiert sein kann. Beispiele für derartige Heterocyclen sind Piperidinyl, Morpholinyl, Pyrrolidinyl, 4-Methylpiperazinyl und 4-Methylpiperidinyl.

$R^6$ steht vorzugsweise für H, $C_1$-$C_4$-Alkyl, Phenyl oder Benzyl. In Substituenten $SR^6$ steht $R^6$ vorzugsweise für H, $C_1$-$C_4$-Alkyl, Phenyl oder Benzyl. In Substituenten $SOR^6$ steht $R^6$ vorzugsweise für Phenyl oder $C_1$-$C_4$-Alkyl. In Substituenten $SO_2R^6$ steht $R^6$ vorzugsweise für H oder $C_1$-$C_4$-Alkyl. Insbesondere bevorzugt steht $SO_2R^6$ für Methylsulfonyl.

In Substituenten $COOR^7$ steht $R^7$ für H oder $C_1$-$C_4$-Alkyl. Insbesondere bevorzugt steht $COOR^7$ für $C_1$-$C_4$-Alkoxycarbonyl, wie Methoxycarbonyl, Ethoxycarbonyl, n-Propoxy-carbonyl, i-Propoxycarbonyl, n-Butoxycarbonyl oder t-Butoxycarbonyl.

In den Substituenten $COR^8$ und $OC(O)R^8$ steht $R^8$ vorzugsweise für H, $C_1$-$C_4$-Alkyl oder Phenyl. Insbesondere bevorzugt steht $COR^8$ für Formyl, Acetyl oder Benzoyl.

Als Tautomere können insbesondere Verbindungen der Formel I.A vorliegen, worin einer oder beide Reste $R^1$ oder $R^2$ für OH oder $NHR^4$ stehen, worin $R^4$ die zuvor genannten Bedeutungen aufweist.

Unter den Verbindungen der allgemeinen Formel I.A sind insbesondere die Verbindungen der allgemeinen Formel I.A-1 bevorzugt

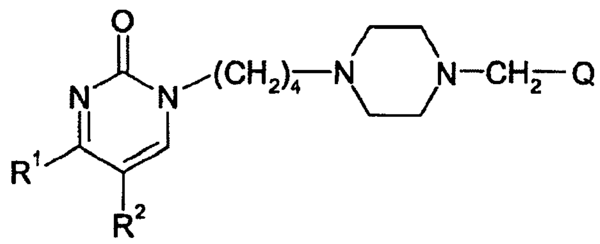

(I.A-1),

worin $R^1$ und $R^2$ die zuvor angegebenen Bedeutungen, insbesondere die als bevorzugt angegebenen Bedeutungen aufweisen; und Q für Phenyl steht, das durch 1 oder 2 Gruppen $R^a$, wie zuvor definiert, die jeweils gleich oder verschieden und auch miteinander verbunden sein können, substituiert ist.

Beispiele für derartige Verbindungen sind die in der nachfolgenden Tabelle 1 aufgeführten Verbindungen I.A-1.1 bis I.A-1.1386, wobei die Variablen $R^1$, $R^2$ und Q jeweils gemeinsam die in einer Zeile der Tabelle 1 angegebene Bedeutung aufweisen, sowie für $R^1$ = OH die Tautomere dieser Verbindungen.

Tabelle 1:

| | $R^1$ | $R^2$ | Q |
|---|---|---|---|
| 1 | Phenyl | H | 2-Chlor-4-fluorphenyl |
| 2 | Phenyl | $CH_3$ | 2-Chlor-4-fluorphenyl |
| 3 | H | $CH_3$ | 2-Chlor-4-fluorphenyl |
| 4 | $CH_3$ | H | 2-Chlor-4-fluorphenyl |
| 5 | OH | H | 2-Chlor-4-fluorphenyl |
| 6 | $C(CH_3)_3$ | H | 2-Chlor-4-fluorphenyl |
| 7 | $CF_3$ | H | 2-Chlor-4-fluorphenyl |
| 8 | $CH(CH_3)_2$ | H | 2-Chlor-4-fluorphenyl |
| 9 | 2-Furyl | H | 2-Chlor-4-fluorphenyl |
| 10 | Cyclohexyl | H | 2-Chlor-4-fluorphenyl |
| 11 | Cyclobutyl | H | 2-Chlor-4-fluorphenyl |
| 12 | 4-Methylphenyl | H | 2-Chlor-4-fluorphenyl |
| 13 | 2-Methylphenyl | H | 2-Chlor-4-fluorphenyl |
| 14 | 2-Fluorphenyl | H | 2-Chlor-4-fluorphenyl |
| 15 | 3-Fluorphenyl | H | 2-Chlor-4-fluorphenyl |
| 16 | 4-Fluorphenyl | H | 2-Chlor-4-fluorphenyl |
| 17 | OH | $CH_3$ | 2-Chlor-4-fluorphenyl |
| 18 | OH | $CF_3$ | 2-Chlor-4-fluorphenyl |
| 19 | OH | F | 2-Chlor-4-fluorphenyl |
| 20 | OH | CN | 2-Chlor-4-fluorphenyl |
| 21 | OH | Cl | 2-Chlor-4-fluorphenyl |
| 22 | OH | $C_2H_5$ | 2-Chlor-4-fluorphenyl |
| 23 | Phenyl | H | 2-Chlor-3-fluorphenyl |
| 24 | Phenyl | $CH_3$ | 2-Chlor-3-fluorphenyl |
| 25 | H | $CH_3$ | 2-Chlor-3-fluorphenyl |
| 26 | $CH_3$ | H | 2-Chlor-3-fluorphenyl |
| 27 | OH | H | 2-Chlor-3-fluorphenyl |

(fortgesetzt)

| | $R^1$ | $R^2$ | Q |
|---|---|---|---|
| 28 | $C(CH_3)_3$ | H | 2-Chlor-3-fluorphenyl |
| 29 | $CF_3$ | H | 2-Chlor-3-fluorphenyl |
| 30 | $CH(CH_3)_2$ | H | 2-Chlor-3-fluorphenyl |
| 31 | 2-Furyl | H | 2-Chlor-3-fluorphenyl |
| 32 | Cyclohexyl | H | 2-Chlor-3-fluorphenyl |
| 33 | Cyclobutyl | H | 2-Chlor-3-fluorphenyl |
| 34 | 4-Methylphenyl | H | 2-Chlor-3-fluorphenyl |
| 35 | 2-Methylphenyl | H | 2-Chlor-3-fluorphenyl |
| 36 | 2-Fluorphenyl | H | 2-Chlor-3-fluorphenyl |
| 37 | 3-Fluorphenyl | H | 2-Chlor-3-fluorphenyl |
| 38 | 4-Fluorphenyl | H | 2-Chlor-3-fluorphenyl |
| 39 | OH | $CH_3$ | 2-Chlor-3-fluorphenyl |
| 40 | OH | $CF_3$ | 2-Chlor-3-fluorphenyl |
| 41 | OH | F | 2-Chlor-3-fluorphenyl |
| 42 | OH | CN | 2-Chlor-3-fluorphenyl |
| 43 | OH | Cl | 2-Chlor-3-fluorphenyl |
| 44 | OH | $C_2H_5$ | 2-Chlor-3-fluorphenyl |
| 45 | Phenyl | H | 3-Chlor-4-fluorphenyl |
| 46 | Phenyl | $CH_3$ | 3-Chlor-4-fluorphenyl |
| 47 | H | $CH_3$ | 3-Chlor-4-fluorphenyl |
| 48 | $CH_3$ | H | 3-Chlor-4-fluorphenyl |
| 49 | OH | H | 3-Chlor-4-fluorphenyl |
| 50 | $C(CH_3)_3$ | H | 3-Chlor-4-fluorphenyl |
| 51 | $CF_3$ | H | 3-Chlor-4-fluorphenyl |
| 52 | $CH(CH_3)_2$ | H | 3-Chlor-4-fluorphenyl |
| 53 | 2-Furyl | H | 3-Chlor-4-fluorphenyl |
| 54 | Cyclohexyl | H | 3-Chlor-4-fluorphenyl |
| 55 | Cyclobutyl | H | 3-Chlor-4-fluorphenyl |
| 56 | 4-Methylphenyl | H | 3-Chlor-4-fluorphenyl |
| 57 | 2-Methylphenyl | H | 3-Chlor-4-fluorphenyl |
| 58 | 2-Fluorphenyl | H | 3-Chlor-4-fluorphenyl |
| 59 | 3-Fluorphenyl | H | 3-Chlor-4-fluorphenyl |
| 60 | 4-Fluorphenyl | H | 3-Chlor-4-fluorphenyl |
| 61 | OH | $CH_3$ | 3-Chlor-4-fluorphenyl |
| 62 | OH | $CF_3$ | 3-Chlor-4-fluorphenyl |
| 63 | OH | F | 3-Chlor-4-fluorphenyl |
| 64 | OH | CN | 3-Chlor-4-fluorphenyl |
| 65 | OH | Cl | 3-Chlor-4-fluorphenyl |

(fortgesetzt)

| | $R^1$ | $R^2$ | Q |
|---|---|---|---|
| 66 | OH | $C_2H_5$ | 3-Chlor-4-fluorphenyl |
| 67 | Phenyl | H | 2,4-Dichlorphenyl |
| 68 | Phenyl | $CH_3$ | 2,4-Dichlorphenyl |
| 69 | H | $CH_3$ | 2,4-Dichlorphenyl |
| 70 | $CH_3$ | H | 2,4-Dichlorphenyl |
| 71 | OH | H | 2,4-Dichlorphenyl |
| 72 | $C(CH_3)_3$ | H | 2,4-Dichlorphenyl |
| 73 | $CF_3$ | H | 2,4-Dichlorphenyl |
| 74 | $CH(CH_3)_2$ | H | 2,4-Dichlorphenyl |
| 75 | 2-Furyl | H | 2,4-Dichlorphenyl |
| 76 | Cyclohexyl | H | 2,4-Dichlorphenyl |
| 77 | Cyclobutyl | H | 2,4-Dichlorphenyl |
| 78 | 4-Methylphenyl | H | 2,4-Dichlorphenyl |
| 79 | 2-Methylphenyl | H | 2,4-Dichlorphenyl |
| 80 | 2-Fluorphenyl | H | 2,4-Dichlorphenyl |
| 81 | 3-Fluorphenyl | H | 2,4-Dichlorphenyl |
| 82 | 4-Fluorphenyl | H | 2,4-Dichlorphenyl |
| 83 | OH | $CH_3$ | 2,4-Dichlorphenyl |
| 84 | OH | $CF_3$ | 2,4-Dichlorphenyl |
| 85 | OH | F | 2,4-Dichlorphenyl |
| 86 | OH | CN | 2,4-Dichlorphenyl |
| 87 | OH | Cl | 2,4-Dichlorphenyl |
| 88 | OH | $C_2H_5$ | 2,4-Dichlorphenyl |
| 89 | Phenyl | H | 3,4-Dichlorphenyl |
| 90 | Phenyl | $CH_3$ | 3,4-Dichlorphenyl |
| 91 | H | $CH_3$ | 3,4-Dichlorphenyl |
| 92 | $CH_3$ | H | 3,4-Dichlorphenyl |
| 93 | OH | H | 3,4-Dichlorphenyl |
| 94 | $C(CH_3)_3$ | H | 3,4-Dichlorphenyl |
| 95 | $CF_3$ | H | 3,4-Dichlorphenyl |
| 96 | $CH(CH_3)_2$ | H | 3,4-Dichlorphenyl |
| 97 | 2-Furyl | H | 3,4-Dichlorphenyl |
| 98 | Cyclohexyl | H | 3,4-Dichlorphenyl |
| 99 | Cyclobutyl | H | 3,4-Dichlorphenyl |
| 100 | 4-Methylphenyl | H | 3,4-Dichlorphenyl |
| 101 | 2-Methylphenyl | H | 3,4-Dichlorphenyl |
| 102 | 2-Fluorphenyl | H | 3,4-Dichlorphenyl |
| 103 | 3-Fluorphenyl | H | 3,4-Dichlorphenyl |

(fortgesetzt)

| | $R^1$ | $R^2$ | Q |
|---|---|---|---|
| 104 | 4-Fluorphenyl | H | 3,4-Dichlorphenyl |
| 105 | OH | $CH_3$ | 3,4-Dichlorphenyl |
| 106 | OH | $CF_3$ | 3,4-Dichlorphenyl |
| 107 | OH | F | 3,4-Dichlorphenyl |
| 108 | OH | CN | 3,4-Dichlorphenyl |
| 109 | OH | Cl | 3,4-Dichlorphenyl |
| 110 | OH | $C_2H_5$ | 3,4-Dichlorphenyl |
| 111 | Phenyl | H | 2,4-Dimethoxyphenyl |
| 112 | Phenyl | $CH_3$ | 2,4-Dimethoxyphenyl |
| 113 | H | $CH_3$ | 2,4-Dimethoxyphenyl |
| 114 | $CH_3$ | H | 2,4-Dimethoxyphenyl |
| 115 | OH | H | 2,4-Dimethoxyphenyl |
| 116 | $C(CH_3)_3$ | H | 2,4-Dimethoxyphenyl |
| 117 | $CF_3$ | H | 2,4-Dimethoxyphenyl |
| 118 | $CH(CH_3)_2$ | H | 2,4-Dimethoxyphenyl |
| 119 | 2-Furyl | H | 2,4-Dimethoxyphenyl |
| 120 | Cyclohexyl | H | 2,4-Dimethoxyphenyl |
| 121 | Cyclobutyl | H | 2,4-Dimethoxyphenyl |
| 122 | 4-Methylphenyl | H | 2,4-Dimethoxyphenyl |
| 123 | 2-Methylphenyl | H | 2,4-Dimethoxyphenyl |
| 124 | 2-Fluorphenyl | H | 2,4-Dimethoxyphenyl |
| 125 | 3-Fluorphenyl | H | 2,4-Dimethoxyphenyl |
| 126 | 4-Fluorphenyl | H | 2,4-Dimethoxyphenyl |
| 127 | OH | $CH_3$ | 2,4-Dimethoxyphenyl |
| 128 | OH | $CF_3$ | 2,4-Dimethoxyphenyl |
| 129 | OH | F | 2,4-Dimethoxyphenyl |
| 130 | OH | CN | 2,4-Dimethoxyphenyl |
| 131 | OH | Cl | 2,4-Dimethoxyphenyl |
| 132 | OH | $C_2H_5$ | 2,4-Dimethoxyphenyl |
| 133 | Phenyl | H | 3,4-Dimethoxyphenyl |
| 134 | Phenyl | $CH_3$ | 3,4-Dimethoxyphenyl |
| 135 | H | $CH_3$ | 3,4-Dimethoxyphenyl |
| 136 | $CH_3$ | H | 3,4-Dimethoxyphenyl |
| 137 | OH | H | 3,4-Dimethoxyphenyl |
| 138 | $C(CH_3)_3$ | H | 3,4-Dimethoxyphenyl |
| 139 | $CF_3$ | H | 3,4-Dimethoxyphenyl |
| 140 | $CH(CH_3)_2$ | H | 3,4-Dimethoxyphenyl |
| 141 | 2-Furyl | H | 3,4-Dimethoxyphenyl |

(fortgesetzt)

| | $R^1$ | $R^2$ | Q |
|---|---|---|---|
| 142 | Cyclohexyl | H | 3,4-Dimethoxyphenyl |
| 143 | Cyclobutyl | H | 3,4-Dimethoxyphenyl |
| 144 | 4-Methylphenyl | H | 3,4-Dimethoxyphenyl |
| 145 | 2-Methylphenyl | H | 3,4-Dimethoxyphenyl |
| 146 | 2-Fluorphenyl | H | 3,4-Dimethoxyphenyl |
| 147 | 3-Fluorphenyl | H | 3,4-Dimethoxyphenyl |
| 148 | 4-Fluorphenyl | H | 3,4-Dimethoxyphenyl |
| 149 | OH | $CH_3$ | 3,4-Dimethoxyphenyl |
| 150 | OH | $CF_3$ | 3,4-Dimethoxyphenyl |
| 151 | OH | F | 3,4-Dimethoxyphenyl |
| 152 | OH | CN | 3,4-Dimethoxyphenyl |
| 153 | OH | Cl | 3,4-Dimethoxyphenyl |
| 154 | OH | $C_2H_5$ | 3,4-Dimethoxyphenyl |
| 155 | Phenyl | H | 2,4-Dimethylphenyl |
| 156 | Phenyl | $CH_3$ | 2,4-Dimethylphenyl |
| 157 | H | $CH_3$ | 2,4-Dimethylphenyl |
| 158 | $CH_3$ | H | 2,4-Dimethylphenyl |
| 159 | OH | H | 2,4-Dimethylphenyl |
| 160 | $C(CH_3)_3$ | H | 2,4-Dimethylphenyl |
| 161 | $CF_3$ | H | 2,4-Dimethylphenyl |
| 162 | $CH(CH_3)_2$ | H | 2,4-Dimethylphenyl |
| 163 | 2-Furyl | H | 2,4-Dimethylphenyl |
| 164 | Cyclohexyl | H | 2,4-Dimethylphenyl |
| 165 | Cyclobutyl | H | 2,4-Dimethylphenyl |
| 166 | 4-Methylphenyl | H | 2,4-Dimethylphenyl |
| 167 | 2-Methylphenyl | H | 2,4-Dimethylphenyl |
| 168 | 2-Fluorphenyl | H | 2,4-Dimethylphenyl |
| 169 | 3-Fluorphenyl | H | 2,4-Dimethylphenyl |
| 170 | 4-Fluorphenyl | H | 2,4-Dimethylphenyl |
| 171 | OH | $CH_3$ | 2,4-Dimethylphenyl |
| 172 | OH | $CF_3$ | 2,4-Dimethylphenyl |
| 173 | OH | F | 2,4-Dimethylphenyl |
| 174 | OH | CN | 2,4-Dimethylphenyl |
| 175 | OH | Cl | 2,4-Dimethylphenyl |
| 176 | OH | $C_2H_5$ | 2,4-Dimethylphenyl |
| 177 | Phenyl | H | 3,4-Dimethylphenyl |
| 178 | Phenyl | $CH_3$ | 3,4-Dimethylphenyl |
| 179 | H | $CH_3$ | 3,4-Dimethylphenyl |

(fortgesetzt)

| | $R^1$ | $R^2$ | Q |
|---|---|---|---|
| 180 | $CH_3$ | H | 3,4-Dimethylphenyl |
| 181 | OH | H | 3,4-Dimethylphenyl |
| 182 | $C(CH_3)_3$ | H | 3,4-Dimethylphenyl |
| 183 | $CF_3$ | H | 3,4-Dimethylphenyl |
| 184 | $CH(CH_3)_2$ | H | 3,4-Dimethylphenyl |
| 185 | 2-Furyl | H | 3,4-Dimethylphenyl |
| 186 | Cyclohexyl | H | 3,4-Dimethylphenyl |
| 187 | Cyclobutyl | H | 3,4-Dimethylphenyl |
| 188 | 4-Methylphenyl | H | 3,4-Dimethylphenyl |
| 189 | 2-Methylphenyl | H | 3,4-Dimethylphenyl |
| 190 | 2-Fluorphenyl | H | 3,4-Dimethylphenyl |
| 191 | 3-Fluorphenyl | H | 3,4-Dimethylphenyl |
| 192 | 4-Fluorphenyl | H | 3,4-Dimethylphenyl |
| 193 | OH | $CH_3$ | 3,4-Dimethylphenyl |
| 194 | OH | $CF_3$ | 3,4-Dimethylphenyl |
| 195 | OH | F | 3,4-Dimethylphenyl |
| 196 | OH | CN | 3,4-Dimethylphenyl |
| 197 | OH | Cl | 3,4-Dimethylphenyl |
| 198 | OH | $C_2H_5$ | 3,4-Dimethylphenyl |
| 199 | Phenyl | H | 2-Chlor-4-methylphenyl |
| 200 | Phenyl | $CH_3$ | 2-Chlor-4-methylphenyl |
| 201 | H | $CH_3$ | 2-Chlor-4-methylphenyl |
| 202 | $CH_3$ | H | 2-Chlor-4-methylphenyl |
| 203 | OH | H | 2-Chlor-4-methylphenyl |
| 204 | $C(CH_3)_3$ | H | 2-Chlor-4-methylphenyl |
| 205 | $CF_3$ | H | 2-Chlor-4-methylphenyl |
| 206 | $CH(CH_3)_2$ | H | 2-Chlor-4-methylphenyl |
| 207 | 2-Furyl | H | 2-Chlor-4-methylphenyl |
| 208 | Cyclohexyl | H | 2-Chlor-4-methylphenyl |
| 209 | Cyclobutyl | H | 2-Chlor-4-methylphenyl |
| 210 | 4-Methylphenyl | H | 2-Chlor-4-methylphenyl |
| 211 | 2-Methylphenyl | H | 2-Chlor-4-methylphenyl |
| 212 | 2-Fluorphenyl | H | 2-Chlor-4-methylphenyl |
| 213 | 3-Fluorphenyl | H | 2-Chlor-4-methylphenyl |
| 214 | 4-Fluorphenyl | H | 2-Chlor-4-methylphenyl |
| 215 | OH | $CH_3$ | 2-Chlor-4-methylphenyl |
| 216 | OH | $CF_3$ | 2-Chlor-4-methylphenyl |
| 217 | OH | F | 2-Chlor-4-methylphenyl |

(fortgesetzt)

| | $R^1$ | $R^2$ | Q |
|---|---|---|---|
| 218 | OH | CN | 2-Chlor-4-methylphenyl |
| 219 | OH | Cl | 2-Chlor-4-methylphenyl |
| 220 | OH | $C_2H_5$ | 2-Chlor-4-methylphenyl |
| 221 | Phenyl | H | 4-Chlor-2-fluorphenyl |
| 222 | Phenyl | $CH_3$ | 4-Chlor-2-fluorphenyl |
| 223 | H | $CH_3$ | 4-Chlor-2-fluorphenyl |
| 224 | $CH_3$ | H | 4-Chlor-2-fluorphenyl |
| 225 | OH | H | 4-Chlor-2-fluorphenyl |
| 226 | $C(CH_3)_3$ | H | 4-Chlor-2-fluorphenyl |
| 227 | $CF_3$ | H | 4-Chlor-2-fluorphenyl |
| 228 | $CH(CH_3)_2$ | H | 4-Chlor-2-fluorphenyl |
| 229 | 2-Furyl | H | 4-Chlor-2-fluorphenyl |
| 230 | Cyclohexyl | H | 4-Chlor-2-fluorphenyl |
| 231 | Cyclobutyl | H | 4-Chlor-2-fluorphenyl |
| 232 | 4-Methylphenyl | H | 4-Chlor-2-fluorphenyl |
| 233 | 2-Methylphenyl | H | 4-Chlor-2-fluorphenyl |
| 234 | 2-Fluorphenyl | H | 4-Chlor-2-fluorphenyl |
| 235 | 3-Fluorphenyl | H | 4-Chlor-2-fluorphenyl |
| 236 | 4-Fluorphenyl | H | 4-Chlor-2-fluorphenyl |
| 237 | OH | $CH_3$ | 4-Chlor-2-fluorphenyl |
| 238 | OH | $CF_3$ | 4-Chlor-2-fluorphenyl |
| 239 | OH | F | 4-Chlor-2-fluorphenyl |
| 240 | OH | CN | 4-Chlor-2-fluorphenyl |
| 241 | OH | Cl | 4-Chlor-2-fluorphenyl |
| 242 | OH | $C_2H_5$ | 4-Chlor-2-fluorphenyl |
| 243 | Phenyl | H | 4-Chlor-3-fluorphenyl |
| 244 | Phenyl | $CH_3$ | 4-Chlor-3-fluorphenyl |
| 245 | H | $CH_3$ | 4-Chlor-3-fluorphenyl |
| 246 | $CH_3$ | H | 4-Chlor-3-fluorphenyl |
| 247 | OH | H | 4-Chlor-3-fluorphenyl |
| 248 | $C(CH_3)_3$ | H | 4-Chlor-3-fluorphenyl |
| 249 | $CF_3$ | H | 4-Chlor-3-fluorphenyl |
| 250 | $CH(CH_3)_2$ | H | 4-Chlor-3-fluorphenyl |
| 251 | 2-Furyl | H | 4-Chlor-3-fluorphenyl |
| 252 | Cyclohexyl | H | 4-Chlor-3-fluorphenyl |
| 253 | Cyclobutyl | H | 4-Chlor-3-fluorphenyl |
| 254 | 4-Methylphenyl | H | 4-Chlor-3-fluorphenyl |
| 255 | 2-Methylphenyl | H | 4-Chlor-3-fluorphenyl |

(fortgesetzt)

| | $R^1$ | $R^2$ | Q |
|---|---|---|---|
| 256 | 2-Fluorphenyl | H | 4-Chlor-3-fluorphenyl |
| 257 | 3-Fluorphenyl | H | 4-Chlor-3-fluorphenyl |
| 258 | 4-Fluorphenyl | H | 4-Chlor-3-fluorphenyl |
| 259 | OH | $CH_3$ | 4-Chlor-3-fluorphenyl |
| 260 | OH | $CF_3$ | 4-Chlor-3-fluorphenyl |
| 261 | OH | F | 4-Chlor-3-fluorphenyl |
| 262 | OH | CN | 4-Chlor-3-fluorphenyl |
| 263 | OH | Cl | 4-Chlor-3-fluorphenyl |
| 264 | OH | $C_2H_5$ | 4-Chlor-3-fluorphenyl |
| 265 | Phenyl | H | 3-Chlor-2-fluorphenyl |
| 266 | Phenyl | $CH_3$ | 3-Chlor-2-fluorphenyl |
| 267 | H | $CH_3$ | 3-Chlor-2-fluorphenyl |
| 268 | $CH_3$ | H | 3-Chlor-2-fluorphenyl |
| 269 | OH | H | 3-Chlor-2-fluorphenyl |
| 270 | $C(CH_3)_3$ | H | 3-Chlor-2-fluorphenyl |
| 271 | $CF_3$ | H | 3-Chlor-2-fluorphenyl |
| 272 | $CH(CH_3)_2$ | H | 3-Chlor-2-fluorphenyl |
| 273 | 2-Furyl | H | 3-Chlor-2-fluorphenyl |
| 274 | Cyclohexyl | H | 3-Chlor-2-fluorphenyl |
| 275 | Cyclobutyl | H | 3-Chlor-2-fluorphenyl |
| 276 | 4-Methylphenyl | H | 3-Chlor-2-fluorphenyl |
| 277 | 2-Methylphenyl | H | 3-Chlor-2-fluorphenyl |
| 278 | 2-Fluorphenyl | H | 3-Chlor-2-fluorphenyl |
| 279 | 3-Fluorphenyl | H | 3-Chlor-2-fluorphenyl |
| 280 | 4-Fluorphenyl | H | 3-Chlor-2-fluorphenyl |
| 281 | OH | $CH_3$ | 3-Chlor-2-fluorphenyl |
| 282 | OH | $CF_3$ | 3-Chlor-2-fluorphenyl |
| 283 | OH | F | 3-Chlor-2-fluorphenyl |
| 284 | OH | CN | 3-Chlor-2-fluorphenyl |
| 285 | OH | Cl | 3-Chlor-2-fluorphenyl |
| 286 | OH | $C_2H_5$ | 3-Chlor-2-fluorphenyl |
| 287 | Phenyl | H | 2-Chlorphenyl |
| 288 | Phenyl | $CH_3$ | 2-Chlorphenyl |
| 289 | H | $CH_3$ | 2-Chlorphenyl |
| 290 | $CH_3$ | H | 2-Chlorphenyl |
| 291 | OH | H | 2-Chlorphenyl |
| 292 | $C(CH_3)_3$ | H | 2-Chlorphenyl |
| 293 | $CF_3$ | H | 2-Chlorphenyl |

(fortgesetzt)

| | $R^1$ | $R^2$ | Q |
|---|---|---|---|
| 294 | $CH(CH_3)_2$ | H | 2-Chlorphenyl |
| 295 | 2-Furyl | H | 2-Chlorphenyl |
| 296 | Cyclohexyl | H | 2-Chlorphenyl |
| 297 | Cyclobutyl | H | 2-Chlorphenyl |
| 298 | 4-Methylphenyl | H | 2-Chlorphenyl |
| 299 | 2-Methylphenyl | H | 2-Chlorphenyl |
| 300 | 2-Fluorphenyl | H | 2-Chlorphenyl |
| 301 | 3-Fluorphenyl | H | 2-Chlorphenyl |
| 302 | 4-Fluorphenyl | H | 2-Chlorphenyl |
| 303 | OH | $CH_3$ | 2-Chlorphenyl |
| 304 | OH | $CF_3$ | 2-Chlorphenyl |
| 305 | OH | F | 2-Chlorphenyl |
| 306 | OH | CN | 2-Chlorphenyl |
| 307 | OH | Cl | 2-Chlorphenyl |
| 308 | OH | $C_2H_5$ | 2-Chlorphenyl |
| 309 | Phenyl | H | 4-Chlorphenyl |
| 310 | Phenyl | $CH_3$ | 4-Chlorphenyl |
| 311 | H | $CH_3$ | 4-Chlorphenyl |
| 312 | $CH_3$ | H | 4-Chlorphenyl |
| 313 | OH | H | 4-Chlorphenyl |
| 314 | $C(CH_3)_3$ | H | 4-Chlorphenyl |
| 315 | $CF_3$ | H | 4-Chlorphenyl |
| 316 | $CH(CH_3)_2$ | H | 4-Chlorphenyl |
| 317 | 2-Furyl | H | 4-Chlorphenyl |
| 318 | Cyclohexyl | H | 4-Chlorphenyl |
| 319 | Cyclobutyl | H | 4-Chlorphenyl |
| 320 | 4-Methylphenyl | H | 4-Chlorphenyl |
| 321 | 2-Methylphenyl | H | 4-Chlorphenyl |
| 322 | 2-Fluorphenyl | H | 4-Chlorphenyl |
| 323 | 3-Fluorphenyl | H | 4-Chlorphenyl |
| 324 | 4-Fluorphenyl | H | 4-Chlorphenyl |
| 325 | OH | $CH_3$ | 4-Chlorphenyl |
| 326 | OH | $CF_3$ | 4-Chlorphenyl |
| 327 | OH | F | 4-Chlorphenyl |
| 328 | OH | CN | 4-Chlorphenyl |
| 329 | OH | Cl | 4-Chlorphenyl |
| 330 | OH | $C_2H_5$ | 4-Chlorphenyl |
| 331 | Phenyl | H | 3-Chlorphenyl |

(fortgesetzt)

| | $R^1$ | $R^2$ | Q |
|---|---|---|---|
| 332 | Phenyl | $CH_3$ | 3-Chlorphenyl |
| 333 | H | $CH_3$ | 3-Chlorphenyl |
| 334 | $CH_3$ | H | 3-Chlorphenyl |
| 335 | OH | H | 3-Chlorphenyl |
| 336 | $C(CH_3)_3$ | H | 3-Chlorphenyl |
| 337 | $CF_3$ | H | 3-Chlorphenyl |
| 338 | $CH(CH_3)_2$ | H | 3-Chlorphenyl |
| 339 | 2-Furyl | H | 3-Chlorphenyl |
| 340 | Cyclohexyl | H | 3-Chlorphenyl |
| 341 | Cyclobutyl | H | 3-Chlorphenyl |
| 342 | 4-Methylphenyl | H | 3-Chlorphenyl |
| 343 | 2-Methylphenyl | H | 3-Chlorphenyl |
| 344 | 2-Fluorphenyl | H | 3-Chlorphenyl |
| 345 | 3-Fluorphenyl | H | 3-Chlorphenyl |
| 346 | 4-Fluorphenyl | H | 3-Chlorphenyl |
| 347 | OH | $CH_3$ | 3-Chlorphenyl |
| 348 | OH | $CF_3$ | 3-Chlorphenyl |
| 349 | OH | F | 3-Chlorphenyl |
| 350 | OH | CN | 3-Chlorphenyl |
| 351 | OH | Cl | 3-Chlorphenyl |
| 352 | OH | $C_2H_5$ | 3-Chlorphenyl |
| 353 | Phenyl | H | 4-Fluorphenyl |
| 354 | Phenyl | $CH_3$ | 4-Fluorphenyl |
| 355 | H | $CH_3$ | 4-Fluorphenyl |
| 356 | $CH_3$ | H | 4-Fluorphenyl |
| 357 | OH | H | 4-Fluorphenyl |
| 358 | $C(CH_3)_3$ | H | 4-Fluorphenyl |
| 359 | $CF_3$ | H | 4-Fluorphenyl |
| 360 | $CH(CH_3)_2$ | H | 4-Fluorphenyl |
| 361 | 2-Furyl | H | 4-Fluorphenyl |
| 362 | Cyclohexyl | H | 4-Fluorphenyl |
| 363 | Cyclobutyl | H | 4-Fluorphenyl |
| 364 | 4-Methylphenyl | H | 4-Fluorphenyl |
| 365 | 2-Methylphenyl | H | 4-Fluorphenyl |
| 366 | 2-Fluorphenyl | H | 4-Fluorphenyl |
| 367 | 3-Fluorphenyl | H | 4-Fluorphenyl |
| 368 | 4-Fluorphenyl | H | 4-Fluorphenyl |
| 369 | OH | $CH_3$ | 4-Fluorphenyl |

(fortgesetzt)

| | $R^1$ | $R^2$ | Q |
|---|---|---|---|
| 370 | OH | $CF_3$ | 4-Fluorphenyl |
| 371 | OH | F | 4-Fluorphenyl |
| 372 | OH | CN | 4-Fluorphenyl |
| 373 | OH | Cl | 4-Fluorphenyl |
| 374 | OH | $C_2H_5$ | 4-Fluorphenyl |
| 375 | Phenyl | H | 2-Fluorphenyl |
| 376 | Phenyl | $CH_3$ | 2-Fluorphenyl |
| 377 | H | $CH_3$ | 2-Fluorphenyl |
| 378 | $CH_3$ | H | 2-Fluorphenyl |
| 379 | OH | H | 2-Fluorphenyl |
| 380 | $C(CH_3)_3$ | H | 2-Fluorphenyl |
| 381 | $CF_3$ | H | 2-Fluorphenyl |
| 382 | $CH(CH_3)_2$ | H | 2-Fluorphenyl |
| 383 | 2-Furyl | H | 2-Fluorphenyl |
| 384 | Cyclohexyl | H | 2-Fluorphenyl |
| 385 | Cyclobutyl | H | 2-Fluorphenyl |
| 386 | 4-Methylphenyl | H | 2-Fluorphenyl |
| 387 | 2-Methylphenyl | H | 2-Fluorphenyl |
| 388 | 2-Fluorphenyl | H | 2-Fluorphenyl |
| 389 | 3-Fluorphenyl | H | 2-Fluorphenyl |
| 390 | 4-Fluorphenyl | H | 2-Fluorphenyl |
| 391 | OH | $CH_3$ | 2-Fluorphenyl |
| 392 | OH | $CF_3$ | 2-Fluorphenyl |
| 393 | OH | F | 2-Fluorphenyl |
| 394 | OH | CN | 2-Fluorphenyl |
| 395 | OH | Cl | 2-Fluorphenyl |
| 396 | OH | $C_2H_5$ | 2-Fluorphenyl |
| 397 | Phenyl | H | 3-Fluorphenyl |
| 398 | Phenyl | $CH_3$ | 3-Fluorphenyl |
| 399 | H | $CH_3$ | 3-Fluorphenyl |
| 400 | $CH_3$ | H | 3-Fluorphenyl |
| 401 | OH | H | 3-Fluorphenyl |
| 402 | $C(CH_3)_3$ | H | 3-Fluorphenyl |
| 403 | $CF_3$ | H | 3-Fluorphenyl |
| 404 | $CH(CH_3)_2$ | H | 3-Fluorphenyl |
| 405 | 2-Furyl | H | 3-Fluorphenyl |
| 406 | Cyclohexyl | H | 3-Fluorphenyl |
| 407 | Cyclobutyl | H | 3-Fluorphenyl |

(fortgesetzt)

| | $R^1$ | $R^2$ | Q |
|---|---|---|---|
| 408 | 4-Methylphenyl | H | 3-Fluorphenyl |
| 409 | 2-Methylphenyl | H | 3-Fluorphenyl |
| 410 | 2-Fluorphenyl | H | 3-Fluorphenyl |
| 411 | 3-Fluorphenyl | H | 3-Fluorphenyl |
| 412 | 4-Fluorphenyl | H | 3-Fluorphenyl |
| 413 | OH | $CH_3$ | 3-Fluorphenyl |
| 414 | OH | $CF_3$ | 3-Fluorphenyl |
| 415 | OH | F | 3-Fluorphenyl |
| 416 | OH | CN | 3-Fluorphenyl |
| 417 | OH | Cl | 3-Fluorphenyl |
| 418 | OH | $C_2H_5$ | 3-Fluorphenyl |
| 419 | Phenyl | H | 2-Chlor-3-methylphenyl |
| 420 | Phenyl | $CH_3$ | 2-Chlor-3-methylphenyl |
| 421 | H | $CH_3$ | 2-Chlor-3-methylphenyl |
| 422 | $CH_3$ | H | 2-Chlor-3-methylphenyl |
| 423 | OH | H | 2-Chlor-3-methylphenyl |
| 424 | $C(CH_3)_3$ | H | 2-Chlor-3-methylphenyl |
| 425 | $CF_3$ | H | 2-Chlor-3-methylphenyl |
| 426 | $CH(CH_3)_2$ | H | 2-Chlor-3-methylphenyl |
| 427 | 2-Furyl | H | 2-Chlor-3-methylphenyl |
| 428 | Cyclohexyl | H | 2-Chlor-3-methylphenyl |
| 429 | Cyclobutyl | H | 2-Chlor-3-methylphenyl |
| 430 | 4-Methylphenyl | H | 2-Chlor-3-methylphenyl |
| 431 | 2-Methylphenyl | H | 2-Chlor-3-methylphenyl |
| 432 | 2-Fluorphenyl | H | 2-Chlor-3-methylphenyl |
| 433 | 3-Fluorphenyl | H | 2-Chlor-3-methylphenyl |
| 434 | 4-Fluorphenyl | H | 2-Chlor-3-methylphenyl |
| 435 | OH | $CH_3$ | 2-Chlor-3-methylphenyl |
| 436 | OH | $CF_3$ | 2-Chlor-3-methylphenyl |
| 437 | OH | F | 2-Chlor-3-methylphenyl |
| 438 | OH | CN | 2-Chlor-3-methylphenyl |
| 439 | OH | Cl | 2-Chlor-3-methylphenyl |
| 440 | OH | $C_2H_5$ | 2-Chlor-3-methylphenyl |
| 441 | Phenyl | H | 3-Chlor-4-methylphenyl |
| 442 | Phenyl | $CH_3$ | 3-Chlor-4-methylphenyl |
| 443 | H | $CH_3$ | 3-Chlor-4-methylphenyl |
| 444 | $CH_3$ | H | 3-Chlor-4-methylphenyl |
| 445 | OH | H | 3-Chlor-4-methylphenyl |

(fortgesetzt)

| | $R^1$ | $R^2$ | Q |
|---|---|---|---|
| 446 | $C(CH_3)_3$ | H | 3-Chlor-4-methylphenyl |
| 447 | $CF_3$ | H | 3-Chlor-4-methylphenyl |
| 448 | $CH(CH_3)_2$ | H | 3-Chlor-4-methylphenyl |
| 449 | 2-Furyl | H | 3-Chlor-4-methylphenyl |
| 450 | Cyclohexyl | H | 3-Chlor-4-methylphenyl |
| 451 | Cyclobutyl | H | 3-Chlor-4-methylphenyl |
| 452 | 4-Methylphenyl | H | 3-Chlor-4-methylphenyl |
| 453 | 2-Methylphenyl | H | 3-Chlor-4-methylphenyl |
| 454 | 2-Fluorphenyl | H | 3-Chlor-4-methylphenyl |
| 455 | 3-Fluorphenyl | H | 3-Chlor-4-methylphenyl |
| 456 | 4-Fluorphenyl | H | 3-Chlor-4-methylphenyl |
| 457 | OH | $CH_3$ | 3-Chlor-4-methylphenyl |
| 458 | OH | $CF_3$ | 3-Chlor-4-methylphenyl |
| 459 | OH | F | 3-Chlor-4-methylphenyl |
| 460 | OH | CN | 3-Chlor-4-methylphenyl |
| 461 | OH | Cl | 3-Chlor-4-methylphenyl |
| 462 | OH | $C_2H_5$ | 3-Chlor-4-methylphenyl |
| 463 | Phenyl | $CH_3$ | 3-Chlor-2-methylphenyl |
| 464 | Phenyl | $CH_3$ | 3-Chlor-2-methylphenyl |
| 465 | H | $CH_3$ | 3-Chlor-2-methylphenyl |
| 466 | $CH_3$ | H | 3-Chlor-2-methylphenyl |
| 467 | OH | H | 3-Chlor-2-methylphenyl |
| 468 | $C(CH_3)_3$ | H | 3-Chlor-2-methylphenyl |
| 469 | $CF_3$ | H | 3-Chlor-2-methylphenyl |
| 470 | $CH(CH_3)_2$ | H | 3-Chlor-2-methylphenyl |
| 471 | 2-Furyl | H | 3-Chlor-2-methylphenyl |
| 472 | Cyclohexyl | H | 3-Chlor-2-methylphenyl |
| 473 | Cyclobutyl | H | 3-Chlor-2-methylphenyl |
| 474 | 4-Methylphenyl | H | 3-Chlor-2-methylphenyl |
| 475 | 2-Methylphenyl | H | 3-Chlor-2-methylphenyl |
| 476 | 2-Fluorphenyl | H | 3-Chlor-2-methylphenyl |
| 477 | 3-Fluorphenyl | H | 3-Chlor-2-methylphenyl |
| 478 | 4-Fluorphenyl | H | 3-Chlor-2-methylphenyl |
| 479 | OH | $CH_3$ | 3-Chlor-2-methylphenyl |
| 480 | OH | $CF_3$ | 3-Chlor-2-methylphenyl |
| 481 | OH | F | 3-Chlor-2-methylphenyl |
| 482 | OH | CN | 3-Chlor-2-methylphenyl |
| 483 | OH | Cl | 3-Chlor-2-methylphenyl |

(fortgesetzt)

| | $R^1$ | $R^2$ | Q |
|---|---|---|---|
| 484 | OH | $C_2H_5$ | 3-Chlor-2-methylphenyl |
| 485 | Phenyl | H | 4-Chlor-2-methylphenyl |
| 486 | Phenyl | $CH_3$ | 4-Chlor-2-methylphenyl |
| 487 | H | $CH_3$ | 4-Chlor-2-methylphenyl |
| 488 | $CH_3$ | H | 4-Chlor-2-methylphenyl |
| 489 | OH | H | 4-Chlor-2-methylphenyl |
| 490 | $C(CH_3)_3$ | H | 4-Chlor-2-methylphenyl |
| 491 | $CF_3$ | H | 4-Chlor-2-methylphenyl |
| 492 | $CH(CH_3)_2$ | H | 4-Chlor-2-methylphenyl |
| 493 | 2-Furyl | H | 4-Chlor-2-methylphenyl |
| 494 | Cyclohexyl | H | 4-Chlor-2-methylphenyl |
| 495 | Cyclobutyl | H | 4-Chlor-2-methylphenyl |
| 496 | 4-Methylphenyl | H | 4-Chlor-2-methylphenyl |
| 497 | 2-Methylphenyl | H | 4-Chlor-2-methylphenyl |
| 498 | 2-Fluorphenyl | H | 4-Chlor-2-methylphenyl |
| 499 | 3-Fluorphenyl | H | 4-Chlor-2-methylphenyl |
| 500 | 4-Fluorphenyl | H | 4-Chlor-2-methylphenyl |
| 501 | OH | $CH_3$ | 4-Chlor-2-methylphenyl |
| 502 | OH | $CF_3$ | 4-Chlor-2-methylphenyl |
| 503 | OH | F | 4-Chlor-2-methylphenyl |
| 504 | OH | CN | 4-Chlor-2-methylphenyl |
| 505 | OH | Cl | 4-Chlor-2-methylphenyl |
| 506 | OH | $C_2H_5$ | 4-Chlor-2-methylphenyl |
| 507 | Phenyl | H | 4-Chlor-3-methylphenyl |
| 508 | Phenyl | $CH_3$ | 4-Chlor-3-methylphenyl |
| 509 | H | $CH_3$ | 4-Chlor-3-methylphenyl |
| 510 | $CH_3$ | H | 4-Chlor-3-methylphenyl |
| 511 | OH | H | 4-Chlor-3-methylphenyl |
| 512 | $C(CH_3)_3$ | H | 4-Chlor-3-methylphenyl |
| 513 | $CF_3$ | H | 4-Chlor-3-methylphenyl |
| 514 | $CH(CH_3)_2$ | H | 4-Chlor-3-methylphenyl |
| 515 | 2-Furyl | H | 4-Chlor-3-methylphenyl |
| 516 | Cyclohexyl | H | 4-Chlor-3-methylphenyl |
| 517 | Cyclobutyl | H | 4-Chlor-3-methylphenyl |
| 518 | 4-Methylphenyl | H | 4-Chlor-3-methylphenyl |
| 519 | 2-Methylphenyl | H | 4-Chlor-3-methylphenyl |
| 520 | 2-Fluorphenyl | H | 4-Chlor-3-methylphenyl |
| 521 | 3-Fluorphenyl | H | 4-Chlor-3-methylphenyl |

(fortgesetzt)

| | $R^1$ | $R^2$ | Q |
|---|---|---|---|
| 522 | 4-Fluorphenyl | H | 4-Chlor-3-methylphenyl |
| 523 | OH | $CH_3$ | 4-Chlor-3-methylphenyl |
| 524 | OH | $CF_3$ | 4-Chlor-3-methylphenyl |
| 525 | OH | F | 4-Chlor-3-methylphenyl |
| 526 | OH | CN | 4-Chlor-3-methylphenyl |
| 527 | OH | Cl | 4-Chlor-3-methylphenyl |
| 528 | OH | $C_2H_5$ | 4-Chlor-3-methylphenyl |
| 529 | Phenyl | H | 2-Chlor-4-methoxyphenyl |
| 530 | Phenyl | $CH_3$ | 2-Chlor-4-methoxyphenyl |
| 531 | H | $CH_3$ | 2-Chlor-4-methoxyphenyl |
| 532 | $CH_3$ | H | 2-Chlor-4-methoxyphenyl |
| 533 | OH | H | 2-Chlor-4-methoxyphenyl |
| 534 | $C(CH_3)_3$ | H | 2-Chlor-4-methoxyphenyl |
| 535 | $CF_3$ | H | 2-Chlor-4-methoxyphenyl |
| 536 | $CH(CH_3)_2$ | H | 2-Chlor-4-methoxyphenyl |
| 537 | 2-Furyl | H | 2-Chlor-4-methoxyphenyl |
| 538 | Cyclohexyl | H | 2-Chlor-4-methoxyphenyl |
| 539 | Cyclobutyl | H | 2-Chlor-4-methoxyphenyl |
| 540 | 4-Methylphenyl | H | 2-Chlor-4-methoxyphenyl |
| 541 | 2-Methylphenyl | H | 2-Chlor-4-methoxyphenyl |
| 542 | 2-Fluorphenyl | H | 2-Chlor-4-methoxyphenyl |
| 543 | 3-Fluorphenyl | H | 2-Chlor-4-methoxyphenyl |
| 544 | 4-Fluorphenyl | H | 2-Chlor-4-methoxyphenyl |
| 545 | OH | $CH_3$ | 2-Chlor-4-methoxyphenyl |
| 546 | OH | $CF_3$ | 2-Chlor-4-methoxyphenyl |
| 547 | OH | F | 2-Chlor-4-methoxyphenyl |
| 548 | OH | CN | 2-Chlor-4-methoxyphenyl |
| 549 | OH | Cl | 2-Chlor-4-methoxyphenyl |
| 550 | OH | $C_2H_5$ | 2-Chlor-4-methoxyphenyl |
| 551 | Phenyl | H | 2-Chlor-3-methoxyphenyl |
| 552 | Phenyl | $CH_3$ | 2-Chlor-3-methoxyphenyl |
| 553 | H | $CH_3$ | 2-Chlor-3-methoxyphenyl |
| 554 | $CH_3$ | H | 2-Chlor-3-methoxyphenyl |
| 555 | OH | H | 2-Chlor-3-methoxyphenyl |
| 556 | $C(CH_3)_3$ | H | 2-Chlor-3-methoxyphenyl |
| 557 | $CF_3$ | H | 2-Chlor-3-methoxyphenyl |
| 558 | $CH(CH_3)_2$ | H | 2-Chlor-3-methoxyphenyl |
| 559 | 2-Furyl | H | 2-Chlor-3-methoxyphenyl |

(fortgesetzt)

| | $R^1$ | $R^2$ | Q |
|---|---|---|---|
| 560 | Cyclohexyl | H | 2-Chlor-3-methoxyphenyl |
| 561 | Cyclobutyl | H | 2-Chlor-3-methoxyphenyl |
| 562 | 4-Methylphenyl | H | 2-Chlor-3-methoxyphenyl |
| 563 | 2-Methylphenyl | H | 2-Chlor-3-methoxyphenyl |
| 564 | 2-Fluorphenyl | H | 2-Chlor-3-methoxyphenyl |
| 565 | 3-Fluorphenyl | H | 2-Chlor-3-methoxyphenyl |
| 566 | 4-Fluorphenyl | H | 2-Chlor-3-methoxyphenyl |
| 567 | OH | $CH_3$ | 2-Chlor-3-methoxyphenyl |
| 568 | OH | $CF_3$ | 2-Chlor-3-methoxyphenyl |
| 569 | OH | F | 2-Chlor-3-methoxyphenyl |
| 570 | OH | CN | 2-Chlor-3-methoxyphenyl |
| 571 | OH | Cl | 2-Chlor-3-methoxyphenyl |
| 572 | OH | $C_2H_5$ | 2-Chlor-3-methoxyphenyl |
| 573 | Phenyl | H | 3-Chlor-4-methoxyphenyl |
| 574 | Phenyl | $CH_3$ | 3-Chlor-4-methoxyphenyl |
| 575 | H | $CH_3$ | 3-Chlor-4-methoxyphenyl |
| 576 | $CH_3$ | H | 3-Chlor-4-methoxyphenyl |
| 577 | OH | H | 3-Chlor-4-methoxyphenyl |
| 578 | $C(CH_3)_3$ | H | 3-Chlor-4-methoxyphenyl |
| 579 | $CF_3$ | H | 3-Chlor-4-methoxyphenyl |
| 580 | $CH(CH_3)_2$ | H | 3-Chlor-4-methoxyphenyl |
| 581 | 2-Furyl | H | 3-Chlor-4-methoxyphenyl |
| 582 | Cyclohexyl | H | 3-Chlor-4-methoxyphenyl |
| 583 | Cyclobutyl | H | 3-Chlor-4-methoxyphenyl |
| 584 | 4-Methylphenyl | H | 3-Chlor-4-methoxyphenyl |
| 585 | 2-Methylphenyl | H | 3-Chlor-4-methoxyphenyl |
| 586 | 2-Fluorphenyl | H | 3-Chlor-4-methoxyphenyl |
| 587 | 3-Fluorphenyl | H | 3-Chlor-4-methoxyphenyl |
| 588 | 4-Fluorphenyl | H | 3-Chlor-4-methoxyphenyl |
| 589 | OH | $CH_3$ | 3-Chlor-4-methoxyphenyl |
| 590 | OH | $CF_3$ | 3-Chlor-4-methoxyphenyl |
| 591 | OH | F | 3-Chlor-4-methoxyphenyl |
| 592 | OH | CN | 3-Chlor-4-methoxyphenyl |
| 593 | OH | Cl | 3-Chlor-4-methoxyphenyl |
| 594 | OH | $C_2H_5$ | 3-Chlor-4-methoxyphenyl |
| 595 | Phenyl | H | 4-Chlor-3-methoxyphenyl |
| 596 | Phenyl | $CH_3$ | 4-Chlor-3-methoxyphenyl |
| 597 | H | $CH_3$ | 4-Chlor-3-methoxyphenyl |

(fortgesetzt)

| | $R^1$ | $R^2$ | Q |
|---|---|---|---|
| 598 | $CH_3$ | H | 4-Chlor-3-methoxyphenyl |
| 599 | OH | H | 4-Chlor-3-methoxyphenyl |
| 600 | $C(CH_3)_3$ | H | 4-Chlor-3-methoxyphenyl |
| 601 | $CF_3$ | H | 4-Chlor-3-methoxyphenyl |
| 602 | $CH(CH_3)_2$ | H | 4-Chlor-3-methoxyphenyl |
| 603 | 2-Furyl | H | 4-Chlor-3-methoxyphenyl |
| 604 | Cyclohexyl | H | 4-Chlor-3-methoxyphenyl |
| 605 | Cyclobutyl | H | 4-Chlor-3-methoxyphenyl |
| 606 | 4-Methylphenyl | H | 4-Chlor-3-methoxyphenyl |
| 607 | 2-Methylphenyl | H | 4-Chlor-3-methoxyphenyl |
| 608 | 2-Fluorphenyl | H | 4-Chlor-3-methoxyphenyl |
| 609 | 3-Fluorphenyl | H | 4-Chlor-3-methoxyphenyl |
| 610 | 4-Fluorphenyl | H | 4-Chlor-3-methoxyphenyl |
| 611 | OH | $CH_3$ | 4-Chlor-3-methoxyphenyl |
| 612 | OH | $CF_3$ | 4-Chlor-3-methoxyphenyl |
| 613 | OH | F | 4-Chlor-3-methoxyphenyl |
| 614 | OH | CN | 4-Chlor-3-methoxyphenyl |
| 615 | OH | Cl | 4-Chlor-3-methoxyphenyl |
| 616 | OH | $C_2H_5$ | 4-Chlor-3-methoxyphenyl |
| 617 | Phenyl | H | 4-Chlor-2-methoxyphenyl |
| 618 | Phenyl | $CH_3$ | 4-Chlor-2-methoxyphenyl |
| 619 | H | $CH_3$ | 4-Chlor-2-methoxyphenyl |
| 620 | $CH_3$ | H | 4-Chlor-2-methoxyphenyl |
| 621 | OH | H | 4-Chlor-2-methoxyphenyl |
| 622 | $C(CH_3)_3$ | H | 4-Chlor-2-methoxyphenyl |
| 623 | $CF_3$ | H | 4-Chlor-2-methoxyphenyl |
| 624 | $CH(CH_3)_2$ | H | 4-Chlor-2-methoxyphenyl |
| 625 | 2-Furyl | H | 4-Chlor-2-methoxyphenyl |
| 626 | Cyclohexyl | H | 4-Chlor-2-methoxyphenyl |
| 627 | Cyclobutyl | H | 4-Chlor-2-methoxyphenyl |
| 628 | 4-Methylphenyl | H | 4-Chlor-2-methoxyphenyl |
| 629 | 2-Methylphenyl | H | 4-Chlor-2-methoxyphenyl |
| 630 | 2-Fluorphenyl | H | 4-Chlor-2-methoxyphenyl |
| 631 | 3-Fluorphenyl | H | 4-Chlor-2-methoxyphenyl |
| 632 | 4-Fluorphenyl | H | 4-Chlor-2-methoxyphenyl |
| 633 | OH | $CH_3$ | 4-Chlor-2-methoxyphenyl |
| 634 | OH | $CF_3$ | 4-Chlor-2-methoxyphenyl |
| 635 | OH | F | 4-Chlor-2-methoxyphenyl |

(fortgesetzt)

| | $R^1$ | $R^2$ | Q |
|---|---|---|---|
| 636 | OH | CN | 4-Chlor-2-methoxyphenyl |
| 637 | OH | Cl | 4-Chlor-2-methoxyphenyl |
| 638 | OH | $C_2H_5$ | 4-Chlor-2-methoxyphenyl |
| 639 | Phenyl | H | 2,4,5-Trimethoxyphenyl |
| 640 | Phenyl | $CH_3$ | 2,4,5-Trimethoxyphenyl |
| 641 | H | $CH_3$ | 2,4,5-Trimethoxyphenyl |
| 642 | $CH_3$ | H | 2,4,5-Trimethoxyphenyl |
| 643 | OH | H | 2,4,5-Trimethoxyphenyl |
| 644 | $C(CH_3)_3$ | H | 2,4,5-Trimethoxyphenyl |
| 645 | $CF_3$ | H | 2,4,5-Trimethoxyphenyl |
| 646 | $CH(CH_3)_2$ | H | 2,4,5-Trimethoxyphenyl |
| 647 | 2-Furyl | H | 2,4,5-Trimethoxyphenyl |
| 648 | Cyclohexyl | H | 2,4,5-Trimethoxyphenyl |
| 649 | Cyclobutyl | H | 2,4,5-Trimethoxyphenyl |
| 650 | 4-Methylphenyl | H | 2,4,5-Trimethoxyphenyl |
| 651 | 2-Methylphenyl | H | 2,4,5-Trimethoxyphenyl |
| 652 | 2-Fluorphenyl | H | 2,4,5-Trimethoxyphenyl |
| 653 | 3-Fluorphenyl | H | 2,4,5-Trimethoxyphenyl |
| 654 | 4-Fluorphenyl | H | 2,4,5-Trimethoxyphenyl |
| 655 | OH | $CH_3$ | 2,4,5-Trimethoxyphenyl |
| 656 | OH | $CF_3$ | 2,4,5-Trimethoxyphenyl |
| 657 | OH | F | 2,4,5-Trimethoxyphenyl |
| 658 | OH | CN | 2,4,5-Trimethoxyphenyl |
| 659 | OH | Cl | 2,4,5-Trimethoxyphenyl |
| 660 | OH | $C_2H_5$ | 2,4,5-Trimethoxyphenyl |
| 661 | Phenyl | H | 2,3,4-Trimethoxyphenyl |
| 662 | Phenyl | $CH_3$ | 2,3,4-Trimethoxyphenyl |
| 663 | H | $CH_3$ | 2,3,4-Trimethoxyphenyl |
| 664 | $CH_3$ | H | 2,3,4-Trimethoxyphenyl |
| 665 | OH | H | 2,3,4-Trimethoxyphenyl |
| 666 | $C(CH_3)_3$ | H | 2,3,4-Trimethoxyphenyl |
| 667 | $CF_3$ | H | 2,3,4-Trimethoxyphenyl |
| 668 | $CH(CH_3)_2$ | H | 2,3,4-Trimethoxyphenyl |
| 669 | 2-Furyl | H | 2,3,4-Trimethoxyphenyl |
| 670 | Cyclohexyl | H | 2,3,4-Trimethoxyphenyl |
| 671 | Cyclobutyl | H | 2,3,4-Trimethoxyphenyl |
| 672 | 4-Methylphenyl | H | 2,3,4-Trimethoxyphenyl |
| 673 | 2-Methylphenyl | H | 2,3,4-Trimethoxyphenyl |

(fortgesetzt)

| | $R^1$ | $R^2$ | Q |
|---|---|---|---|
| 674 | 2-Fluorphenyl | H | 2,3,4-Trimethoxyphenyl |
| 675 | 3-Fluorphenyl | H | 2,3,4-Trimethoxyphenyl |
| 676 | 4-Fluorphenyl | H | 2,3,4-Trimethoxyphenyl |
| 677 | OH | $CH_3$ | 2,3,4-Trimethoxyphenyl |
| 678 | OH | $CF_3$ | 2,3,4-Trimethoxyphenyl |
| 679 | OH | F | 2,3,4-Trimethoxyphenyl |
| 680 | OH | CN | 2,3,4-Trimethoxyphenyl |
| 681 | OH | Cl | 2,3,4-Trimethoxyphenyl |
| 682 | OH | $C_2H_5$ | 2,3,4-Trimethoxyphenyl |
| 683 | Phenyl | H | 4-tert.-Butylphenyl |
| 684 | Phenyl | $CH_3$ | 4-tert.-Butylphenyl |
| 685 | H | $CH_3$ | 4-tert.-Butylphenyl |
| 686 | $CH_3$ | H | 4-tert.-Butylphenyl |
| 687 | OH | H | 4-tert.-Butylphenyl |
| 688 | $C(CH_3)_3$ | H | 4-tert.-Butylphenyl |
| 689 | $CF_3$ | H | 4-tert.-Butylphenyl |
| 690 | $CH(CH_3)_2$ | H | 4-tert.-Butylphenyl |
| 691 | 2-Furyl | H | 4-tert.-Butylphenyl |
| 692 | Cyclohexyl | H | 4-tert.-Butylphenyl |
| 693 | Cyclobutyl | H | 4-tert.-Butylphenyl |
| 694 | 4-Methylphenyl | H | 4-tert.-Butylphenyl |
| 695 | 2-Methylphenyl | H | 4-tert.-Butylphenyl |
| 696 | 2-Fluorphenyl | H | 4-tert.-Butylphenyl |
| 697 | 3-Fluorphenyl | H | 4-tert.-Butylphenyl |
| 698 | 4-Fluorphenyl | H | 4-tert.-Butylphenyl |
| 699 | OH | $CH_3$ | 4-tert.-Butylphenyl |
| 700 | OH | $CF_3$ | 4-tert.-Butylphenyl |
| 701 | OH | F | 4-tert.-Butylphenyl |
| 702 | OH | CN | 4-tert.-Butylphenyl |
| 703 | OH | Cl | 4-tert.-Butylphenyl |
| 704 | OH | $C_2H_5$ | 4-tert.-Butylphenyl |
| 705 | Phenyl | H | 4-Methylphenyl |
| 706 | Phenyl | $CH_3$ | 4-Methylphenyl |
| 707 | H | $CH_3$ | 4-Methylphenyl |
| 708 | $CH_3$ | H | 4-Methylphenyl |
| 709 | OH | H | 4-Methylphenyl |
| 710 | $C(CH_3)_3$ | H | 4-Methylphenyl |
| 711 | $CF_3$ | H | 4-Methylphenyl |

(fortgesetzt)

| | $R^1$ | $R^2$ | Q |
|---|---|---|---|
| 712 | $CH(CH_3)_2$ | H | 4-Methylphenyl |
| 713 | 2-Furyl | H | 4-Methylphenyl |
| 714 | Cyclohexyl | H | 4-Methylphenyl |
| 715 | Cyclobutyl | H | 4-Methylphenyl |
| 716 | 4-Methylphenyl | H | 4-Methylphenyl |
| 717 | 2-Methylphenyl | H | 4-Methylphenyl |
| 718 | 2-Fluorphenyl | H | 4-Methylphenyl |
| 719 | 3-Fluorphenyl | H | 4-Methylphenyl |
| 720 | 4-Fluorphenyl | H | 4-Methylphenyl |
| 721 | OH | $CH_3$ | 4-Methylphenyl |
| 722 | OH | $CF_3$ | 4-Methylphenyl |
| 723 | OH | F | 4-Methylphenyl |
| 724 | OH | CN | 4-Methylphenyl |
| 725 | OH | Cl | 4-Methylphenyl |
| 726 | OH | $C_2H_5$ | 4-Methylphenyl |
| 727 | Phenyl | H | 3-Methoxyphenyl |
| 728 | Phenyl | $CH_3$ | 3-Methoxyphenyl |
| 729 | H | $CH_3$ | 3-Methoxyphenyl |
| 730 | $CH_3$ | H | 3-Methoxyphenyl |
| 731 | OH | H | 3-Methoxyphenyl |
| 732 | $C(CH_3)_3$ | H | 3-Methoxyphenyl |
| 733 | $CF_3$ | H | 3-Methoxyphenyl |
| 734 | $CH(CH_3)_2$ | H | 3-Methoxyphenyl |
| 735 | 2-Furyl | H | 3-Methoxyphenyl |
| 736 | Cyclohexyl | H | 3-Methoxyphenyl |
| 737 | Cyclobutyl | H | 3-Methoxyphenyl |
| 738 | 4-Methylphenyl | H | 3-Methoxyphenyl |
| 739 | 2-Methylphenyl | H | 3-Methoxyphenyl |
| 740 | 2-Fluorphenyl | H | 3-Methoxyphenyl |
| 741 | 3-Fluorphenyl | H | 3-Methoxyphenyl |
| 742 | 4-Fluorphenyl | H | 3-Methoxyphenyl |
| 743 | OH | $CH_3$ | 3-Methoxyphenyl |
| 744 | OH | $CF_3$ | 3-Methoxyphenyl |
| 745 | OH | F | 3-Methoxyphenyl |
| 746 | OH | CN | 3-Methoxyphenyl |
| 747 | OH | Cl | 3-Methoxyphenyl |
| 748 | OH | $C_2H_5$ | 3-Methoxyphenyl |
| 749 | Phenyl | H | 4-Methoxyphenyl |

(fortgesetzt)

| | $R^1$ | $R^2$ | Q |
|---|---|---|---|
| 750 | Phenyl | $CH_3$ | 4-Methoxyphenyl |
| 751 | H | $CH_3$ | 4-Methoxyphenyl |
| 752 | $CH_3$ | H | 4-Methoxyphenyl |
| 753 | OH | H | 4-Methoxyphenyl |
| 754 | $C(CH_3)_3$ | H | 4-Methoxyphenyl |
| 755 | $CF_3$ | H | 4-Methoxyphenyl |
| 756 | $CH(CH_3)_2$ | H | 4-Methoxyphenyl |
| 757 | 2-Furyl | H | 4-Methoxyphenyl |
| 758 | Cyclohexyl | H | 4-Methoxyphenyl |
| 759 | Cyclobutyl | H | 4-Methoxyphenyl |
| 760 | 4-Methylphenyl | H | 4-Methoxyphenyl |
| 761 | 2-Methylphenyl | H | 4-Methoxyphenyl |
| 762 | 2-Fluorphenyl | H | 4-Methoxyphenyl |
| 763 | 3-Fluorphenyl | H | 4-Methoxyphenyl |
| 764 | 4-Fluorphenyl | H | 4-Methoxyphenyl |
| 765 | OH | $CH_3$ | 4-Methoxyphenyl |
| 766 | OH | $CF_3$ | 4-Methoxyphenyl |
| 767 | OH | F | 4-Methoxyphenyl |
| 768 | OH | CN | 4-Methoxyphenyl |
| 769 | OH | Cl | 4-Methoxyphenyl |
| 770 | OH | $C_2H_5$ | 4-Methoxyphenyl |
| 771 | Phenyl | H | 4-Trifluormethylphenyl |
| 772 | Phenyl | $CH_3$ | 4-Trilfuormethylphenyl |
| 773 | H | $CH_3$ | 4-Trilfuormethylphenyl |
| 774 | $CH_3$ | H | 4-Trilfuormethylphenyl |
| 775 | OH | H | 4-Trilfuormethylphenyl |
| 776 | $C(CH_3)_3$ | H | 4-Trifluormethylphenyl |
| 777 | $CF_3$ | H | 4-Trilfuormethylphenyl |
| 778 | $CH(CH_3)_2$ | H | 4-Trilfuormethylphenyl |
| 779 | 2-Furyl | H | 4-Trilfuormethylphenyl |
| 780 | Cyclohexyl | H | 4-Trilfuormethylphenyl |
| 781 | Cyclobutyl | H | 4-Trilfuormethylphenyl |
| 782 | 4-Methylphenyl | H | 4-Trilfuormethylphenyl |
| 783 | 2-Methylphenyl | H | 4-Trilfuormethylphenyl |
| 784 | 2-Fluorphenyl | H | 4-Trilfuormethylphenyl |
| 785 | 3-Fluorphenyl | H | 4-Trilfuormethylphenyl |
| 786 | 4-Fluorphenyl | H | 4-Trilfuormethylphenyl |
| 787 | OH | $CH_3$ | 4-Trilfuormethylphenyl |

(fortgesetzt)

| | $R^1$ | $R^2$ | Q |
|---|---|---|---|
| 788 | OH | $CF_3$ | 4-Trilfuormethylphenyl |
| 789 | OH | F | 4-Trilfuormethylphenyl |
| 790 | OH | CN | 4-Trilfuormethylphenyl |
| 791 | OH | Cl | 4-Trilfuormethylphenyl |
| 792 | OH | $C_2H_5$ | 4-Trilfuormethylphenyl |
| 793 | Phenyl | H | 2-Chlor-4-trifluormethylphenyl |
| 794 | Phenyl | $CH_3$ | 2-Chlor-4-trifluormethylphenyl |
| 795 | H | $CH_3$ | 2-Chlor-4-trifluormethylphenyl |
| 796 | $CH_3$ | H | 2-Chlor-4-trifluormethylphenyl |
| 797 | OH | H | 2-Chlor-4-trifluormethylphenyl |
| 798 | $C(CH_3)_3$ | H | 2-Chlor-4-trifluormethylphenyl |
| 799 | $CF_3$ | H | 2-Chlor-4-trifluormethylphenyl |
| 800 | $CH(CH_3)_2$ | H | 2-Chlor-4-trifluormethylphenyl |
| 801 | 2-Furyl | H | 2-Chlor-4-trifluormethylphenyl |
| 802 | Cyclohexyl | H | 2-Chlor-4-trifluormethylphenyl |
| 803 | Cyclobutyl | H | 2-Chlor-4-trifluormethylphenyl |
| 804 | 4-Methylphenyl | H | 2-Chlor-4-trifluormethylphenyl |
| 805 | 2-Methylphenyl | H | 2-Chlor-4-trifluormethylphenyl |
| 806 | 2-Fluorphenyl | H | 2-Chlor-4-trifluormethylphenyl |
| 807 | 3-Fluorphenyl | H | 2-Chlor-4-trifluormethylphenyl |
| 808 | 4-Fluorphenyl | H | 2-Chlor-4-trifluormethylphenyl |
| 809 | OH | $CH_3$ | 2-Chlor-4-trifluormethylphenyl |
| 810 | OH | $CF_3$ | 2-Chlor-4-trifluormethylphenyl |
| 811 | OH | F | 2-Chlor-4-trifluormethylphenyl |
| 812 | OH | CN | 2-Chlor-4-trifluormethylphenyl |
| 813 | OH | Cl | 2-Chlor-4-trifluormethylphenyl |
| 814 | OH | $C_2H_5$ | 2-Chlor-4-trifluormethylphenyl |
| 815 | Phenyl | H | 3-Chlor-4-trifluormethylphenyl |
| 816 | Phenyl | $CH_3$ | 3-Chlor-4-trifluormethylphenyl |
| 817 | H | $CH_3$ | 3-Chlor-4-trifluormethylphenyl |
| 818 | $CH_3$ | H | 3-Chlor-4-trifluormethylphenyl |
| 819 | OH | H | 3-Chlor-4-trifluormethylphenyl |
| 820 | $C(CH_3)_3$ | H | 3-Chlor-4-trifluormethylphenyl |
| 821 | $CF_3$ | H | 3-Chlor-4-trifluormethylphenyl |
| 822 | $CH(CH_3)_2$ | H | 3-Chlor-4-trifluormethylphenyl |
| 823 | 2-Furyl | H | 3-Chlor-4-trifluormethylphenyl |
| 824 | Cyclohexyl | H | 3-Chlor-4-trifluormethylphenyl |
| 825 | Cyclobutyl | H | 3-Chlor-4-trifluormethylphenyl |

(fortgesetzt)

| | $R^1$ | $R^2$ | Q |
|---|---|---|---|
| 826 | 4-Methylphenyl | H | 3-Chlor-4-trifluormethylphenyl |
| 827 | 2-Methylphenyl | H | 3-Chlor-4-trifluormethylphenyl |
| 828 | 2-Fluorphenyl | H | 3-Chlor-4-trifluormethylphenyl |
| 829 | 3-Fluorphenyl | H | 3-Chlor-4-trifluormethylphenyl |
| 830 | 4-Fluorphenyl | H | 3-Chlor-4-trifluormethylphenyl |
| 831 | OH | $CH_3$ | 3-Chlor-4-trifluormethylphenyl |
| 832 | OH | $CF_3$ | 3-Chlor-4-trifluormethylphenyl |
| 833 | OH | F | 3-Chlor-4-trifluormethylphenyl |
| 834 | OH | CN | 3-Chlor-4-trifluormethylphenyl |
| 835 | OH | Cl | 3-Chlor-4-trifluormethylphenyl |
| 836 | OH | $C_2H_5$ | 3-Chlor-4-trifluormethylphenyl |
| 837 | Phenyl | H | 2-Trilfuormethylphenyl |
| 838 | Phenyl | $CH_3$ | 2-Trilfuormethylphenyl |
| 839 | H | $CH_3$ | 2-Trilfuormethylphenyl |
| 840 | $CH_3$ | H | 2-Trifluormethylphenyl |
| 841 | OH | H | 2-Trilfuormethylphenyl |
| 842 | $C(CH_3)_3$ | H | 2-Trilfuormethylphenyl |
| 843 | $CF_3$ | H | 2-Trilfuormethylphenyl |
| 844 | $CH(CH_3)_2$ | H | 2-Trilfuormethylphenyl |
| 845 | 2-Furyl | H | 2-Trilfuormethylphenyl |
| 846 | Cyclohexyl | H | 2-Trilfuormethylphenyl |
| 847 | Cyclobutyl | H | 2-Trilfuormethylphenyl |
| 848 | 4-Methylphenyl | H | 2-Trilfuormethylphenyl |
| 849 | 2-Methylphenyl | H | 2-Trilfuormethylphenyl |
| 850 | 2-Fluorphenyl | H | 2-Trilfuormethylphenyl |
| 851 | 3-Fluorphenyl | H | 2-Trifluormethylphenyl |
| 852 | 4-Fluorphenyl | H | 2-Trilfuormethylphenyl |
| 853 | OH | $CH_3$ | 2-Trifluormethylphenyl |
| 854 | OH | $CF_3$ | 2-Trilfuormethylphenyl |
| 855 | OH | F | 2-Trilfuormethylphenyl |
| 856 | OH | CN | 2-Trilfuormethylphenyl |
| 857 | OH | Cl | 2-Trifluormethylphenyl |
| 858 | OH | $C_2H_5$ | 2-Trilfuormethylphenyl |
| 859 | Phenyl | H | 4-Trifluormethoxyphenyl |
| 860 | Phenyl | $CH_3$ | 4-Trifluormethoxyphenyl |
| 861 | H | $CH_3$ | 4-Trifluormethoxyphenyl |
| 862 | $CH_3$ | H | 4-Trifluormethoxyphenyl |
| 863 | OH | H | 4-Trifluormethoxyphenyl |

(fortgesetzt)

| | $R^1$ | $R^2$ | Q |
|---|---|---|---|
| 864 | $C(CH_3)_3$ | H | 4-Trifluormethoxyphenyl |
| 865 | $CF_3$ | H | 4-Trifluormethoxyphenyl |
| 866 | $CH(CH_3)_2$ | H | 4-Trifluormethoxyphenyl |
| 867 | 2-Furyl | H | 4-Trifluormethoxyphenyl |
| 868 | Cyclohexyl | H | 4-Trifluormethoxyphenyl |
| 869 | Cyclobutyl | H | 4-Trifluormethoxyphenyl |
| 870 | 4-Methylphenyl | H | 4-Trifluormethoxyphenyl |
| 871 | 2-Methylphenyl | H | 4-Trifluormethoxyphenyl |
| 872 | 2-Fluorphenyl | H | 4-Trifluormethoxyphenyl |
| 873 | 3-Fluorphenyl | H | 4-Trifluormethoxyphenyl |
| 874 | 4-Fluorphenyl | H | 4-Trifluormethoxyphenyl |
| 875 | OH | $CH_3$ | 4-Trifluormethoxyphenyl |
| 876 | OH | $CF_3$ | 4-Trifluormethoxyphenyl |
| 877 | OH | F | 4-Trifluormethoxyphenyl |
| 878 | OH | CN | 4-Trifluormethoxyphenyl |
| 879 | OH | Cl | 4-Trifluormethoxyphenyl |
| 880 | OH | $C_2H_5$ | 4-Trifluormethoxyphenyl |
| 881 | Phenyl | H | 4-Isopropylphenyl |
| 882 | Phenyl | $CH_3$ | 4-Isopropylphenyl |
| 883 | H | $CH_3$ | 4-Isopropylphenyl |
| 884 | $CH_3$ | H | 4-Isopropylphenyl |
| 885 | OH | H | 4-Isopropylphenyl |
| 886 | $C(CH_3)_3$ | H | 4-Isopropylphenyl |
| 887 | $CF_3$ | H | 4-Isopropylphenyl |
| 888 | $CH(CH_3)_2$ | H | 4-Isopropylphenyl |
| 889 | 2-Furyl | H | 4-Isopropylphenyl |
| 890 | Cyclohexyl | H | 4-Isopropylphenyl |
| 891 | Cyclobutyl | H | 4-Isopropylphenyl |
| 892 | 4-Methylphenyl | H | 4-Isopropylphenyl |
| 893 | 2-Methylphenyl | H | 4-Isopropylphenyl |
| 894 | 2-Fluorphenyl | H | 4-Isopropylphenyl |
| 895 | 3-Fluorphenyl | H | 4-Isopropylphenyl |
| 896 | 4-Fluorphenyl | H | 4-Isopropylphenyl |
| 897 | OH | $CH_3$ | 4-Isopropylphenyl |
| 898 | OH | $CF_3$ | 4-Isopropylphenyl |
| 899 | OH | F | 4-Isopropylphenyl |
| 900 | OH | CN | 4-Isopropylphenyl |
| 901 | OH | Cl | 4-Isopropylphenyl |

(fortgesetzt)

| | $R^1$ | $R^2$ | Q |
|---|---|---|---|
| 902 | OH | $C_2H_5$ | 4-Isopropylphenyl |
| 903 | Phenyl | H | 4-Cyclopropylphenyl |
| 904 | Phenyl | $CH_3$ | 4-Cyclopropylphenyl |
| 905 | H | $CH_3$ | 4-Cyclopropylphenyl |
| 906 | $CH_3$ | H | 4-Cyclopropylphenyl |
| 907 | OH | H | 4-Cyclopropylphenyl |
| 908 | $C(CH_3)_3$ | H | 4-Cyclopropylphenyl |
| 909 | $CF_3$ | H | 4-Cyclopropylphenyl |
| 910 | $CH(CH_3)_2$ | H | 4-Cyclopropylphenyl |
| 911 | 2-Furyl | H | 4-Cyclopropylphenyl |
| 912 | Cyclohexyl | H | 4-Cyclopropylphenyl |
| 913 | Cyclobutyl | H | 4-Cyclopropylphenyl |
| 914 | 4-Methylphenyl | H | 4-Cyclopropylphenyl |
| 915 | 2-Methylphenyl | H | 4-Cyclopropylphenyl |
| 916 | 2-Fluorphenyl | H | 4-Cyclopropylphenyl |
| 917 | 3-Fluorphenyl | H | 4-Cyclopropylphenyl |
| 918 | 4-Fluorphenyl | H | 4-Cyclopropylphenyl |
| 919 | OH | $CH_3$ | 4-Cyclopropylphenyl |
| 920 | OH | $CF_3$ | 4-Cyclopropylphenyl |
| 921 | OH | F | 4-Cyclopropylphenyl |
| 922 | OH | CN | 4-Cyclopropylphenyl |
| 923 | OH | Cl | 4-Cyclopropylphenyl |
| 924 | OH | $C_2H_5$ | 4-Cyclopropylphenyl |
| 925 | Phenyl | H | 4-Dimethylaminophenyl |
| 926 | Phenyl | $CH_3$ | 4-Dimethylaminophenyl |
| 927 | H | $CH_3$ | 4-Dimethylaminophenyl |
| 928 | $CH_3$ | H | 4-Dimethylaminophenyl |
| 929 | OH | H | 4-Dimethylaminophenyl |
| 930 | $C(CH_3)_3$ | H | 4-Dimethylaminophenyl |
| 931 | $CF_3$ | H | 4-Dimethylaminophenyl |
| 932 | $CH(CH_3)_2$ | H | 4-Dimethylaminophenyl |
| 933 | 2-Furyl | H | 4-Dimethylaminophenyl |
| 934 | Cyclohexyl | H | 4-Dimethylaminophenyl |
| 935 | Cyclobutyl | H | 4-Dimethylaminophenyl |
| 936 | 4-Methylphenyl | H | 4-Dimethylaminophenyl |
| 937 | 2-Methylphenyl | H | 4-Dimethylaminophenyl |
| 938 | 2-Fluorphenyl | H | 4-Dimethylaminophenyl |
| 939 | 3-Fluorphenyl | H | 4-Dimethylaminophenyl |

(fortgesetzt)

| | $R^1$ | $R^2$ | Q |
|---|---|---|---|
| 940 | 4-Fluorphenyl | H | 4-Dimethylaminophenyl |
| 941 | OH | $CH_3$ | 4-Dimethylaminophenyl |
| 942 | OH | $CF_3$ | 4-Dimethylaminophenyl |
| 943 | OH | F | 4-Dimethylaminophenyl |
| 944 | OH | CN | 4-Dimethylaminophenyl |
| 945 | OH | Cl | 4-Dimethylaminophenyl |
| 946 | OH | $C_2H_5$ | 4-Dimethylaminophenyl |
| 947 | Phenyl | H | 2-Pyridinyl |
| 948 | Phenyl | $CH_3$ | 2-Pyridinyl |
| 949 | H | $CH_3$ | 2-Pyridinyl |
| 950 | $CH_3$ | H | 2-Pyridinyl |
| 951 | OH | H | 2-Pyridinyl |
| 952 | $C(CH_3)_3$ | H | 2-Pyridinyl |
| 953 | $CF_3$ | H | 2-Pyridinyl |
| 954 | $CH(CH_3)_2$ | H | 2-Pyridinyl |
| 955 | 2-Furyl | H | 2-Pyridinyl |
| 956 | Cyclohexyl | H | 2-Pyridinyl |
| 957 | Cyclobutyl | H | 2-Pyridinyl |
| 958 | 4-Methylphenyl | H | 2-Pyridinyl |
| 959 | 2-Methylphenyl | H | 2-Pyridinyl |
| 960 | 2-Fluorphenyl | H | 2-Pyridinyl |
| 961 | 3-Fluorphenyl | H | 2-Pyridinyl |
| 962 | 4-Fluorphenyl | H | 2-Pyridinyl |
| 963 | OH | $CH_3$ | 2-Pyridinyl |
| 964 | OH | $CF_3$ | 2-Pyridinyl |
| 965 | OH | F | 2-Pyridinyl |
| 966 | OH | CN | 2-Pyridinyl |
| 967 | OH | Cl | 2-Pyridinyl |
| 968 | OH | $C_2H_5$ | 2-Pyridinyl |
| 969 | Phenyl | H | 3-Pyridinyl |
| 970 | Phenyl | $CH_3$ | 3-Pyridinyl |
| 971 | H | $CH_3$ | 3-Pyridinyl |
| 972 | $CH_3$ | H | 3-Pyridinyl |
| 973 | OH | H | 3-Pyridinyl |
| 974 | $C(CH_3)_3$ | H | 3-Pyridinyl |
| 975 | $CF_3$ | H | 3-Pyridinyl |
| 976 | $CH(CH_3)_2$ | H | 3-Pyridinyl |
| 977 | 2-Furyl | H | 3-Pyridinyl |

(fortgesetzt)

| | $R^1$ | $R^2$ | Q |
|---|---|---|---|
| 978 | Cyclohexyl | H | 3-Pyridinyl |
| 979 | Cyclobutyl | H | 3-Pyridinyl |
| 980 | 4-Methylphenyl | H | 3-Pyridinyl |
| 981 | 2-Methylphenyl | H | 3-Pyridinyl |
| 982 | 2-Fluorphenyl | H | 3-Pyrid inyl |
| 983 | 3-Fluorphenyl | H | 3-Pyridinyl |
| 984 | 4-Fluorphenyl | H | 3-Pyridinyl |
| 985 | OH | $CH_3$ | 3-Pyridinyl |
| 986 | OH | $CF_3$ | 3-Pyridinyl |
| 987 | OH | F | 3-Pyridinyl |
| 988 | OH | CN | 3-Pyridinyl |
| 989 | OH | Cl | 3-Pyridinyl |
| 990 | OH | $C_2H_5$ | 3-Pyridinyl |
| 991 | Phenyl | H | 4-Pyridinyl |
| 992 | Phenyl | $CH_3$ | 4-Pyridinyl |
| 993 | H | $CH_3$ | 4-Pyridinyl |
| 994 | $CH_3$ | H | 4-Pyridinyl |
| 995 | OH | H | 4-Pyridinyl |
| 996 | $C(CH_3)_3$ | H | 4-Pyridinyl |
| 997 | $CF_3$ | H | 4-Pyridinyl |
| 998 | $CH(CH_3)_2$ | H | 4-Pyridinyl |
| 999 | 2-Furyl | H | 4-Pyridinyl |
| 1000 | Cyclohexyl | H | 4-Pyridinyl |
| 1001 | Cyclobutyl | H | 4-Pyridinyl |
| 1002 | 4-Methylphenyl | H | 4-Pyridinyl |
| 1003 | 2-Methylphenyl | H | 4-Pyridinyl |
| 1004 | 2-Fluorphenyl | H | 4-Pyridinyl |
| 1005 | 3-Fluorphenyl | H | 4-Pyridinyl |
| 1006 | 4-Fluorphenyl | H | 4-Pyridinyl |
| 1007 | OH | $CH_3$ | 4-Pyridinyl |
| 1008 | OH | $CF_3$ | 4-Pyridinyl |
| 1009 | OH | F | 4-Pyridinyl |
| 1010 | OH | CN | 4-Pyridinyl |
| 1011 | OH | Cl | 4-Pyridinyl |
| 1012 | OH | $C_2H_5$ | 4-Pyridinyl |
| 1013 | Phenyl | H | 4,6-Dimethoxypyrimidin-2-yl |
| 1014 | Phenyl | $CH_3$ | 4,6-Dimethoxypyrimidin-2-yl |
| 1015 | H | $CH_3$ | 4,6-Dimethoxypyrimidin-2-yl |

(fortgesetzt)

| | $R^1$ | $R^2$ | Q |
|---|---|---|---|
| 1016 | $CH_3$ | H | 4,6-Dimethoxypyrimidin-2-yl |
| 1017 | OH | H | 4,6-Dimethoxypyrimidin-2-yl |
| 1018 | $C(CH_3)_3$ | H | 4,6-Dimethoxypyrimidin-2-yl |
| 1019 | $CF_3$ | H | 4,6-Dimethoxypyrimidin-2-yl |
| 1020 | $CH(CH_3)_2$ | H | 4,6-Dimethoxypyrimidin-2-yl |
| 1021 | 2-Furyl | H | 4,6-Dimethoxypyrimidin-2-yl |
| 1022 | Cyclohexyl | H | 4,6-Dimethoxypyrimidin-2-yl |
| 1023 | Cyclobutyl | H | 4,6-Dimethoxypyrimidin-2-yl |
| 1024 | 4-Methylphenyl | H | 4,6-Dimethoxypyrimidin-2-yl |
| 1025 | 2-Methylphenyl | H | 4,6-Dimethoxypyrimidin-2-yl |
| 1026 | 2-Fluorphenyl | H | 4,6-Dimethoxypyrimidin-2-yl |
| 1027 | 3-Fluorphenyl | H | 4,6-Dimethoxypyrimidin-2-yl |
| 1028 | 4-Fluorphenyl | H | 4,6-Dimethoxypyrimidin-2-yl |
| 1029 | OH | $CH_3$ | 4,6-Dimethoxypyrimidin-2-yl |
| 1030 | OH | $CF_3$ | 4,6-Dimethoxypyrimidin-2-yl |
| 1031 | OH | F | 4,6-Dimethoxypyrimidin-2-yl |
| 1032 | OH | CN | 4,6-Dimethoxypyrimidin-2-yl |
| 1033 | OH | Cl | 4,6-Dimethoxypyrimidin-2-yl |
| 1034 | OH | $C_2H_5$ | 4,6-Dimethoxypyrimidin-2-yl |
| 1035 | Phenyl | H | 2-Thienyl |
| 1036 | Phenyl | $CH_3$ | 2-Thienyl |
| 1037 | H | $CH_3$ | 2-Thienyl |
| 1038 | $CH_3$ | H | 2-Thienyl |
| 1039 | OH | H | 2-Thienyl |
| 1040 | $C(CH_3)_3$ | H | 2-Thienyl |
| 1041 | $CF_3$ | H | 2-Thienyl |
| 1042 | $CH(CH_3)_2$ | H | 2-Thienyl |
| 1043 | 2-Furyl | H | 2-Thienyl |
| 1044 | Cyclohexyl | H | 2-Thienyl |
| 1045 | Cyclobutyl | H | 2-Thienyl |
| 1046 | 4-Methylphenyl | H | 2-Thienyl |
| 1047 | 2-Methylphenyl | H | 2-Thienyl |
| 1048 | 2-Fluorphenyl | H | 2-Thienyl |
| 1049 | 3-Fluorphenyl | H | 2-Thienyl |
| 1050 | 4-Fluorphenyl | H | 2-Thienyl |
| 1051 | OH | $CH_3$ | 2-Thienyl |
| 1052 | OH | $CF_3$ | 2-Thienyl |
| 1053 | OH | F | 2-Thienyl |

(fortgesetzt)

| | $R^1$ | $R^2$ | Q |
|---|---|---|---|
| 1054 | OH | CN | 2-Thienyl |
| 1055 | OH | Cl | 2-Thienyl |
| 1056 | OH | $C_2H_5$ | 2-Thienyl |
| 1057 | Phenyl | H | 2-Furyl |
| 1058 | Phenyl | $CH_3$ | 2-Furyl |
| 1059 | H | $CH_3$ | 2-Furyl |
| 1060 | $CH_3$ | H | 2-Furyl |
| 1061 | OH | H | 2-Furyl |
| 1062 | $C(CH_3)_3$ | H | 2-Furyl |
| 1063 | $CF_3$ | H | 2-Furyl |
| 1064 | $CH(CH_3)_2$ | H | 2-Furyl |
| 1065 | 2-Furyl | H | 2-Furyl |
| 1066 | Cyclohexyl | H | 2-Furyl |
| 1067 | Cyclobutyl | H | 2-Furyl |
| 1068 | 4-Methylphenyl | H | 2-Furyl |
| 1069 | 2-Methylphenyl | H | 2-Furyl |
| 1070 | 2-Fluorphenyl | H | 2-Furyl |
| 1071 | 3-Fluorphenyl | H | 2-Furyl |
| 1072 | 4-Fluorphenyl | H | 2-Furyl |
| 1073 | OH | $CH_3$ | 2-Furyl |
| 1074 | OH | $CF_3$ | 2-Furyl |
| 1075 | OH | F | 2-Furyl |
| 1076 | OH | CN | 2-Furyl |
| 1077 | OH | Cl | 2-Furyl |
| 1078 | OH | $C_2H_5$ | 2-Furyl |
| 1079 | Phenyl | H | Benzimidazol-2-yl |
| 1080 | Phenyl | $CH_3$ | Benzimidazol-2-yl |
| 1081 | H | $CH_3$ | Benzimidazol-2-yl |
| 1082 | $CH_3$ | H | Benzimidazol-2-yl |
| 1083 | OH | H | Benzimidazol-2-yl |
| 1084 | $C(CH_3)_3$ | H | Benzimidazol-2-yl |
| 1085 | $CF_3$ | H | Benzimidazol-2-yl |
| 1086 | $CH(CH_3)_2$ | H | Benzimidazol-2-yl |
| 1087 | 2-Furyl | H | Benzimidazol-2-yl |
| 1088 | Cyclohexyl | H | Benzimidazol-2-yl |
| 1089 | Cyclobutyl | H | Benzimidazol-2-yl |
| 1090 | 4-Methylphenyl | H | Benzimidazol-2-yl |
| 1091 | 2-Methylphenyl | H | Benzimidazol-2-yl |

(fortgesetzt)

| | $R^1$ | $R^2$ | Q |
|---|---|---|---|
| 1092 | 2-Fluorphenyl | H | Benzimidazol-2-yl |
| 1093 | 3-Fluorphenyl | H | Benzimidazol-2-yl |
| 1094 | 4-Fluorphenyl | H | Benzimidazol-2-yl |
| 1095 | OH | $CH_3$ | Benzimidazol-2-yl |
| 1096 | OH | $CF_3$ | Benzimidazol-2-yl |
| 1097 | OH | F | Benzimidazol-2-yl |
| 1098 | OH | CN | Benzimidazol-2-yl |
| 1099 | OH | Cl | Benzimidazol-2-yl |
| 1100 | OH | $C_2H_5$ | Benzimidazol-2-yl |
| 1101 | Phenyl | H | Benzoxazol-2-yl |
| 1102 | Phenyl | $CH_3$ | Benzoxazol-2-yl |
| 1103 | H | $CH_3$ | Benzoxazol-2-yl |
| 1104 | $CH_3$ | H | Benzoxazol-2-yl |
| 1105 | OH | H | Benzoxazol-2-yl |
| 1106 | $C(CH_3)_3$ | H | Benzoxazol-2-yl |
| 1107 | $CF_3$ | H | Benzoxazol-2-yl |
| 1108 | $CH(CH_3)_2$ | H | Benzoxazol-2-yl |
| 1109 | 2-Furyl | H | Benzoxazol-2-yl |
| 1110 | Cyclohexyl | H | Benzoxazol-2-yl |
| 1111 | Cyclobutyl | H | Benzoxazol-2-yl |
| 1112 | 4-Methylphenyl | H | Benzoxazol-2-yl |
| 1113 | 2-Methylphenyl | H | Benzoxazol-2-yl |
| 1114 | 2-Fluorphenyl | H | Benzoxazol-2-yl |
| 1115 | 3-Fluorphenyl | H | Benzoxazol-2-yl |
| 1116 | 4-Fluorphenyl | H | Benzoxazol-2-yl |
| 1117 | OH | $CH_3$ | Benzoxazol-2-yl |
| 1118 | OH | $CF_3$ | Benzoxazol-2-yl |
| 1119 | OH | F | Benzoxazol-2-yl |
| 1120 | OH | CN | Benzoxazol-2-yl |
| 1121 | OH | Cl | Benzoxazol-2-yl |
| 1122 | OH | $C_2H_5$ | Benzoxazol-2-yl |
| 1123 | Phenyl | H | Benzothiazol-2-yl |
| 1124 | Phenyl | $CH_3$ | Benzothiazol-2-yl |
| 1125 | H | $CH_3$ | Benzothiazol-2-yl |
| 1126 | $CH_3$ | H | Benzothiazol-2-yl |
| 1127 | OH | H | Benzothiazol-2-yl |
| 1128 | $C(CH_3)_3$ | H | Benzothiazol-2-yl |
| 1129 | $CF_3$ | H | Benzothiazol-2-yl |

(fortgesetzt)

| | $R^1$ | $R^2$ | Q |
|---|---|---|---|
| 1130 | $CH(CH_3)_2$ | H | Benzothiazol-2-yl |
| 1131 | 2-Furyl | H | Benzothiazol-2-yl |
| 1132 | Cyclohexyl | H | Benzothiazol-2-yl |
| 1133 | Cyclobutyl | H | Benzothiazol-2-yl |
| 1134 | 4-Methylphenyl | H | Benzothiazol-2-yl |
| 1135 | 2-Methylphenyl | H | Benzothiazol-2-yl |
| 1136 | 2-Fluorphenyl | H | Benzothiazol-2-yl |
| 1137 | 3-Fluorphenyl | H | Benzothiazol-2-yl |
| 1138 | 4-Fluorphenyl | H | Benzothiazol-2-yl |
| 1139 | OH | $CH_3$ | Benzothiazol-2-yl |
| 1140 | OH | $CF_3$ | Benzothiazol-2-yl |
| 1141 | OH | F | Benzothiazol-2-yl |
| 1142 | OH | CN | Benzothiazol-2-yl |
| 1143 | OH | Cl | Benzothiazol-2-yl |
| 1144 | OH | $C_2H_5$ | Benzothiazol-2-yl |
| 1145 | Phenyl | H | 2-Chlorthiazol-5-yl |
| 1146 | Phenyl | $CH_3$ | 2-Chlorthiazol-5-yl |
| 1147 | H | $CH_3$ | 2-Chlorthiazol-5-yl |
| 1148 | $CH_3$ | H | 2-Chlorthiazol-5-yl |
| 1149 | OH | H | 2-Chlorthiazol-5-yl |
| 1150 | $C(CH_3)_3$ | H | 2-Chlorthiazol-5-yl |
| 1151 | $CF_3$ | H | 2-Chlorthiazol-5-yl |
| 1152 | $CH(CH_3)_2$ | H | 2-Chlorthiazol-5-yl |
| 1153 | 2-Furyl | H | 2-Chlorthiazol-5-yl |
| 1154 | Cyclohexyl | H | 2-Chlorthiazol-5-yl |
| 1155 | Cyclobutyl | H | 2-Chlorthiazol-5-yl |
| 1156 | 4-Methylphenyl | H | 2-Chlorthiazol-5-yl |
| 1157 | 2-Methylphenyl | H | 2-Chlorthiazol-5-yl |
| 1158 | 2-Fluorphenyl | H | 2-Chlorthiazol-5-yl |
| 1159 | 3-Fluorphenyl | H | 2-Chlorthiazol-5-yl |
| 1160 | 4-Fluorphenyl | H | 2-Chlorthiazol-5-yl |
| 1161 | OH | $CH_3$ | 2-Chlorthiazol-5-yl |
| 1162 | OH | $CF_3$ | 2-Chlorthiazol-5-yl |
| 1163 | OH | F | 2-Chlorthiazol-5-yl |
| 1164 | OH | CN | 2-Chlorthiazol-5-yl |
| 1165 | OH | Cl | 2-Chlorthiazol-5-yl |
| 1166 | OH | $C_2H_5$ | 2-Chlorthiazol-5-yl |
| 1167 | Phenyl | H | 6-Chlorpyridin-2-yl |

(fortgesetzt)

| | $R^1$ | $R^2$ | Q |
|---|---|---|---|
| 1168 | Phenyl | $CH_3$ | 6-Chlorpyridin-2-yl |
| 1169 | H | $CH_3$ | 6-Chlorpyridin-2-yl |
| 1170 | $CH_3$ | H | 6-Chlorpyridin-2-yl |
| 1171 | OH | H | 6-Chlorpyridin-2-yl |
| 1172 | $C(CH_3)_3$ | H | 6-Chlorpyridin-2-y! |
| 1173 | $CF_3$ | H | 6-Chlorpyridin-2-yl |
| 1174 | $CH(CH_3)_2$ | H | 6-Chlorpyridin-2-yl |
| 1175 | 2-Furyl | H | 6-Chlorpyridin-2-yl |
| 1176 | Cyclohexyl | H | 6-Chlorpyridin-2-yl |
| 1177 | Cyclobutyl | H | 6-Chlorpyridin-2-yl |
| 1178 | 4-Methylphenyl | H | 6-Chlorpyridin-2-yl |
| 1179 | 2-Methylphenyl | H | 6-Chlorpyridin-2-yl |
| 1180 | 2-Fluorphenyl | H | 6-Chlorpyridin-2-yl |
| 1181 | 3-Fluorphenyl | H | 6-Chlorpyridin-2-yl |
| 1182 | 4-Fluorphenyl | H | 6-Chlorpyridin-2-yl |
| 1183 | OH | $CH_3$ | 6-Chlorpyridin-2-yl |
| 1184 | OH | $CF_3$ | 6-Chlorpyridin-2-yl |
| 1185 | OH | F | 6-Chlorpyridin-2-yl |
| 1186 | OH | CN | 6-Chlorpyridin-2-yl |
| 1187 | OH | Cl | 6-Chlorpyridin-2-yl |
| 1188 | OH | $C_2H_5$ | 6-Chlorpyridin-2-yl |
| 1189 | Phenyl | H | 1-Methylimidazol-2-yl |
| 1190 | Phenyl | $CH_3$ | 1-Methylimidazol-2-yl |
| 1191 | H | $CH_3$ | 1-Methylimidazol-2-yl |
| 1192 | $CH_3$ | H | 1-Methylimidazol-2-yl |
| 1193 | OH | H | 1-Methylimidazol-2-yl |
| 1194 | $C(CH_3)_3$ | H | 1-Methylimidazol-2-yl |
| 1195 | $CF_3$ | H | 1-Methylimidazol-2-yl |
| 1196 | $CH(CH_3)_2$ | H | 1-Methylimidazol-2-yl |
| 1197 | 2-Furyl | H | 1-Methylimidazol-2-yl |
| 1198 | Cyclohexyl | H | 1-Methylimidazol-2-yl |
| 1199 | Cyclobutyl | H | 1-Methylimidazol-2-yl |
| 1200 | 4-Methylphenyl | H | 1-Methylimidazol-2-yl |
| 1201 | 2-Methylphenyl | H | 1-Methylimidazol-2-yl |
| 1202 | 2-Fluorphenyl | H | 1-Methylimidazol-2-yl |
| 1203 | 3-Fluorphenyl | H | 1-Methylimidazol-2-yl |
| 1204 | 4-Fluorphenyl | H | 1-Methylimidazol-2-yl |
| 1205 | OH | $CH_3$ | 1-Methylimidazol-2-yl |

(fortgesetzt)

| | $R^1$ | $R^2$ | Q |
|---|---|---|---|
| 1206 | OH | $CF_3$ | 1-Methylimidazol-2-yl |
| 1207 | OH | F | 1-Methylimidazol-2-y! |
| 1208 | OH | CN | 1-Methylimidazol-2-yl |
| 1209 | OH | Cl | 1-Methylimidazol-2-yl |
| 1210 | OH | $C_2H_5$ | 1-Methylimidazol-2-yl |
| 1211 | Phenyl | H | 2-Methylthiazol-5-yl |
| 1212 | Phenyl | $CH_3$ | 2-Methylthiazol-5-yl |
| 1213 | H | $CH_3$ | 2-Methylthiazol-5-yl |
| 1214 | $CH_3$ | H | 2-Methylthiazol-5-yl |
| 1215 | OH | H | 2-Methylthiazol-5-yl |
| 1216 | $C(CH_3)_3$ | H | 2-Methylthiazol-5-yl |
| 1217 | $CF_3$ | H | 2-Methylthiazol-5-yl |
| 1218 | $CH(CH_3)_2$ | H | 2-Methylthiazol-5-yl |
| 1219 | 2-Furyl | H | 2-Methylthiazol-5-yl |
| 1220 | Cyclohexyl | H | 2-Methylthiazol-5-yl |
| 1221 | Cyclobutyl | H | 2-Methylthiazol-5-yl |
| 1222 | 4-Methylphenyl | H | 2-Methylthiazol-5-yl |
| 1223 | 2-Methylphenyl | H | 2-Methylthiazol-5-yl |
| 1224 | 2-Fluorphenyl | H | 2-Methylthiazol-5-yl |
| 1225 | 3-Fluorphenyl | H | 2-Methylthiazol-5-yl |
| 1226 | 4-Fluorphenyl | H | 2-Methylthiazol-5-yl |
| 1227 | OH | $CH_3$ | 2-Methylthiazol-5-yl |
| 1228 | OH | $CF_3$ | 2-Methylthiazol-5-yl |
| 1229 | OH | F | 2-Methylthiazol-5-yl |
| 1230 | OH | CN | 2-Methylthiazol-5-yl |
| 1231 | OH | Cl | 2-Methylthiazol-5-yl |
| 1232 | OH | $C_2H_5$ | 2-Methylthiazol-5-yl |
| 1233 | Phenyl | H | 2-Methyl-$\alpha$-naphthyl |
| 1234 | Phenyl | $CH_3$ | 2-Methyl-$\alpha$-naphthyl |
| 1235 | H | $CH_3$ | 2-Methyl-$\alpha$-naphthyl |
| 1236 | $CH_3$ | H | 2-Methyl-$\alpha$-naphthyl |
| 1237 | OH | H | 2-Methyl-$\alpha$-naphthyl |
| 1238 | $C(CH_3)_3$ | H | 2-Methyl-$\alpha$-naphthyl |
| 1239 | $CF_3$ | H | 2-Methyl-$\alpha$-naphthyl |
| 1240 | $CH(CH_3)_2$ | H | 2-Methyl-$\alpha$-naphthyl |
| 1241 | 2-Furyl | H | 2-Methyl-$\alpha$-naphthyl |
| 1242 | Cyclohexyl | H | 2-Methyl-$\alpha$-naphthyl |
| 1243 | Cyclobutyl | H | 2-Methyl-$\alpha$-naphthyl |

(fortgesetzt)

| | $R^1$ | $R^2$ | Q |
|---|---|---|---|
| 1244 | 4-Methylphenyl | H | 2-Methyl-$\alpha$-naphthyl |
| 1245 | 2-Methylphenyl | H | 2-Methyl-$\alpha$-naphthyl |
| 1246 | 2-Fluorphenyl | H | 2-Methyl-$\alpha$-naphthyl |
| 1247 | 3-Fluorphenyl | H | 2-Methyl-$\alpha$-naphthyl |
| 1248 | 4-Fluorphenyl | H | 2-Methyl-$\alpha$-naphthyl |
| 1249 | OH | $CH_3$ | 2-Methyl-$\alpha$-naphthyl |
| 1250 | OH | $CF_3$ | 2-Methyl-$\alpha$-naphthyl |
| 1251 | OH | F | 2-Methyl-$\alpha$-naphthyl |
| 1252 | OH | CN | 2-Methyl-$\alpha$-naphthyl |
| 1253 | OH | Cl | 2-Methyl-$\alpha$-naphthyl |
| 1254 | OH | $C_2H_5$ | 2-Methyl-$\alpha$-naphthyl |
| 1255 | Phenyl | H | $\alpha$-naphthyl |
| 1256 | Phenyl | $CH_3$ | $\alpha$-Naphthyl |
| 1257 | H | $CH_3$ | $\alpha$-Naphthyl |
| 1258 | $CH_3$ | H | $\alpha$-Naphthyl |
| 1259 | OH | H | $\alpha$-Naphthyl |
| 1260 | $C(CH_3)_3$ | H | $\alpha$-Naphthyl |
| 1261 | $CF_3$ | H | $\alpha$-Naphthyl |
| 1262 | $CH(CH_3)_2$ | H | $\alpha$-Naphthyl |
| 1263 | 2-Furyl | H | $\alpha$-Naphthyl |
| 1264 | Cyclohexyl | H | $\alpha$-Naphthyl |
| 1265 | Cyclobutyl | H | $\alpha$-Naphthyl |
| 1266 | 4-Methylphenyl | H | $\alpha$-Naphthyl |
| 1267 | 2-Methylphenyl | H | $\alpha$-Naphthyl |
| 1268 | 2-Fluorphenyl | H | $\alpha$-Naphthyl |
| 1269 | 3-Fluorphenyl | H | $\alpha$-Naphthyl |
| 1270 | 4-Fluorphenyl | H | $\alpha$-Naphthyl |
| 1271 | OH | $CH_3$ | $\alpha$-Naphthyl |
| 1272 | OH | $CF_3$ | $\alpha$-Naphthyl |
| 1273 | OH | F | $\alpha$-Naphthyl |
| 1274 | OH | CN | $\alpha$-naphthyl |
| 1275 | OH | Cl | $\alpha$-Naphthyl |
| 1276 | OH | $C_2H_5$ | $\alpha$-Naphthyl |
| 1277 | Phenyl | H | Benzo-1,3-dioxol-5-yl |
| 1278 | Phenyl | $CH_3$ | Benzo-1,3-dioxol-5-yl |
| 1279 | H | $CH_3$ | Benzo-1,3-dioxol-5-yl |
| 1280 | $CH_3$ | H | Benzo-1,3-dioxol-5-yl |
| 1281 | OH | H | Benzo-1,3-dioxol-5-yl |

(fortgesetzt)

| | $R^1$ | $R^2$ | Q |
|---|---|---|---|
| 1282 | $C(CH_3)_3$ | H | Benzo-1,3-dioxol-5-yl |
| 1283 | $CF_3$ | H | Benzo-1,3-dioxol-5-yl |
| 1284 | $CH(CH_3)_2$ | H | Benzo-1,3-dioxol-5-yl |
| 1285 | 2-Furyl | H | Benzo-1,3-dioxol-5-yl |
| 1286 | Cyclohexyl | H | Benzo-1,3-dioxol-5-yl |
| 1287 | Cyclobutyl | H | Benzo-1,3-dioxol-5-yl |
| 1288 | 4-Methylphenyl | H | Benzo-1,3-dioxol-5-yl |
| 1289 | 2-Methylphenyl | H | Benzo-1,3-dioxol-5-yl |
| 1290 | 2-Fluorphenyl | H | Benzo-1,3-dioxol-5-yl |
| 1291 | 3-Fluorphenyl | H | Benzo-1,3-dioxol-5-yl |
| 1292 | 4-Fluorphenyl | H | Benzo-1,3-dioxol-5-yl |
| 1293 | OH | $CH_3$ | Benzo-1,3-dioxol-5-yl |
| 1294 | OH | $CF_3$ | Benzo-1,3-dioxol-5-yl |
| 1295 | OH | F | Benzo-1,3-dioxol-5-yl |
| 1296 | OH | CN | Benzo-1,3-dioxol-5-yl |
| 1297 | OH | Cl | Benzo-1,3-dioxol-5-yl |
| 1298 | OH | $C_2H_5$ | Benzo-1,3-dioxol-5-yl |
| 1299 | Phenyl | H | 2-Cyanophenyl |
| 1300 | Phenyl | $CH_3$ | 2-Cyanophenyl |
| 1301 | H | $CH_3$ | 2-Cyanophenyl |
| 1302 | $CH_3$ | H | 2-Cyanophenyl |
| 1303 | OH | H | 2-Cyanophenyl |
| 1304 | $C(CH_3)_3$ | H | 2-Cyanophenyl |
| 1305 | $CF_3$ | H | 2-Cyanophenyl |
| 1306 | $CH(CH_3)_2$ | H | 2-Cyanophenyl |
| 1307 | 2-Furyl | H | 2-Cyanophenyl |
| 1308 | Cyclohexyl | H | 2-Cyanophenyl |
| 1309 | Cyclobutyl | H | 2-Cyanophenyl |
| 1310 | 4-Methylphenyl | H | 2-Cyanophenyl |
| 1311 | 2-Methylphenyl | H | 2-Cyanophenyl |
| 1312 | 2-Fluorphenyl | H | 2-Cyanophenyl |
| 1313 | 3-Fluorphenyl | H | 2-Cyanophenyl |
| 1314 | 4-Fluorphenyl | H | 2-Cyanophenyl |
| 1315 | OH | $CH_3$ | 2-Cyanophenyl |
| 1316 | OH | $CF_3$ | 2-Cyanophenyl |
| 1317 | OH | F | 2-Cyanophenyl |
| 1318 | OH | CN | 2-Cyanophenyl |
| 1319 | OH | Cl | 2-Cyanophenyl |

(fortgesetzt)

| | $R^1$ | $R^2$ | Q |
|---|---|---|---|
| 1320 | OH | $C_2H_5$ | 2-Cyanophenyl |
| 1321 | Phenyl | H | 4-Cyanophenyl |
| 1322 | Phenyl | $CH_3$ | 4-Cyanophenyl |
| 1323 | H | $CH_3$ | 4-Cyanophenyl |
| 1324 | $CH_3$ | H | 4-Cyanophenyl |
| 1325 | OH | H | 4-Cyanophenyl |
| 1326 | $C(CH_3)_3$ | H | 4-Cyanophenyl |
| 1327 | $CF_3$ | H | 4-Cyanophenyl |
| 1328 | $CH(CH_3)_2$ | H | 4-Cyanophenyl |
| 1329 | 2-Furyl | H | 4-Cyanophenyl |
| 1330 | Cyclohexyl | H | 4-Cyanophenyl |
| 1331 | Cyclobutyl | H | 4-Cyanophenyl |
| 1332 | 4-Methylphenyl | H | 4-Cyanophenyl |
| 1333 | 2-Methylphenyl | H | 4-Cyanophenyl |
| 1334 | 2-Fluorphenyl | H | 4-Cyanophenyl |
| 1335 | 3-Fluorphenyl | H | 4-Cyanophenyl |
| 1336 | 4-Fluorphenyl | H | 4-Cyanophenyl |
| 1337 | OH | $CH_3$ | 4-Cyanophenyl |
| 1338 | OH | $CF_3$ | 4-Cyanophenyl |
| 1339 | OH | F | 4-Cyanophenyl |
| 1340 | OH | CN | 4-Cyanophenyl |
| 1341 | OH | Cl | 4-Cyanophenyl |
| 1342 | OH | $C_2H_5$ | 4-Cyanophenyl |
| 1343 | Phenyl | H | 3-Trifluormethylphenyl |
| 1344 | Phenyl | $CH_3$ | 3-Trifluormethylphenyl |
| 1345 | H | $CH_3$ | 3-Trifluormethylphenyl |
| 1346 | $CH_3$ | H | 3-Trifluormethylphenyl |
| 1347 | OH | H | 3-Trifluormethylphenyl |
| 1348 | $C(CH_3)_3$ | H | 3-Trifluormethylphenyl |
| 1349 | $CF_3$ | H | 3-Trifluormethylphenyl |
| 1350 | $CH(CH_3)_2$ | H | 3-Trifluormethylphenyl |
| 1351 | 2-Furyl | H | 3-Trifluormethylphenyl |
| 1352 | Cyclohexyl | H | 3-Trifluormethylphenyl |
| 1353 | Cyclobutyl | H | 3-Trifluormethylphenyl |
| 1354 | 4-Methylphenyl | H | 3-Trifluormethylphenyl |
| 1355 | 2-Methylphenyl | H | 3-Trifluormethylphenyl |
| 1356 | 2-Fluorphenyl | H | 3-Trifluormethylphenyl |
| 1357 | 3-Fluorphenyl | H | 3-Trifluormethylphenyl |

(fortgesetzt)

| | $R^1$ | $R^2$ | Q |
|---|---|---|---|
| 1358 | 4-Fluorphenyl | H | 3-Trifluormethylphenyl |
| 1359 | OH | $CH_3$ | 3-Trifluormethylphenyl |
| 1360 | OH | $CF_3$ | 3-Trifluormethylphenyl |
| 1361 | OH | F | 3-Trifluormethylphenyl |
| 1362 | OH | CN | 3-Trifluormethylphenyl |
| 1363 | OH | Cl | 3-Trifluormethylphenyl |
| 1364 | OH | $C_2H_5$ | 3-Trifluormethylphenyl |
| 1365 | Phenyl | H | 4-Methoxycarbonylphenyl |
| 1366 | Phenyl | $CH_3$ | 4-Methoxycarbonylphenyl |
| 1367 | H | $CH_3$ | 4-Methoxycarbonylphenyl |
| 1368 | $CH_3$ | H | 4-Methoxycarbonylphenyl |
| 1369 | OH | H | 4-Methoxycarbonylphenyl |
| 1370 | $C(CH_3)_3$ | H | 4-Methoxycarbonylphenyl |
| 1371 | $CF_3$ | H | 4-Methoxycarbonylphenyl |
| 1372 | $CH(CH_3)_2$ | H | 4-Methoxycarbonylphenyl |
| 1373 | 2-Furyl | H | 4-Methoxycarbonylphenyl |
| 1374 | Cyclohexyl | H | 4-Methoxycarbonylphenyl |
| 1375 | Cyclobutyl | H | 4-Methoxycarbonylphenyl |
| 1376 | 4-Methylphenyl | H | 4-Methoxycarbonylphenyl |
| 1377 | 2-Methylphenyl | H | 4-Methoxycarbonylphenyl |
| 1378 | 2-Fluorphenyl | H | 4-Methoxycarbonylphenyl |
| 1379 | 3-Fluorphenyl | H | 4-Methoxycarbonylphenyl |
| 1380 | 4-Fluorphenyl | H | 4-Methoxycarbonylphenyl |
| 1381 | OH | $CH_3$ | 4-Methoxycarbonylphenyl |
| 1382 | OH | $CF_3$ | 4-Methoxycarbonylphenyl |
| 1383 | OH | F | 4-Methoxycarbonylphenyl |
| 1384 | OH | CN | 4-Methoxycarbonylphenyl |
| 1385 | OH | Cl | 4-Methoxycarbonylphenyl |
| 1386 | OH | $C_2H_5$ | 4-Methoxycarbonylphenyl |

Die Herstellung der erfindungsgemäßen substituierten N-heterocyclischen Verbindungen I erfolgt analog zu literaturbekannten Methoden. Ein wichtiger Zugang zu den erfindungsgemäßen Verbindungen I.A ist in Schema 1 dargestellt.

**Schema 1:**

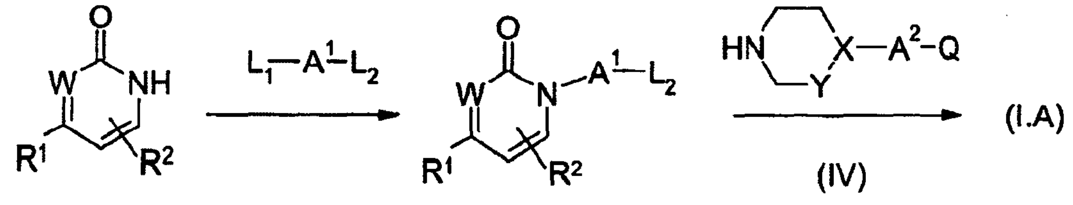


(II) (III)

In Schema 1 haben W, $R^1$, $R^2$, $A^2$, und Q die zuvor genannten Bedeutungen. $A^1$ steht für $(CH_2)_n$, X für N und Y für $CH_2$. $L_1$ und $L_2$ stehen für nucleophil verdrängbare Abgangsgruppen. Beispiele für geeignete nucleophil verdrängbare Abgangsgruppen sind Halogen, insbesondere Chlor, Brom oder Iod, Alkyl- und Arylsulfonat wie Mesylat, Tosylat. $L_1$ und $L_2$ sind vorzugsweise voneinander verschieden sind und weisen eine unterschiedliche Reaktivität auf. Beispielsweise steht $L_1$ für Brom oder Iod und $L_2$ für Chlor. Die für die Umsetzung erforderlichen Reaktionsbedingungen entsprechen den für nucleophile Substitutionen üblichen Reaktionsbedingungen.

Verbindungen der allgemeinen Formel IV sind z.T. kommerziell erhältlich und/oder literaturbekannt, z.B. aus Chem. Pharm. Bull. 1987, 35 (7), 2782-91; Bioorganical and Medicinal Chemistry Letters (BOMCL) 2002,12 (8), 1149-52; J. Med. Chem. 1998, 33 (5), 339-47; J. Med. Chem. 1989, 32 (3), 593-7; JCS. Perkin Trans 1: Org. and Bioorg. Chem. 1998, 15,2239-42; Chem. Pharm. Bull. 1988, 36 (12), 4825-33.

Die Pyrimidinon-Verbindungen der Formeln II (mit W = N) sind bekannt und teilweise kommerziell erhältlich oder können nach bekannten Verfahren zur Pyrimidinonsynthese hergestellt werden, wie z. B. beschrieben in Austr. J. Chem. 1968, 221, S. 243-255; J. Med. Chem. 1978, 21, S. 623-628; Tetrahedron Lett. 1986, 27, S. 2611-2612; Chemiker Ztg. 1977, 6, S. 305.

Die Pyridinon-Verbindungen der Formeln II (mit W = CH bzw. $CR^1$) sind bekannt und teilweise kommerziell erhältlich oder können nach bekannten Verfahren zur Pyridinonsynthese hergestellt werden, wie z. B. beschrieben in J. Med. Chem. 16(5), 1973, S. 524-528, J. Org. Chem., 67, 2002, S. 4304-4308, Bioorg. Med. Chem. Lett, 12, 2002, S. 3537-3541.

Die Verbindungen II können auch nach den in den Schemata 2, 3 und 4 angegebenen Methoden hergestellt werden.

So können die Verbindungen der Formel II, sofern $R^1$ für gegebenenfalls substituiertes Alkenyl, gegebenenfalls substituiertes Phenyl oder gegebenenfalls substituiertes, C-gebundenes Heteroaryl steht, gemäß der in Schema 2 gezeigten Route via Suzuki-Kupplung hergestellt werden.

Schema 2:

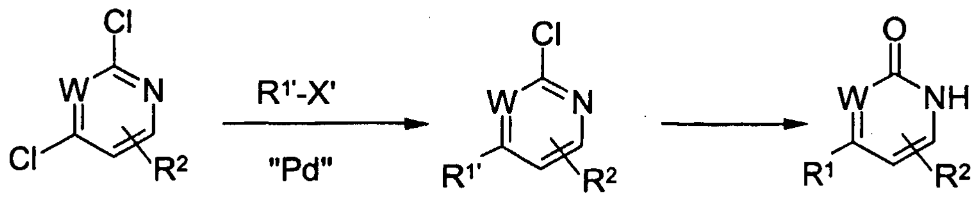


(V) (VI) (II)

In Schema 2 hat W die zuvor angegebenen Bedeutungen. $R^{1'}$ steht für gegebenenfalls substituiertes Alkenyl, gegebenenfalls substituiertes Phenyl oder gegebenenfalls substituiertes, C-gebundenes Heteroaryl. X' steht für eine Gruppe $B(OH)_2$, $B(OR)_2$ oder für den von dem entsprechenden Boronsäureanhydrid abgeleiteten Rest $(BO)_3/3$. "Pd" steht für einen Palladium(0)komplex, der vorzugsweise 4 Trialkylphosphin- oder Triarylphosphin-Liganden aufweist. $R^2$ hat die zuvor angegebenen Bedeutungen und steht insbesondere für Wasserstoff oder $C_1$-$C_4$-Alkyl.

Die Kupplung von V mit der Verbindung $R^{1'}$-X' erfolgt unter den Bedingungen einer Suzuki-Kupplung (Übersicht siehe A. Suzuki et al. in Chem. Rev. 1995, 95, S. 2457-2483). Die für die Suzuki-Kupplung von 2,4-Dichlorpyrimidinen V mit $R^{1'}$-X' erforderlichen Reaktionsbedingungen sind aus der Literatur bekannt, z.B. aus J. Org. Chem. 66(21) (2001), S. 7124-7128. Das dabei erhaltene 2-Chlorpyrimidin VI kann in an sich bekannter Weise, z.B. unter den in Acta Chem. Scand. B, 1984, 38, S. 505-508 angegebenen Bedingungen, in das entsprechende 2-Pyrimidinon II umgewandelt werden.

Ferner können die Verbindungen der Formel II, worin W = N und $R^1$ für gegebenenfalls substituiertes $C_1$-$C_6$-Alkyl

oder $C_3$-$C_6$-Cycloalkyl, insbesondere für Ethyl, Isopropyl, Cyclopropyl, Cyclobutyl, Cyclohexyl, steht, und $R^2$ für H steht, beispielsweise nach dem im Schema 3 gezeigten Verfahren hergestellt werden.

Schema 3:

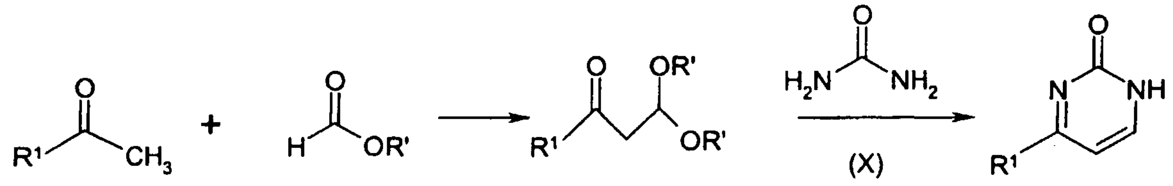


(VII) (VIII) (IX) {II: W = N}

In Schema 3 steht R' beispielsweise für $C_1$-$C_4$-Alkyl. In Schema 3 wird man zunächst ein Keton VII mit einem Ameisensäureester VIII, z. B. Ameisensäuremethylester, in an sich bekannter Weise in das Ketal IX überführen (siehe Helv. Chim. Acta 2002, 85, 2926-2929, Bsp. 6). Üblicherweise führt man die Umsetzung in Gegenwart einer Base wie einem Alkoholat in einem inerten Lösungsmittel wie einem Ether durch. Die Umsetzung des erhaltenen Ketals IX mit Harnstoff X unter Bildung des entsprechenden 2-Pyrimidinons II erfolgt unter literaturbekannten Bedingungen, z. B. gemäß Aust. J. Chem. 1968, 21, 243-55 (insbesondere S. 252).

Die Herstellung der 2-Pyri(mi)dinone II, worin $R^1$ für Wasserstoff steht und $R^2$ für gegebenenfalls substituiertes Phenyl steht, kann beispielsweise nach dem im Schema 4 gezeigten Verfahren erfolgen.

Schema 4:

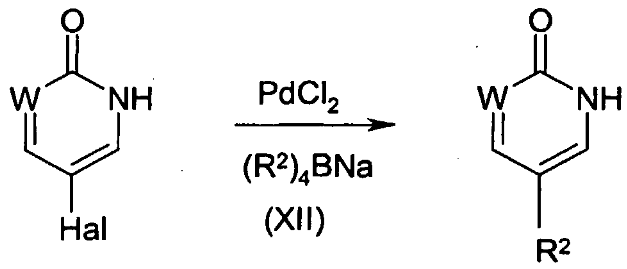


(XI) (II)

In Schema 4 hat W die zuvor angegebenen Bedeutungen. Hal steht für Halogen, insbesondere für Brom oder Chlor. Die Kupplung des Halogenpyrimidinons XI mit dem Borat XII erfolgt unter Suzuki-Bedingungen (siehe Tetrahedron 1997, 53, 14437-50). Die modifizierte Suzuki-Kreuzkupplung zwischen dem Pyridinon XI und dem Borat XII erfolgt üblicherweise in wässrigen Lösungsmitteln in Gegenwart eines phosphinfreien Pd-Katalysators wie Palladium(II)-chlorid und in Gegenwart einer Base. Geeignete Basen sind beispielsweise Alkalimetallhydroxide wie Natriumhydroxid. Die Pyridinone XI und die Borate XII sind literaturbekannt und kommerziell erhältlich.

Die Herstellung der erfindungsgemäßen Pyri(mi)dinonverbindungen I.A, worin $R^1$ für $NR^4R^5$ steht, kann beispielsweise nach dem im Schema 5 gezeigten Verfahren erfolgen.

Schema 5:

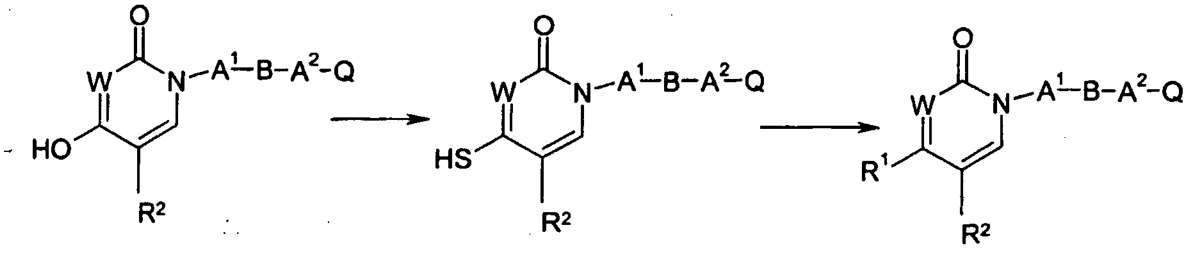

I: {$R^2$ = H, $CH_3$}  I: {$R^2$ = H, $CH_3$}

In Schema 5 haben W, $A^1$, B, $A^2$ und Q die zuvor genannten Bedeutungen. Gemäß Schema 5 wird man zunächst die Verbindung I.A, worin $R^1$ für OH steht, in das entsprechende Thiol I mit $R^1$ = SH überführen. Beispiele für geeignete Schwefelungsmittel sind Organophosphorsulfide wie das Lawesson-Reagenz, Organozinnsulfide oder Phosphor(V)-sulfid. Ein bevorzugtes Schwefelungsmittel ist Phosphorpentasulfid ($P_4S_{10}$). Die für die Thionierung erforderlichen Bedingungen sind dem Fachmann bekannt, z. B. aus J. Med. Chem. 1984, 27, 1470-80 (insbesondere S. 1478, Beispiel 8b). Das hierbei erhaltene Thiol I mit $R^1$ = SH kann dann durch Umsetzung mit einer Verbindung der Formel $HNR^4R^5$, worin $R^4$ und $R^5$ die zuvor genannten Bedeutungen aufweisen, in andere Verbindungen I.A mit $R^1$ = $NR^4R^5$ überführt werden. Die Umsetzung erfolgt in der Regel in einem inerten Lösungsmittel. Die zur Umsetzung erforderliche Aktivierungsenergie kann mittels Mikrowellen in die Reaktionsmischung eingetragen werden (zur Umsetzung unter Einsatz von Mikrowellen, siehe Tetrahedron 2001, 57, S. 9199 ff, S. 9225 ff sowie allgemein "Microwaves in Organic Synthesis", André Loupy (Hrsg.), Wiley-VCH 2002).

In den Verbindungen I.A mit $R^1$ = SH kann die Thiolgruppe nach Standardverfahren der organischen Chemie in andere Reste $R^1$ umgewandelt werden. Eine Übersicht gibt das Schema 6.

Schema 6:

I: {$R^1$ = SH} → I: {$R^1$ = $SR^6$, $S(O)R^6$, $S(O)_2R^6$, $S(O)_2NR^4R^5$, OH, $OR^{3a}$, $OC(O)NR^4R5$, $OC(O)R^8$}

I: {$R^1$ = SH} ↓ I: {$R^1$ = SCN}

I: {$R^1$ = SCN} → I: {$R^1$ = $NR^4R^5$, $OR^{3a}$}

Verfahren hierzu sind dem Fachmann bekannt und umfassen die Umwandlung von SH in $SR^6$ via Alkylierung, die Oxidation von $SR^6$ zu den korrespondierenden Gruppen $SOR^6$ und $SO_2R^6$, den oxidativen Abbau von SH zu OH, mit gegebenenfalls sich anschließender Alkylierung oder Veresterung zu den Gruppen $OR^{3a}$, $OC(O)NR^4R^5$ oder $OC(O)R^8$.

Die Herstellung der Pyrimidinonverbindungen II, worin W = N und $R^1$ für $NR^4R^5$ steht, kann beispielsweise in Analogie zu obigem Schema 5 erfolgen. Die Herstellung ist in Schema 7 skizziert.

Schema 7:

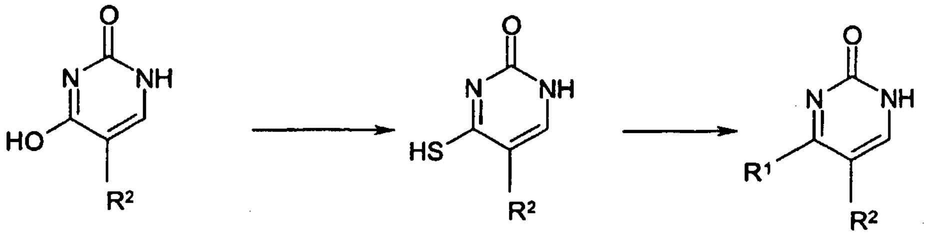

II: {$R^2$ = H, $CH_3$}  II: {$R^2$ = H, $CH_3$}  II: {$R^1$ = $NR^4R^5$; $R^2$ = H, $CH_3$}

Die Herstellung von Tautomeren der Formel I.A kann in analoger Weise wie die hier beschriebene Herstellung der Verbindung LA erfolgen. Beispielsweise kann man die tautomeren Derivate I.A-a

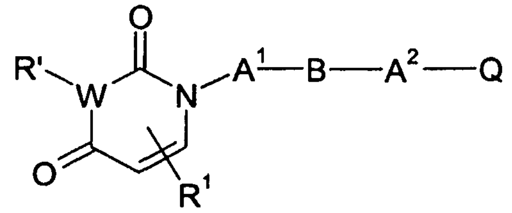

(I.A-a)

worin R' für H oder $C_1$-$C_4$-Alkyl steht, in Analogie zu dem in Schema 1 gezeigten Syntheseweg herstellen.

Darüber hinaus sind Tautomere der Formel I.A-a mit R' = Methyl auch durch mehrstündige Behandlung der H-analogen Verbindung mit einem Überschuss von z.B. 5 molaren Äquivalenten MeI/KOH in DMSO bei Raumtemperatur zugänglich, wie in Schema 8 dargestellt.

## Schema 8:

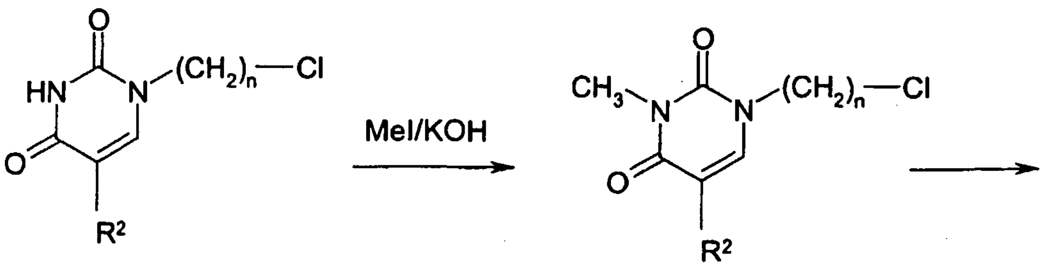


(I.A-a)

{$R^2$ = H, $CH_3$ ; n = 3-6} {$R^2$ = H, $CH_3$ ; n = 3-6}

Außerdem kann man die Verbindung I.A in ihre Tautomere I.A-b

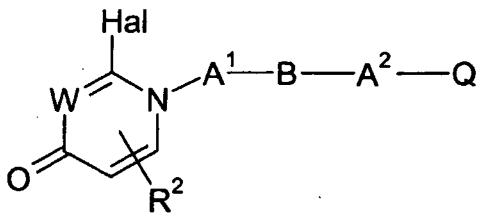

(I.A-b),

worin Hal für Halogen steht, umwandeln, indem man sie mit einem geeigneten Halogenierungsmittel wie $PCl_3$ oder $POCl_3$ behandelt.

Sofern nichts anderes angegeben wird, erfolgen die oben beschriebenen Umsetzungen im Allgemeinen in einem Lösungsmittel bei Temperaturen zwischen Raumtemperatur und der Siedetemperatur des verwendeten Lösungsmittels. Brauchbare Lösungsmittel sind beispielsweise Ether, wie Diethylether, Diisopropylether, Methyl-tert.-butylether oder Tetrahydrofuran, Dimethylformamid, Dimethylsulfoxid, Dimethoxyethan, Toluol, Xylol, Acetonitril, Ketone, wie Aceton oder Methylethylketon, oder Alkohole, wie Methanol, Ethanol oder Butanol.

Die zur Umsetzung erforderliche Aktivierungsenergie kann mittels Mikrowellen in die Reaktionsmischung eingetragen werden (zur Umsetzung unter Einsatz von Mikrowellen, siehe Tetrahedron 2001, 57, S. 9199 ff, S. 9225 ff sowie allgemein "Microwaves in Organic Synthesis", André Loupy (Hrsg.), Wiley-VCH 2002).

Gewünschtenfalls arbeitet man in Gegenwart einer Base zur Neutralisation von bei den Umsetzungen freiwerdenden Protonen. Geeignete Basen umfassen anorganische Basen, wie Natrium- oder Kaliumcarbonat, Natrium- oder Kaliumhydrogencarbonat, weiterhin Alkoholate wie Natriummethylat, Natriumethylat, Alkalimetallhydride wie Natriumhydrid, metallorganische Verbindungen, wie Butyllithium- oder Alkylmagnesium-Verbindungen, oder organische Stickstoffbasen, wie Triethylamin oder Pyridin. Letztere können gleichzeitig als Lösungsmittel dienen.

Die Isolierung des Rohprodukts erfolgt in üblicher Weise, beispielsweise durch Filtration, Abdestillieren des Lösungsmittels oder Extraktion aus dem Reaktionsgemisch etc. Die Reinigung der erhaltenen Verbindungen kann in üblicher Weise erfolgen, beispielsweise durch Umkristallisieren aus einem Lösungsmittel, Chromatographie oder durch Überführen in eine Säureadditionssalz.

Die Säureadditionssalze werden in üblicher Weise durch Mischen der freien Base mit der entsprechenden Säure, gegebenenfalls in Lösung in einem organischen Lösungsmittel, beispielsweise einem niedermolekularen Alkohol, wie Methanol, Ethanol oder Propanol, einem Ether, wie Methyl-tert-butylether, Diisopropylether, einem Keton, wie Aceton oder Methylethylketon oder einem Ester, wie Essigsäureethylester, hergestellt.

Bei den erfindungsgemäßen Verbindungen der Formel I.A handelt es sich um hochselektive Dopamin-$D_3$-Rezeptor-Liganden, die auf Grund ihrer geringen Affinität gegenüber anderen Rezeptoren, insbesondere gegenüber Dopamin-$D_2$-Rezeptoren, nebenwirkungsärmer als die klassischen Neuroleptika sind, bei denen es sich um $D_2$-Rezeptor-Antagonisten handelt.

Die hohe Affinität der erfindungsgemäßen Verbindungen gegenüber $D_3$-Rezeptoren spiegelt sich in sehr geringen in vitro $K_1$-Werte von in der Regel weniger als 100 nM (nmol/l) und vor allem von weniger als 50 nM wieder. Beispielsweise können Bindungsaffinitäten zu $D_3$-Rezeptoren in Rezeptorbindungsstudien über die Verdrängung von [$^{125}$I]-Iodosulprid bestimmt werden.

Erfindungsgemäß von besonderer Bedeutung sind Verbindungen deren Selektivität $K_i(D_2)/K_i(D_3)$ vorzugsweise wenigstens 10, besser noch wenigstens 30 und besonders vorteilhaft wenigstens 50 beträgt. Rezeptorbindungsstudien an $D_1$-, $D_2$- und $D_4$-Rezeptoren können beispielsweise über die Verdrängung von [$^3$H]SCH23390, [$^{125}$I]Iodosulprid bzw. [$^{125}$I]Spiperon vorgenommen werden.

Die Verbindungen sind aufgrund ihres Bindungsprofils zur Behandlung von Erkrankungen brauchbar, die auf Dopamin-$D_3$-Liganden ansprechen, d. h. sie sind zur Behandlung von solchen Störungen bzw. Erkrankungen wirksam, bei denen eine Beeinflussung (Modulation) der Dopamin-$D_3$-Rezeptoren zur Verbesserung des Krankheitsbilds oder zum Ausheilen der Krankheit führt. Derartige Erkrankungen sind z. B. Störungen oder Erkrankungen des zentralen Nervensystems.

Unter Störungen bzw. Erkrankungen des zentralen Nervensystems versteht man Störungen, die Rückenmark und vor allem das Gehirn betreffen. Der Begriff "Störung" im erfindungsgemäßen Sinne bezeichnet Anomalien, die in der Regel als krankhafte Zustände bzw. Funktionen angesehen werden und sich in Form bestimmter Anzeichen, Symptome und/oder Fehlfunktionen zu erkennen geben können. Die erfindungsgemäße Behandlung kann auf einzelne Störungen, d. h. Anomalien bzw. krankhafte Zustände gerichtet sein, es können aber auch mehrere, gegebenenfalls ursächlich miteinander verbundene Anomalien zu Mustern, d.h. Syndromen, zusammengefaßt sein, die erfindungsgemäß behandelt werden können.

Zu den erfindungsgemäß behandelbaren Störungen gehören vor allem psychiatrische und neurologische Störungen. Hierzu zählen insbesondere organische Störungen, symptomatische Störungen eingeschlossen, wie Psychosen vom akuten exogenen Reaktionstyp oder Begleit-Psychosen organischer bzw. exogener Ursache, z. B. bei Stoffwechselstörungen, Infektionen und Endokrinopathien; endogene Psychosen, wie Schizophrenie sowie schizotype und wahnhafte Störungen; affektive Störungen, wie Depressionen, Manie bzw. manisch-depressive Zustände; sowie Mischformen der zuvor geschilderten Störungen; neurotische und somatoforme Störungen sowie Störungen bei Belastung; dissoziative Störungen, z.B. Bewußtseinsausfälle, -eintrübungen und -spaltungen und Persönlichkeitsstörungen; Störungen von Aufmerksamkeit und Wach/Schlafverhalten, wie Verhaltensstörungen und emotionale Störungen, deren Beginn in der Kindheit und Jugend liegt, z.B. Hyperaktivität bei Kindern, intellektuelle Defizite, insbesondere Aufmerksamkeitsstörungen (attention deficit disorders), Gedächtnis- und kognitive Störungen, z.B. Lern- und Gedächtnisschwäche (impaired cognitive function), Demenz, Narkolepsie und Schlafstörungen, z.B. restless legs syndrome; Entwicklungsstörungen; Angstzustände; Delirium; Störungen des Sexuallebens, z.B. Impotenz des Mannes; Eßstörungen, z.B. Anorexie oder Bulimie; Sucht; und weitere nicht näher bezeichnete psychiatrische Störungen.

Zu den erfindungsgemäß behandelbaren Störungen gehören auch Parkinson und Epilepsie und insbesondere die damit in Zusammenhang stehenden affektiven Störungen. Zu Suchterkrankungen gehören die durch den Missbrauch von psychotropen Substanzen, wie Arzneimittel oder Drogen, verursachte psychische Störungen und Verhaltensstörungen sowie andere Suchterkrankungen, wie beispielsweise die Spielsucht (Impulse Control Disorders not elsewhere classified). Suchterzeugende Substanzen sind beispielsweise: Opioide (z. B. Morphin, Heroin, Codein); Kokain; Nikotin; Alkohol; Substanzen, die mit dem GABA-Chlorid-Kanal-Komplex interagieren, Sedativa, Hypnotika oder Tranquilizer, beispielsweise Benzodiazepine; LSD; Cannabinoide; psychomotorische Stimulanzien, wie 3,4-Methylendioxy-N-methylamphetamin (Ecstasy); Amphetamin und amphetaminartigen Substanzen wie Methylphenidat oder sonstige Stimulanzien einschließlich Koffein. Als suchterzeugende Substanzen kommen insbesondere Opioide, Kokain, Amphetamin oder amphetaminartige Substanzen, Nikotin und Alkohol in Betracht.

Im Hinblick auf die Behandlung von Suchterkrankungen sind unter den erfindungsgemäßen Verbindungen der Formel I solche besonders bevorzugt, die selbst keine psychotrope Wirkung besitzen. Dies kann im Test auch an Ratten beobachtet werden, die nach Verabreichung erfindungsgemäß brauchbarer Verbindungen die Selbstverabreichung von

psychotropen Substanzen, beispielsweise Kokain, drosseln.

Gemäß einem weiteren Aspekt der vorliegenden Erfindung sind die erfindungsgemäßen Verbindungen zur Behandlung von Störungen geeignet, deren Ursachen zumindest zum Teil auf eine anomale Aktivität von Dopamin-$D_3$-Rezeptoren zurückzuführen sind.

Gemäß einem anderen Aspekt der vorliegenden Erfindung richtet sich die Behandlung vor allem auf diejenigen Störungen, die sich über eine Bindung von vorzugsweise exogen zugesetzten Bindungspartnern (Liganden) an Dopamin-$D_3$-Rezeptoren im Sinne einer zweckmäßigen medizinischen Behandlung beeinflussen lassen.

Die mit den erfindungsgemäßen Verbindungen behandelbaren Erkrankungen sind häufig gekennzeichnet durch eine progressive Entwicklung, d.h. die vorstehend beschriebenen Zustände verändern sich im Laufe der Zeit, in der Regel nimmt der Schweregrad zu und gegebenenfalls können Zustände ineinander übergehen oder weitere Zustände zu bereits bestehenden Zuständen hinzutreten.

Durch die erfindungsgemäßen Verbindungen lassen sich eine Vielzahl von Anzeichen, Symptomen und/oder Fehlfunktionen behandeln, die mit den Störungen des zentralen Nervensystems und insbesondere den vorstehend genannten Zuständen zusammenhängen. Hierzu gehören beispielsweise ein gestörter Realitätsbezug, mangelnde Einsicht und Fähigkeit, üblichen sozialen Normen bzw. Lebensanforderungen zu genügen, Wesensveränderungen, Veränderungen der Einzeltriebe, wie Hunger, Schlaf, Durst, etc., und der Stimmungslage, Störungen der Merk- und Kombinationsfähigkeit, Persönlichkeitsveränderungen, insbesondere Affektlabilität, Halluzinationen, Ich-Störungen, Zerfahrenheit, Ambivalenz, Autismus, Depersonalisation bzw. Sinnestäuschungen, Wahnideen, skandierende Sprache, fehlende Mitbewegung, kleinschrittiger Gang, Beugehaltung von Rumpf und Gliedern, Tremor, Mimikarmut, monotone Sprache, Depressionen, Apathie, erschwerte Spontanität und Entschlusskraft, verarmte Assoziationsfähigkeit, Angst, nervöse Unruhe, Stottern, soziale Phobie, Panikstörungen, Entzugssyndrome bei Abhängigkeit, maniforme Syndrome, Erregungs- und Verwirrtheitszustände, Dysphorie, dyskinetische Syndrome und Tic-Störungen, z.B. Chorea-Huntington, Gilles-de-la-Tourette-Syndrom, Schwindelsyndrome, z.B. peripherer Lage-, Dreh- und Schwankschwindel, Melancholie, Hysterie, Hypochondrie und ähnliches. Eine Behandlung im erfindungsgemäßen Sinne umfasst nicht nur die Behandlung akuter oder chronischer Anzeichen, Symptome und/oder Fehlfunktionen sondern auch eine vorbeugende Behandlung (Prophylaxe) insbesondere als Rezidiv- oder Phasen-Prophylaxe. Die Behandlung kann symptomatisch, beispielsweise als Symptomsuppression ausgerichtet sein. Sie kann kurzzeitig erfolgen, mittelfristig ausgerichtet sein, oder es kann sich auch um eine Langzeitbehandlung, beispielsweise im Rahmen einer Erhaltungstherapie, handeln.

Vorzugsweise eignen sich die erfindungsgemäßen Verbindungen zur Behandlung von Erkrankungen des zentralen Nervensystems, insbesondere zur Behandlung von affektiven Störungen; neurotischen, Belastungs- und somatoformen Störungen und Psychosen und speziell zur Behandlung der Schizophrenie und der Depression. Aufgrund ihrer hohen Selektivität bezüglich des $D_3$-Rezeptors sind die erfindungsgemäßen Verbindungen I auch zur Behandlung von Nierenfunktionsstörungen, insbesondere von Nierenfunktionsstörungen, die durch Diabetes-Mellitus hervorgerufen wird (siehe WO 00/67847).

Die erfindungsgemäße Verwendung der beschriebenen Verbindungen beinhaltet im Rahmen der Behandlung ein Verfahren. Dabei wird dem zu behandelnden Individuum, vorzugsweise einem Säuger, insbesondere einem Menschen, Nutz- oder Haustier, eine wirksame Menge eines oder mehrerer Verbindungen, in der Regel der pharmazeutischen und tierarzneilichen Praxis entsprechend formuliert, verabreicht. Ob eine solche Behandlung angezeigt ist und in welcher Form sie zu erfolgen hat, hängt vom Einzelfall ab und unterliegt einer medizinischen Beurteilung (Diagnose), die vorhandene Anzeichen, Symptome und/oder Fehlfunktionen, Risiken, bestimmte Anzeichen, Symptome und/oder Fehlfunktionen zu entwickeln, und weitere Faktoren miteinbezieht. Die Behandlung erfolgt in der Regel durch einmalige oder mehrmalige tägliche Verabfolgung, gegebenenfalls zusammen oder im Wechsel mit anderen Wirkstoffen oder wirkstoffhaltigen Präparaten, so dass einem zu behandelnden Individuum eine Tagesdosis von vorzugsweise etwa 0,1 bis 1000 mg/kg Körpergewicht bei oraler Gabe bzw. von etwa 0,1 bis 100 mg/kg Körpergewicht bei parenteraler Gabe zugeführt wird. Die Erfindung betrifft auch die Herstellung pharmazeutischer Mittel zur Behandlung eines Individuums, vorzugsweise eines Säugers, insbesondere eines Menschen, Nutz- oder Haustieres. So werden die Liganden gewöhnlich in Form von pharmazeutischen Zusammensetzungen verabreicht, die einen pharmazeutisch verträglichen Exzipienten mit wenigstens einem erfindungsgemäßen Liganden und gegebenenfalls weiteren Wirkstoffen umfassen. Diese Zusammensetzungen können beispielsweise auf oralem, rektalem, transdermalem, subkutanem, intravenösem, intramuskulärem oder intranasalem Weg verabreicht werden.

Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, Suppositorien oder vaginale Arzneiformen, halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Inhibitoren verwendet werden. Ferner können auch Liposomen oder Mikrosphären zur Anwendung kommen. Bei der Herstellung der Zusammensetzungen werden erfindungsgemäße Inhibitoren gewöhnlich mit einem Exzipienten vermischt oder verdünnt. Exzipienten können feste,

halbfeste oder flüssige Materialien sein, die als Vehikel, Träger oder Medium für den Wirkstoff dienen.

Geeignete Exzipienten sind in den einschlägigen Arzneimonographien gelistet. Ferner können die Formulierungen pharmazeutisch akzeptable Träger oder übliche Hilfsstoffe, wie Gleitmittel; Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Die nachfolgenden Beispiele dienen zur Erläuterung der Erfindung ohne sie zu begrenzen.

Die magnetischen Kernresonanzspektraleigenschaften (NMR) beziehen sich auf chemische Verschiebungen ($\delta$), ausgedrückt in Teile pro Million (ppm). Die relative Fläche für die Verschiebungen in dem $^1$H-NMR-Spektrum entspricht der Anzahl der Wasserstoffatome für einen bestimmten funktionalen Typ in dem Molekül. Die Art der Verschiebung hinsichtlich der Multiplizität ist angegeben als Singulett (s), breites Singulett (s. br.), Dublett (d), breites Dublett (d br.), Triplett (t), breites Triplett (t br.), Quartett (q), Quintett (quint.), Multiplett (m).

Herstellungsbeispiele:

Vorstufen:

a. 1-(4-Chlorbutyl)-5-methyl-1H-pyrimidin-2,4-dion

10,1 g (80,0 mmol) 4-Hydroxy-5-methylpyrimidin-2(1*H*)-on (Thymin) in 300 ml Dimethylsulfoxid (DMSO) und 11,1 g (80,0 mmol) $K_2CO_3$ wurden 1 Stunde bei Raumtemperatur gerührt. Danach tropfte man zu dem Gemisch 13,7 g (80,0 mmol) 1-Brom-4-chlorbutan und rührte das Reaktionsgemisch 5 Stunden bei Raumtemperatur nach. Man versetzte das Reaktionsgemisch mit Wasser und extrahierte anschließend mit Essigsäureethylester. Danach neutralisierte man die wässrige Phase und extrahierte mit Methylenchlorid. Nach Trocknen der organischen Phase, Abfiltrieren des Trockenmittels und Einengen des Lösungsmittels im Vakuum bis zur Trockne erhielt man 7,1 g der Titelverbindung
ESI-MS: 219,1, [M+H$^+$] = 217,1;
$^1$H-NMR (500 MHz, $CDCl_3$) $\delta$(ppm): 9,97 (1H, s.), 7,02 (1H, s.), 3,74 (2H, t), 3,55 (2H, t), 1,93 (3H, s), 2,02-1,75 (4H, m).

b. 1-(4-Chlorbutyl)-1H-pyrimidin-2,4-dion

Man stellte die Titelverbindung analog zu der in J. Am. Chem. Soc. 1993, 115, 7636 beschriebenen Vorschrift zur Herstellung von 1-(4-Brombutyl)pyrimidin-2,4(1*H*,3*H*)-dion her.

c. 1-(4-Chlorbutyl)-4-phenyl-1H-pyrimidin-2-on

c.1 2-Chlor-4-phenylpyrimidin

Zu 1,00 g (6,71 mmol) 2,4-Dichlorpyrimidin und 0,82 g (6,71 mmol) Benzolboronsäure in 29 ml Toluol und 7 ml Methanol gab man 2,78 g (20,14 mmol) $K_2CO_3$, 0,21g (0,18 mmol) Tetrakis(triphenylphosphin)Pd(O) und rührte das Reaktionsgemisch 3 Stunden bei Raumtemperatur. Nach Einengen des Reaktionsgemisches nahm man den Rückstand in Wasser/Methyl-tert.-butylether auf. Anschließend extrahierte man die wässrige Phase zweimal mit Methyl-tert.-butylether. Danach wusch man die vereinte organische Phase mit Wasser und mit einer gesättigten, wässrigen NaCl-Lösung, trocknete die organische Phase, filtrierte das Trockenmittel ab und engte ein. Man reinigte den festen, braunen Rückstand durch Flash-Chromatographie an Kieselgel (Laufmittel: Essigsäureethylester/Cyclohexan: 10:90); Ausbeute: 0,90 g.

$^1$H-NMR (400 MHz, $CDCl_3$) $\delta$ (ppm): 8,64 (1H, d), 8,10 (2H, d), 7,650 (1H, d), 7,58-7,48 (3H, m).

c.2 4-Phenylpyrimidin-2-ol

Man erhitzte 0,80 g (4,20 mmol) 2-Chlor-4-phenylpyrimidin aus c.1 in 3,20 ml konz. HCl 1 Stunde auf 100 °C. Anschließend engte man den Ansatz ein, suspendierte ihn in Methylenchlorid und engte erneut ein. Ausbeute: 0,83 g.

ESI-MS: 174,3, [M+H$^+$] = 173,2.

c.3 1-(4-Chlorbutyl)-4-phenylpyrimidin-2(1*H*)-on

Man rührte 0,84 g (4,20 mmol) 4-Phenylpyrimidin-2-ol aus Beispiel c.2 in 8,4 ml N,N-Dimethylformamid (DMF) und 0,58 g (4,20 mmol) $K_2CO_3$ 1 Stunde bei Raumtemperatur. Anschließend tropfte man 0,72 g (4,20 mmol) 1-Brom-4-chlorbutan zu, rührte das Reaktionsgemisch 12 Stunden bei Raumtemperatur, filtrierte das Reaktionsgemisch und engte ein. Danach nahm man den Rückstand in Toluol auf, engte ein, nahm erneut den Rückstand in Toluol auf und engte ein. Den erhaltenen Rückstand verrührte man mit Pentan und filtrierte. Ausbeute: 0,74 g.

$^1$H-NMR (400 MHz, $CDCl_3$) $\delta$ (ppm): 8,10 (2H, d), 7,71 (1H, d), 7,63-7,40 (3H, m), 6,82 (1H, d), 3,98 (2H, t), 3,58 (2H, t), 2,00 (2H, quint.), 1,90 (2H, quint.).

d. 1-(4-Chlorbutyl)-4-methyl-1H-pyrimidin-2-on

d.1 4-Methylpyrimidin-2(1H)-on (Analog zu Aust. J. Chem. 1968, 21, 243-55)

Zu 26,4 g (0,2 mol) 4,4-Dimethoxybutan-2-on in 40 ml Ethanol und 12,0 g (0,2 mol) Harnstoff tropfte man 20,0 ml konz. HCl. Nach kurzer Zeit entstand eine klare braune Lösung und nach weiteren 10 Minuten fiel ein gelber Niederschlag aus. Man erhitzte das Reaktionsgemisch 1,5 Stunden am Rückfluss erhitzt und ließ dann abkühlen (Eiswasserbad). Danach saugte man die ausgefallenen Kristalle ab, wusch mit Ethanol und trocknete die Kristalle bei 40 °C im Vakuum; Ausbeute: 22,0 g.

d.2 1-(4-Chlorbutyl)-4-methyl-1 H-pyrimidin-2-on

Man rührte 0,1 mol 4-Methylpyrimidin-2(1*H*)-on aus d.1, 0,1 mol 1-Brom-4-chlorbutan und 0,3 mol $K_2CO_3$ in 200 ml Dimethylsulfoxid 12 Stunden bei Raumtemperatur. Man gab das Reaktionsgemisch auf Eiswasser und extrahierte das wässrige Gemisch zweimal mit Diethylether. Die wässrige Phase wurde zweimal mit Methylenchlorid extrahiert. Man trocknete die Methylenchloridphase über $Na_2SO_4$, filtrierte das Trockenmittel ab und engte das Lösungsmittel bis zur Trockne im Vakuum ein. Man verrührte den erhaltenen festen Rückstand mit Diethylether, saugte den Niederschlag ab, wusch mit Diethylether und trocknete.

$^1$H-NMR (400 MHz, $CDCl_3$) $\delta$ (ppm): 8,46 (1H, d), 7,46 (1H, d), 3,90 (2H, t), 3,57 (2H, t), 2,11 (3H, s.), 1,95 (2H, quint.), 1,88-1,78 (2H, quint.).

e. 1-(4-Chlorbutyl)-5-fluor-1H-pyrimidin-2,4-dion

Zu einer Lösung von 1,95 g (15,0 mmol) 2,4-Dihydroxy-5-fluorpyrimidin in 50 ml Dimethylsulfoxid und 20,0 ml N,N-Dimethylformamid (DMF) tropfte man bei 0 °C 2,6 g (15,0 mmol) 1-Brom-4-chlorbutan zu. Portionsweise gab man über 1 Stunde 2,07 g (15,0 mmol) $K_2CO_3$ zu und rührte das Gemisch 1 Stunde bei 20 °C (dialkyliertes Produkt ist bereits erkennbar). Anschließend gab man zum Reaktionsgemisch Wasser und extrahierte das wässrige Gemisch zweimal mit Diethylether und zweimal mit Methylenchlorid. Man stellte die wässrige Phase mit Salzsäure auf pH 3-4 und extrahierte danach die wässrige Phase mit Methylenchlorid. Anschließend trocknete man die organische Phase, filtrierte das Trockenmittel ab und engte das Lösungsmittel bis zur Trockne im Vakuum ein; Ausbeute: 0,6 g.

Beispiel 1:

1-{4-[4-(2,4-Dichlorbenzyl)-piperazin-1-yl]-butyl}-5-methyl-1H-pyrimidin-2,4-dion

Man erhitzte 1-(4-Chlorbutyl)-5-methyl-1H-pyrimidin-2,4-dion (0,69 mmol, 0,15 g), 1-(2,4-Dichlorbenzyl)-piperazin (0,62 mmol, 0,15 g), Natriumbromid (3,46 mmol, 0,36 g) und Diisopropylethylamin (6,92 mmol, 0,89 g) in N-Methylpyrrolidinon (0,6 ml) 5 Stunden auf 120 °C. Anschließend ließ man das Reaktionsgemisch abkühlen, saugte die Suspension ab und engte das Filtrat ein. Danach nahm man den Rückstand in Essigsäureethylester auf und wusch mit gesättigter Kochsalzlösung. Man trocknete die organische Schicht, filtrierte das Trockenmittel ab und dampfte im Vakuum ein. Den erhaltenen Rückstand reinigte man durch Chromatographie an Kieselgel (Eluierungsmittel: Methyl-tert-butylether/Methanol (0-100%), wobei man 88,0 mg der Titelverbindung erhielt.

ESI-MS: $[M+H^+]$ = 425,0;
$^1$H-NMR (400 MHz, DMSO-$d_6$) $\delta$ (ppm): 11,19 (1H, s br.), 7,60-7,36 (4H, m), 3,59 (2H, t), 3,52 (2H, s), 2,41 (8H, s br.), 2,33 (2H, t), 1,74 (3H, s), 1,55 (2H, quint.), 1,38 (2H, quint.).

Anschließend wurde die freie Base in ihr Fumarsäuresalz überführt.

Beispiel 2:

1-{4-[4-(2,4-Dichlorbenzyl)-piperazin-1-yl)-butyl}-1H-pyrimidin-2,4-dion

Analog zu Beispiel 1 erhielt man aus 1-(4-Chlorbutyl)-1H-pyrimidin-2,4-dion (0,49 mmol, 0,10 g) und 1-(2,4-Dichlorbenzyl)-piperazin (0,44 mmol, 0,11g) 0,17 g der Titelverbindung.

ESI-MS: 413,05, 411,15;

$^1$H-NMR (500 MHz, DMSO-$d_6$) $\delta$ (ppm): 11,18 (1H, s br.), 7,63 (1H, d), 7,57 (1H, s), 7,48 (1H, m), 7,41 (1H, m), 5,52 (1H, d), 3,64 (2H, t), 3,55 (2H, s), 2,46 (8H, s br.), 2,38 (2H, t), 1,55 (2H, quint.), 1,41 (2H, quint.).

Anschließend wurde die freie Base in ihr Fumarsäuresalz überführt.

Beispiel 3:

1-{4-[4-(2,4-Dichlorbenzyl)-piperazin-1-yl]-butyl}-4-phenyl-1H-pyrimidin-2-on

Analog zu Beispiel 1 erhielt man durch Umsetzung von 1-(4-Chlorbutyl)-4-phenyl-1H-pyrimidin-2-on (0,76 mmol, 0,20 g) mit 1-(2,4-Dichlorbenzyl)-piperazin (0,69 mmol, 0,17 g) 0,20 g der Titelverbindung.

ESI-MS: [M+Na$^+$] = 493,1, 473,15, 471,15, 236,1.

Anschließend wurde die freie Base in ihr Fumarsäuresalz überführt.

Beispiel 4:

1-{4-[4-(2,4-Dichlorbenzyl)-piperazin-1-yl]-butyl}-4-methyl-1H-pyrimidin-2-on

Analog zu Beispiel 1 erhielt man durch Umsetzung von 1-(4-Chlorbutyl)-4-methyl-1H-pyrimidin-2-on (0,37 mmol, 75,0 mg) mit 1-(2,4-Dichlorbenzyl)-piperazin (0,36 mmol, 87,0 mg) 21,0 mg der Titelverbindung.

ESI-MS: 411,15, 409,15, 205,1.

Beispiel 5:

1-{4-[4-(2,4-Dichlorbenzyl)-piperazin-1-yl]-butyl}-5-fluor-1H-pyrimidin-2,4-dion

Analog zu Beispiel 1 erhielt man durch Umsetzung von 1-(4-Chlorbutyl)-5-fluor-1H-pyrimidin-2,4-dion (0,45 mmol, 0,10 g) mit 1-(2,4-Dichlorbenzyl)-piperazin (0,41 mmol, 0,10 mg) 22,0 mg der Titelverbindung.

ESI-MS: 431,15, 429,15, 243,1.

Anschließend wurde die freie Base in ihr Fumarsäuresalz überführt.

Beispiel 6:

1-{4-[4-(2-Fluorbenzyl)-piperazin-1-yl]-butyl}-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit 1-(2-Fluorbenzyl)-piperazin die Titelverbindung.

ESI-MS: [M+H$^+$] = 375,0.

Beispiel 7:

1-{4-[4-(2-Methoxybenzyl)-piperazin-1-yl]-butyl}-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit 1-(2-Methoxybenzyl)-piperazin die Titelverbindung.

ESI-MS: [M+Na$^+$] = 409,0, [M+H$^+$] = 387,2, 264,9, 120,9.

Beispiel 8:

1-{4-[4-(2-Chlorbenzyl)-piperazin-1-yl]-butyl}-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit

1-(2-Chlorbenzyl)-piperazin die Titelverbindung.
ESI-MS: [M+H+] = 390,9.

Beispiel 9:

5-Methyl-1-{4-[4-(2-methylbenzyl)-piperazin-1-yl]-butyl}-1H-pyrimidin-2,4-dion

Analog zu Beispiel 1 erhielt man durch Umsetzung von 1-(4-Chlorbutyl)-5-methyl-1H-pyrimidin-2,4-dion mit 1-(2-Methylbenzyl)-piperazin die Titelverbindung.
ESI-MS: 371,6, [M+H+] = 371,0.

Beispiel 10:

1-{4-[4-(3,4-Dichlorbenzyl)-piperazin-1-yl]-butyl}-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit 1-(3,4-Dichlorbenzyl)-piperazin die Titelverbindung.
ESI-MS: [M+H+] = 425,0.

Beispiel 11:

1-{4-[4-(2-Chlor-4-fluorbenzyl)-piperazin-1-yl]-butyl}-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit 1-(2-Chlor-4-fluorbenzyl)-piperazin die Titelverbindung.
ESI-MS: [M+H+] = 409,0.

Beispiel 12:

1-[4-(4-Benzylpiperazin-1-yl)-butyl]-5-methyl-1H-pyrimidin-2,4-dion

Analog zu Beispiel 1 erhielt man durch Umsetzung von 1-(4-Chlorbutyl)-5-methyl-1 H-pyrimidin-2,4-dion mit N-Benzylpiperazin die Titelverbindung.
ESI-MS: [M+H+] = 357,2.

Beispiel 13:

5-Methyl-1-{4-[4-(4-methylbenzyl)-piperazin-1-yl]-butyl}-1H-pyrimidin-2,4-dion

Analog zu Beispiel 1 erhielt man durch Umsetzung von 1-(4-Chlorbutyl)-5-methyl-1 H-pyrimidin-2,4-dion mit 1-(4-Methylbenzyl)-piperazin die Titelverbindung.
ESI-MS: [M+H+] = 371,2.

Beispiel 14:

5-Methyl-1-{4-[4-(3-methylbenzyl)-piperazin-1-yl]-butyl}-1H-pyrimidin-2,4-dion

Analog zu Beispiel 1 erhielt man durch Umsetzung von 1-(4-Chlorbutyl)-5-methyl-1H-pyrimidin-2,4-dion mit 1-(3-Methylbenzyl)-piperazin die Titelverbindung.
ESI-MS: 551,2, [M+H+] = 371,1.

Beispiel 15:

1-{4-[4-(4-Fluorbenzyl)-piperazin-1-yl]-butyl}-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit 1-(4-Fluorbenzyl)-piperazin die Titelverbindung.
ESI-MS: [M+H+] = 375,1.

Beispiel 16:

1-{4-[4-(3,4-Dimethylbenzyl)-piperazin-1-yl]-butyl}-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit 1-(3,4-Dimethylbenzyl)-piperazin die Titelverbindung.

ESI-MS: [M+H$^+$] = 385,3.

Beispiel 17:

1-{4-[4-(4-Methoxybenzyl)-piperazin-1-yl]-butyl}-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit 1-(4-Methoxybenzyl)-piperazin die Titelverbindung.

ESI-MS: [M+H$^+$] = 387,1, 120,9.

Beispiel 18:

5-Methyl-1-{4-[4-(2,4,6-trimethylbenzyl)-piperazin-1-yl]-butyl}-1H-pyrimidin-2,4-dion

Analog zu Beispiel 1 erhielt man durch Umsetzung von 1-(4-Chlorbutyl)-5-methyl-1H-pyrimidin-2,4-dion mit 1-(2,4,6-Trimethylbenzyl)-piperazin die Titelverbindung.

ESI-MS: [M+H$^+$] = 399,3, 133,0.

Beispiel 19:

1-[4-(4-Benzo[1,3]dioxol-5-ylmethyl-piperazin-1-yl)-butyl]-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit 1-(Benzo[1,3]dioxol-5-ylmethyl)-piperazin die Titelverbindung.

ESI-MS: [M+H$^+$] = 401,1, 134,9.

Beispiel 20:

5-Methyl-1-[4-(4-naphthalin-2-ylmethyl-piperazin-1-yl)-butyl]-1H-pyrimidin-2,4-dion

Analog zu Beispiel 1 erhielt man durch Umsetzung von 1-(4-Chlorbutyl)-5-methyl-1H-pyrimidin-2,4-dion mit 1-(Naphthalin-2-ylmethyl)-piperazin die Titelverbindung.

ESI-MS: [2M+H$^+$] = 813,4, 587,3, [M+H$^+$] = 407,1, 140,9.

Beispiel 21:

1-{4-[4-(2-Chlor-6-fluorbenzyl)-piperazin-1-yl]-butyl}-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit 1-(2-Chlor-6-fluorbenzyl)-piperazin die Titelverbindung.

ESI-MS: [M+H$^+$] = 409,0.

Beispiel 22:

1-{4-[4-(4-tert-Butylbenzyl)-piperazin-1-yl]-butyl}-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit 1-(4-tert-Butylbenzyl)-piperazin die Titelverbindung.

ESI-MS: 593,2, [M+H$^+$] = 413,1.

Beispiel 23:

5-Methyl-1-[4-(4-pyridin-4-ylmethyl-piperazin-1-yl)-butyl]-1H-pyrimidin-2,4-dion

Analog zu Beispiel 1 erhielt man durch Umsetzung von 1-(4-Chlorbutyl)-5-methyl-1H-pyrimidin-2,4-dion mit 1-(Pyridin-4-ylmethyl)-piperazin die Titelverbindung.

ESI-MS: [M+H$^+$] = 357,7, 130,0.

Beispiel 24:

5-Methyl-1-[4-(4-pyridin-2-ylmethyl-piperazin-1-yl)-butyl]-1H-pyrimidin-2,4-dion

Analog zu Beispiel 1 erhielt man durch Umsetzung von 1-(4-Chlorbutyl)-5-methyl-1H-pyrimidin-2,4-dion mit 1-(Pyridin-2-ylmethyl)-piperazin die Titelverbindung.

ESI-MS: [2M+H$^+$] = 715,3, [M+H$^+$] = 358,1, 130,0.

Beispiel 25:

5-Methyl-1-[4-(4-pyridin-3-ylmethyl-piperazin-1-yl)-butyl]-1H-pyrimidin-2,4-dion

Analog zu Beispiel 1 erhielt man durch Umsetzung von 1-(4-Chlorbutyl)-5-methyl-1H-pyrimidin-2,4-dion mit 1-(Pyridin-3-ylmethyl)-piperazin die Titelverbindung.

ESI-MS: [2M+H$^+$] = 715,2, [M+H$^+$] = 358,0, 264,8, 130,0.

Beispiel 26:

1-[4-(4-Benzylpiperidin-1-yl)-butyl]-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit 4-Benzylpiperidin die Titelverbindung.

ESI-MS: 536,5, [M+H$^+$] = 356,1.

Beispiel 27:

5-Methyl-1-{4-[4-(tetrahydrofuran-2-ylmethyl)-piperazin-1-yl]-butyl}-1H-pyrimidin-2,4-dion

Analog zu Beispiel 1 erhielt man durch Umsetzung von 1-(4-Chlorbutyl)-5-methyl-1H-pyrimidin-2,4-dion mit 1-(Tetrahydrofuran-2-yl-methyl)-piperazin die Titelverbindung.

ESI-MS: [M+H$^+$] = 351,1, 130,0.

Beispiel 28:

5-Methyl-1-{4-[4-(2-pyrrol-1-yl-ethyl)-piperazin-1-yl]-butyl}-1H-pyrimidin-2,4-dion

Analog zu Beispiel 1 erhielt man durch Umsetzung von 1-(4-Chlorbutyl)-5-methyl-1H-pyrimidin-2,4-dion mit 1-(2-Pyrrol-1-yl-ethyl)-piperazin die Titelverbindung.

ESI-MS: [M+H$^+$] = 360,2.

Beispiel 29:

1-{4-[4-(Furan-2-carbonyl)-piperazin-1-yl]-butyl}-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit Furan-2-yl-piperazin-1-ylmethanon die Titelverbindung.

Beispiel 30:

1-{4-[4-(2-Imidazol-1-yl-ethyl)-piperazin-1-yl]-butyl-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit 1-(2-Imidazol-1-yl-ethyl)-piperazin die Titelverbindung.

ESI-MS: [2M+H$^+$] = 721,3, [M+H$^+$] = 361,3, 293,0, 130,0.

Beispiel 31:

1-[4-(4-Cyclohexylmethyl-piperazin-1-yl)-butyl]-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit 1-(Cyclohexylmethyl)-piperazin die Titelverbindung.

ESI-MS: [M+H$^+$] = 363,3.

Beispiel 32:

5-Methyl-1-{4-[4-(tetrahydrofuran-2-carbonyl)-piperazin-1-yl]-butyl}-1H-pyrimidin-2,4-dion

Analog zu Beispiel 1 erhielt man durch Umsetzung von 1-(4-Chlorbutyl)-5-methyl-1H-pyrimidin-2,4-dion mit Piperazin-1-yl-tetrahydrofuran-2-yl-methanon die Titelverbindung.

ESI-MS: [2M+H$^+$] = 729,3, [M+H$^+$] = 365,4, 267,0, 130,0.

Beispiel 33:

5-Methyl-1-{4-[4-(2-thiophen-2-yl-ethyl)-piperazin-1-yl]-butyl}-1H-pyrimidin-2,4-dion

Analog zu Beispiel 1 erhielt man durch Umsetzung von 1-(4-Chlorbutyl)-5-methyl-1H-pyrimidin-2,4-dion mit 1-(2-Thiophen-2-yl-ethyl)-piperazin die Titelverbindung.

ESI-MS: [M+H$^+$] = 377,0.

Beispiel 34:

1-{4-[4-(2-Cyclohexylethyl)-piperazin-1-yl]-butyl}-5-methyl-1H-pyrimidin-2,4-dion

Analog zu Beispiel 1 erhielt man durch Umsetzung von 1-(4-Chlorbutyl)-5-methyl-1H-pyrimidin-2,4-dion mit 1-(2-Cyclohexyl)-piperazin die Titelverbindung.

ESI-MS: 557,5, [M+H$^+$] = 377,1.

Beispiel 35:

5-Methyl-1-{4-[4-(2-oxo-2-pyrrolidin-1-yl-ethyl)-piperazin-1-yl]-butyl}-1H-pyrimidin-2,4-dion

Analog zu Beispiel 1 erhielt man durch Umsetzung von 1-(4-Chlorbutyl)-5-methyl-1H-pyrimidin-2,4-dion mit 2-Piperazin-1-yl-1-pyrrolidin-1-yl-ethanon die Titelverbindung.

ESI-MS: [2M+H$^+$] = 755,3, [M+H$^+$] = 378,5, 130,0.

Beispiel 36:

5-Methyl-1-{4-[4-(2-oxo-2-piperidin-1-yl-ethyl)-piperazin-1-yl]-butyl}-1H-pyrimidin-2,4-dion

Analog zu Beispiel 1 erhielt man durch Umsetzung von 1-(4-Chlorbutyl)-5-methyl-1H-pyrimidin-2,4-dion mit 2-Piperazin-1-yl-1-piperidin-1-yl-ethanon die Titelverbindung.

ESI-MS: [M+H$^+$] = 392,2.

Beispiele für galenische Applikationsformen

A) Tabletten

Auf einer Tablettenpresse werden in üblicher Weise Tabletten folgender Zusammensetzung gepresst:

| | |
|---|---|
| 40 mg | Substanz des Beispiels 1 |
| 120 mg | Maisstärke |
| 13,5 mg | Gelatine |
| 45 mg | Milchzucker |
| 2,25 mg | Aerosil® (chemisch reine Kieselsäure in submikroskopisch feiner Verteilung) |
| 6,75 mg | Kartoffelstärke (als 6%iger Kleister) |

B) Dragees

| | |
|---|---|
| 20 mg | Substanz des Beispiels 1 |
| 60 mg | Kernmasse |
| 70 mg | Verzuckerungsmasse |

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

Biologische Untersuchungen - Rezeptorbindungsstudien:

Die zu testende Substanz wurde entweder in Methanol/Chremophor® (BASF-AG) oder in Dimethylsulfoxid gelöst und anschließend mit Wasser auf die gewünschte Konzentration verdünnt.

Dopamin-$D_3$-Rezeptor

Der Ansatz (0,250 ml) setzte sich zusammen aus Membranen von ~$10^6$ HEK-293 Zellen mit stabil exprimierten humanen Dopamin-$D_3$-Rezeptoren, 0,1 nM [$^{125}$I]-Jodosulprid und Inkubationspuffer (totale Bindung) oder zusätzlich Testsubstanz (Hemmkurve) oder 1μM Spiperon (unspezifische Bindung). Dreifach-Ansätze wurden durchgeführt.

Der Inkubationspuffer enthielt 50 mM Tris, 120 mM NaCl, 5 mM KCl, 2 mM $CaCl_2$, 2 mM $MgCl_2$ und 0,1% Rinderserumalbumin, 10 μM Quinolone, 0.1 % Ascorbinsäure (täglich frisch hergestellt). Der Puffer wurde mit HCl auf pH 7.4 eingestellt.

Dopamin-$D_{2L}$-Rezeptor

Der Ansatz (1 ml) setzte sich zusammen aus Membranen von ~ $10^6$ HEK-293 Zellen mit stabil exprimierten humanen Dopamin-$D_{2L}$ Rezeptoren (lange Isoform) sowie 0,01 nM [$^{125}$I]-Jodospiperon und Inkubationspuffer (totale Bindung) oder zusätzlich Testsubstanz (Hemmkurve) oder 1μM Haloperidol (unspezifische Bindung). Dreifach-Ansätze wurden durchgeführt.

Der Inkubationspuffer enthielt 50 mM Tris, 120 mM NaCl, 5 mM KCl, 2 mM $CaCl_2$, 2 mM $MgCl_2$ und 0,1% Rinderserumalbumin. Der Puffer wurde mit HCl auf pH 7.4 eingestellt.

Messung und Auswertung:

Nach Inkubation für 60 Minuten bei 25 °C wurden die Ansätze mit einem Zellsammelgerät über Whatman GF/B Glasfaserfilter unter Vakuum filtriert. Die Filter wurden mit einem Filter-Transfer-System in Szintillationsgläser überführt. Nach Zugabe von 4 ml Ultima Gold® (Packard) wurden die Proben eine Stunde geschüttelt und anschließend die Radioaktivität im Beta-Counter (Packard, Tricarb 2000 oder 2200CA) gezählt. Die cp-Werte wurden anhand einer Standard-Quenchreihe mit Hilfe des geräteeigenen Programms in dpm umgerechnet.

Die Auswertung der Hemmkurven erfolgte durch iterative nichtlineare Regressionsanalyse mit dem Statistical Analysis System (SAS), ähnlich dem von Munson und Rodbard beschriebenen Programm "LIGAND".

Die erfindungsgemäßen Verbindungen zeigen in diesen Tests sehr gute Affinitäten am $D_3$-Rezeptor (< 100 nM häufig < 50 nM) und binden selektiv an den $D_3$-Rezeptor. Die Ergebnisse der Bindungstests sind in der nachfolgenden Tabelle 3 angegeben.

Tabelle 3:

| Beispiel | $K_i$ ($D_3$) [nM] | Selektivität $D_3$ vs. $D_2L$* |
|---|---|---|
| 1 | 8,9 | 98 |
| 2 | 4,6 | 94 |
| 3 | 3,16 | 204 |
| 4 | 17,0 | 31 |
| 5 | 10,6 | 99 |
| 10 | 18,1 | 51 |
| 11 | 22,3 | 48 |
| * $K_i(D_3)/K_i(D_{2L})$ | | |

**Patentansprüche**

**1.** Substituierte N-heterocyclische Verbindungen der allgemeinen Formel (I.A)

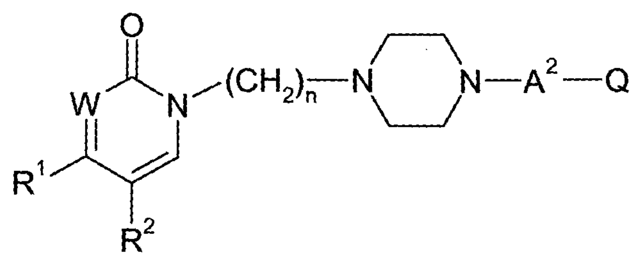

(I.A)

worin

W für CH oder N steht;
$R^1$ für Wasserstoff oder eine Gruppe $R^a$ oder $R^{a1}$ steht;
$R^2$ für Wasserstoff oder eine Gruppe $R^a$ steht;
jedes $R^a$ unabhängig unter CN, $NO_2$, Halogen, $OR^3$, $NR^4R^5$, $C(O)NR^4R^5$, $O-C(O)NR^4R^5$, $SR^6$, $SOR^6$, $SO_2R^6$, $SO_2NR^4R^5$, $COOR^7$, $O-C(O)R^8$, $COR^8$, $C_1$-$C_6$-Alkyl, $C_2$-$C_6$-Alkenyl, $C_2$-$C_6$-Alkinyl, $C_3$-$C_6$-Cycloalkyl ausgewählt sind; wobei $C_1$-$C_6$-Alkyl und $C_2$-$C_6$-Alkenyl gegebenenfalls durch 1, 2 oder 3 Reste substituiert sind, die unabhängig voneinander unter Halogen, $OR^3$, $NR^4R^5$, $C(O)NR^4R^5$, $O-C(O)NR^4R^5$, $SR^6$, $SOR^6$, $SO_2R^6$,$SO_2NR^4R^5$, $COOR^7$, $O-C(O)R^8$, $COR^8$, $C_3$-$C_6$-Cycloalkyl, Phenyl und 4- bis 6-gliedrigem Heterocyclyl mit 1, 2 oder 3 unter O, S und N ausgewählten Heteroatomen, ausgewählt sind; wobei Phenyl und Heterocyclyl ihrerseits durch ein oder zwei Reste substituiert sein können, die unabhängig voneinander ausgewählt sind unter $C_1$-$C_4$-Alkyl, $C_1$-$C_4$-Alkoxy, $NR^4R^5$, OH, CN, $C_1$-$C_2$-Fluoralkyl und Halogen;
$R^{a1}$ für einen Phenylrest oder einen 4- bis 6-gliedrigen Heterocyclylrest, der 1, 2, 3 oder 4 unabhängig voneinander unter O, S und N ausgewählte Heteroatome als Ringglieder aufweist, steht; wobei der Phenylrest und der Heterocyclylrest gegebenenfalls durch 1, 2, 3 oder 4 unabhängig voneinander ausgewählte Gruppen $R^a$ substituiert ist;
n für 4, 5 oder 6, insbesondere für 4 steht;
$A^2$ für eine 1- bis 2-gliedrige Kohlenwasserstoffkette steht, die 1 oder 2 Methylgruppen als Substituenten aufweisen kann, worin 1 Kohlenstoffatom durch eine Carbonylgruppe ersetzt sein kann;
Q für 5- oder 6-gliedriges Carbocyclyl oder Heterocyclyl mit 1, 2 oder 3 Heteroatomen, ausgewählt unter O, N

und S, steht;
wobei Carbocyclyl und Heterocyclyl jeweils vollständig gesättigt, teilweise ungesättigt oder aromatisch sein kann und 1, 2 oder 3 Substituenten aufweisen kann, die unabhängig voneinander ausgewählt sind unter
$C_1$-$C_6$-Alkyl, das gegebenenfalls ein- oder mehrfach durch OH, $C_1$-$C_4$-Alkoxy, Halogen oder Phenyl substituiert ist, das seinerseits 1, 2 oder 3 Substituenten, ausgewählt unter $C_1$-$C_4$-Alkyl, $C_1$-$C_4$-Alkoxy, $NR^4R^5$, OH, CN, $C_1$-$C_2$-Fluoralkyl und Halogen, tragen kann;
$C_2$-$C_6$-Alkenyl, $C_2$-$C_6$-Alkinyl, $C_3$-$C_6$-Cycloalkyl, $C_4$-$C_{10}$-Bicycloalkyl, $C_6$-$C_{10}$-Tricycloalkyl, wobei die fünf letztgenannten Gruppen gegebenenfalls durch Halogen oder $C_1$-$C_4$-Alkyl substituiert sein können;
Halogen, CN, $OR^3$, $NR^4R^5$, $NO_2$, $SR^6$, $SO_2R^6$, $SO_2NR^4R^5$, $COOR^7$, $COR^8$; Phenyl, 5- oder 6-gliedrigem Heterocyclyl mit 1, 2 oder 3 Heteroatomen, ausgewählt unter O, S und N, wobei Phenyl und Heterocyclyl gegebenenfalls 1 oder 2 Substituenten tragen, die unabhängig voneinander ausgewählt sind unter $C_1$-$C_4$-Alkyl, $C_1$-$C_4$-Alkoxy, $NR^4R^5$, OH, CN, $C_1$-$C_2$-Fluoralkyl und Halogen; und
wobei 2 an benachbarte C-Atome des 5- oder 6-gliedrigen Carbocyclyls oder Heterocyclyls gebundene Substituenten gemeinsam für $C_3$- oder $C_4$-Alkylen stehen können oder gemeinsam mit den C-Atomen, an die sie gebunden sind, für einen anellierten, ungesättigten 4-, 5- oder 6-gliedrigen Carbocyclus oder für einen 4-, 5- oder 6-gliedrigen Heterocyclus mit 1 oder 2 Heteroatomen, ausgewählt unter O, N und S, als Ringglieder stehen können;
$R^3$, $R^4$, $R^5$, $R^6$, $R^7$ $R^8$ unabhängig voneinander für H, $C_1$-$C_6$-Alkyl, das gegebenenfalls durch OH, $C_1$-$C_4$-Alkoxy oder Phenyl substituiert ist, das seinerseits 1, 2 oder 3 Substituenten, ausgewählt unter $C_1$-$C_4$-Alkyl, $C_1$-$C_4$-Alkoxy, $NR^4R^5$, OH, CN, $C_1$-$C_2$-Fluoralkyl oder Halogen, tragen kann, $COR^{11}$, $C_1$-$C_6$-Halogenalkyl oder Phenyl, das seinerseits 1, 2 oder 3 Substituenten, ausgewählt unter $C_1$-$C_4$-Alkyl, $C_1$-$C_4$-Alkoxy, $NR^4R^5$, OH, CN, $C_1$-$C_2$-Fluoralkyl oder Halogen, tragen kann, stehen; wobei
$R^5$ auch eine Gruppe $COR^9$ bedeuten kann; und wobei
$R^4$ mit $R^5$ auch gemeinsam mit dem Stickstoffatom, an das sie gebunden sind, einen 4-, 5- oder 6-gliedrigen, gesättigten oder ungesättigten Heterocyclus bilden können, der ein weiteres Heteroatom, ausgewählt unter O, S und $NR^{10}$ als Ringglied aufweisen kann, wobei der Heterocyclus unsubstituiert ist oder ein oder zwei $C_1$-$C_4$-Alkylgruppen trägt;
$R^9$ für Wasserstoff, $C_1$-$C_4$-Alkyl oder Phenyl, das gegebenenfalls durch 1, 2 oder 3 Reste substituiert ist, die unabhängig voneinander ausgewählt sind unter $C_1$-$C_4$-Alkyl, $C_1$-$C_4$-Alkoxy, $NR^4R^5$, CN, $C_1$-$C_2$-Fluoralkyl oder Halogen, steht;
$R^{10}$ für Wasserstoff oder $C_1$-$C_4$-Alkyl steht; und
$R^{11}$ für H, $C_1$-$C_6$-Alkyl, das gegebenenfalls durch OH, $C_1$-$C_4$-Alkoxy oder Phenyl substituiert ist, das seinerseits 1, 2 oder 3 Substituenten, ausgewählt unter $C_1$-$C_4$-Alkyl, $C_1$-$C_4$-Alkoxy, $NR^4R^5$, OH, CN, $C_1$-$C_2$-Fluoralkyl oder Halogen, tragen kann, $C_1$-$C_6$-Halogenalkyl oder Phenyl, das seinerseits 1, 2 oder 3 Substituenten, ausgewählt unter $C_1$-$C_4$-Alkyl, $C_1$-$C_4$-Alkoxy, $NR^4R^5$, OH, CN, $C_1$-$C_2$-Fluoralkyl oder Halogen, tragen kann, steht;
sowie die Tautomere der Verbindungen I.A, die physiologisch akzeptablen Salze der Verbindungen I.A und die physiologisch akzeptablen Salze der Tautomere der Verbindungen I.A.

**2.** Verbindungen nach Anspruch 1, worin W für N steht.

**3.** Verbindungen nach Anspruch 1 oder 2, worin $R^1$ ausgewählt ist unter $OR^3$, $NR^4R^5$, $SR^6$, $C_3$-$C_6$-Cycloalkyl, $C_1$-$C_6$-Alkyl, das gegebenenfalls durch 1, 2, 3 oder 4 unabhängig voneinander ausgewählte Reste OH, $C_1$-$C_4$-Alkoxy, Halogen oder Phenyl substituiert ist, Phenyl und 5- oder 6-gliedrigem aromatischem Heterocyclyl mit 1, 2 oder 3 Heteroatomen, ausgewählt unter O, S und N, wobei Phenyl und Heterocyclyl durch ein oder zwei Reste substituiert sein können, die unabhängig voneinander ausgewählt sind unter $C_1$-$C_4$-Alkyl, $C_1$-$C_4$-Alkoxy, $NR^4R^5$, CN, OH, $C_1$-$C_2$-Fluoralkyl oder Halogen.

**4.** Verbindungen nach Anspruch 3, worin $R^1$ für $OR^3$, $C_1$-$C_6$-Alkyl, $C_1$-$C_2$-Fluoralkyl, $C_3$-$C_6$-Cycloalkyl, 5- oder 6-gliedriges aromatisches Heterocyclyl mit 1 oder 2 Heteroatomen, ausgewählt unter O, S und N, oder Phenyl steht, wobei Heterocyclyl und Phenyl durch ein oder zwei Reste substituiert sein können, die unabhängig voneinander ausgewählt sind unter $C_1$-$C_4$-Alkyl, $C_1$-$C_4$-Alkoxy, $NR^4R^5$, OH, CN, $C_1$-$C_2$-Fluoralkyl oder Halogen, steht.

**5.** Verbindungen nach Anspruch 4, worin $R^1$ für OH, Methyl, iso-Propyl, tert-Butyl, $CF_3$, Cyclobutyl, Cyclohexyl, Phenyl, p-Fluorphenyl, m-Fluorphenyl, o-Fluorphenyl, p-Methylphenyl, m-Methylphenyl, o-Methylphenyl oder 2-Furyl steht.

**6.** Verbindungen nach einem der Ansprüche 1 bis 5, worin $R^2$ ausgewählt ist unter H, $C_1$-$C_4$-Alkyl, $CF_3$, Halogen oder Cyano.

7. Verbindungen nach einem der Ansprüche 1 bis 6, worin $R^1$ für OH, Methyl, iso-Propyl, tert-Butyl, $CF_3$, Cyclobutyl, Cyclohexyl, Phenyl, p-Fluorphenyl, m-Fluorphenyl, o-Fluorphenyl, p-Methylphenyl, m-Methylphenyl, o-Methylphenyl oder 2-Furyl steht, und $R^2$ für H, $C_1$-$C_4$-Alkyl, $CF_3$, Halogen oder Cyano steht.

8. Verbindungen nach einem der vorhergehenden Ansprüche, worin $A^2$ für $CH_2$, $CH_2CH_2$, CO, $CH_2CO$ oder $COCH_2$, insbesondere für $CH_2$ steht.

9. Verbindungen nach Anspruch 8, worin $A^2$ für $CH_2$ steht.

10. Verbindungen nach einem der vorhergehenden Ansprüche, worin Q für Phenyl steht, das gegebenenfalls 1, 2 oder 3 Substituenten $R^Q$ aufweist, die unabhängig voneinander ausgewählt sind unter OH, $C_1$-$C_6$-Alkyl, das gegebenenfalls vollständig oder teilweise durch Halogen substituiert ist, Halogen, Cyano, $C_1$-$C_4$-Alkoxy, $C_1$-$C_2$-Halogenalkoxy, $NR^4R^5$, $C_3$-$C_6$-Cycloalkyl, oder wobei 2 an benachbarte C-Atome des Phenyls gebundene Substituenten gemeinsam mit den C-Atomen, an die sie gebunden sind, für einen anellierten, ungesättigten 4-, 5- oder 6-gliedrigen Carbocyclus oder für einen 4-, 5- oder 6-gliedrigen Heterocyclus mit 1 oder 2 Heteroatomen, ausgewählt unter O, N und S, als Ringglieder stehen.

11. Verbindungen nach Anspruch 10, worin die Substituenten $R^Q$ unabhängig voneinander ausgewählt sind unter Methyl, Ethyl, n-Propyl, iso-Propyl, tert-Butyl, Cyclopropyl, Cyclo-butyl, Hydroxyl, Methoxy, Ethoxy, Trifluormethyl, Trifluormethoxy, Fluor, Chlor, Cyano und Dimethylamino.

12. Verbindungen nach Anspruch 10 oder 11, worin Q für Phenyl steht, das zwei Substituenten $R^Q$ aufweist.

13. Verbindungen nach Anspruch 12, worin sich die zwei Substituenten $R^Q$ in 2,3-Position am Phenylring befinden.

14. Verbindungen nach Anspruch 12, worin sich die zwei Substituenten $R^Q$ in 2,4-Position am Phenylring befinden.

15. Verbindungen nach Anspruch 12, worin sich die zwei Substituenten $R^Q$ in 3,4-Position am Phenylring befinden.

16. Verbindungen nach Anspruch 1, ausgewählt unter
1-{4-[4-(2,4-Dichlorbenzyl)-piperazin-1-yl]-butyl}-5-methyl-1H-pyrimidin-2,4-dion; 1-{4-[4-(2,4-Dichlorbenzyl)-piperazin-1-yl]-butyl}-1H-pyrimidin-2,4-dion; 1-{4-[4-(2,4-Dichlorbenzyl)-piperazin-1-yl]-butyl}-4-phenyl-1H-pyrimidin-2-on; 1-{4-[4-(2,4-Dichlorbenzyl)-piperazin-1-yl]-butyl}-4-methyl-1H-pyrimidin-2-on; 1-{4-[4-(2,4-Dichlorbenzyl)-piperazin-1-yl]-butyl}-5-fluor-1H-pyrimidin-2,4-dion; 1-{4-[4-(2-Fluorbenzyl)-piperazin-1-yl]-butyl}-5-methyl-1H-pyrimidin-2,4-dion; 1-{4-[4-(2-Methoxybenzyl)-piperazin-1-yl]-butyl}-5-methyl-1H-pyrimidin-2,4-dion; 1-{4-[4-(2-Chlorbenzyl)-piperazin-1-yl]-butyl}-5-methyl-1H-pyrimidin-2,4-dion; 5-Methyl-1-{4-[4-(2-methylbenzyl)-piperazin-1-yl]-butyl}-1H-pyrimidin-2,4-dion; 1-{4-[4-(3,4-Dichlorbenzyl)-piperazin-1-yl]-butyl}-5-methyl-1H-pyrimidin-2,4-dion; 1-{4-[4-(2-Chlor-4-fluorbenzyl)-piperazin-1-yl]-butyl}-5-methyl-1H-pyrimidin-2,4-dion; 1-[4-(4-Benzylpiperazin-1-yl)-butyl]-5-methyl-1H-pyrimidin-2,4-dion; 5-Methyl-1-{4-[4-(4-methylbenzyl)-piperazin-1-yl]-butyl}-1H-pyrimidin-2,4-dion; 5-Methyl-1-{4-[4-(3-methylbenzyl)-piperazin-1-yl]-butyl}-1H-pyrimidin-2,4-dion; 1-{4-[4-(4-Fluorbenzyl)-piperazin-1-yl]-butyl}-5-methyl-1H-pyrimidin-2,4-dion; 1-{4-[4-(3,4-Dimethylbenzyl)-piperazin-1-yl]-butyl}-5-methyl-1 H-pyrimidin-2,4-dion; 1-{4-[4-(4-Methoxybenzyl)-piperazin-1-yl]-butyl}-5-methyl-1H-pyrimidin-2,4-dion; 5-Methyl-1-{4-[4-(2,4,6-trimethylbenzyl)-piperazin-1-yl]-butyl}-1H-pyrimidin-2,4-dion; 1-[4-(4-Benzo[1,3]dioxol-5-ylmethyl-piperazin-1-yl)-butyl]-5-methyl-1H-pyrimidin-2,4-dion; 5-Methyl-1-[4-(4-naphthalin-2-ylmethyl-piperazin-1-yl)-butyl]-1 H-pyrimidin-2,4-dion; 1-{4-[4-(2-Chlor-6-fluorbenzyl)-piperazin-1-yl]-butyl}-5-methyl-1H-pyrimidin-2,4-dion; 1-{4-[4-(4-tert-Butylbenzyl)-piperazin-1-yl]-butyl}-5-methyl-1H-pyrimidin-2,4-dion; 5-Methyl-1-[4-(4-pyridin-4-ylmethyl-piperazin-1-yl)-butyl]-1 H-pyrimidin-2,4-dion; 5-Methyl-1-[4-(4-pyridin-2-ylmethyl-piperazin-1-yl)-butyl]-1 H-pyrimidin-2,4-dion; 5-Methyl-1-[4-(4-pyridin-3-ylmethyl-piperazin-1-yl)-butyl]-1H-pyrimidin-2,4-dion; 1-[4-(4-Benzylpiperidin-1-yl)-butyl]-5-methyl-1 H-pyrimidin-2,4-dion; 5-Methyl-1-{4-[4-(tetrahydrofuran-2-ylmethyl)-piperazin-1-yl]-butyl}-1H-pyrimidin-2,4-dion; 5-Methyl-1-{4-[4-(2-pyrrol-1-yl-ethyl)-piperazin-1-yl]-butyl}-1H-pyrimidin-2,4-dion; 1-{4-[4-(Furan-2-carbonyl)-piperazin-1-yl]-butyl}-5-methyl-1H-pyrimidin-2,4-dion; 1-{4-[4-(2-Imidazol-1-yl-ethyl)-piperazin-1-yl]-butyl-5-methyl-1H-pyrimidin-2,4-dion; 1-[4-(4-Cyclohexylmethyl-piperazin-1-yl)-butyl]-5-methyl-1H-pyrimidin-2,4-dion; 5-Methyl-1-{4-[4-(tetrahydrofuran-2-carbonyl)-piperazin-1-yl]-butyl}-1H-pyrimidin-2,4-dion; 5-Methyl-1-(4-[4-(2-thiophen-2-yl-ethyl)-piperazin-1-yl]-butyl}-1H-pyrimidin-2,4-dion; 1-{4-[4-(2-Cyclohexylethyl)-piperazin-1-yl]-butyl}-5-methyl-1H-pyrimidin-2,4-dion; 5-Methyl-1-{4-[4-(2-oxo-2-pyrrolidin-1-yl-ethyl)-piperazin-1-yl]-butyl}-1H-pyrimidin-2,4-dion; 5-Methyl-1-{4-[4-(2-oxo-2-piperidin-1-yl-ethyl)-piperazin-1-yl]-butyl}-1H-pyrimidin-2,4-dion;
und den Tautomeren, den physiologisch akzeptablen Salzen und den physiologisch akzeptablen Salzen der Tautomere davon.

**17.** Pharmazeutisches Mittel, enthaltend wenigstens eine Verbindung nach einem der Ansprüche 1 bis 16 und/oder deren Salz, gegebenenfalls zusammen mit physiologisch akzeptablen Trägern und/oder Hilfsstoffen.

**18.** Verwendung von Verbindungen nach einem der Ansprüche 1 bis 16 und von deren pharmakologisch akzeptablen Salzen zur Herstellung eines pharmazeutischen Mittels zur Behandlung von Erkrankungen, die auf die Beeinflussung durch Dopamin-$D_3$-Rezeptorliganden ansprechen.

**19.** Verwendung von Verbindungen nach einem der Ansprüche 1 bis 16 und von deren pharmakologisch akzeptablen Salzen zur Herstellung eines pharmazeutischen Mittels zur Behandlung von Erkrankungen des zentralen Nervensystems.

**20.** Verwendung nach Anspruch 19 zur Behandlung von Schizophrenie und/oder Depression.

## Claims

**1.** Substituted N-heterocyclic compounds of the formula (I.A)

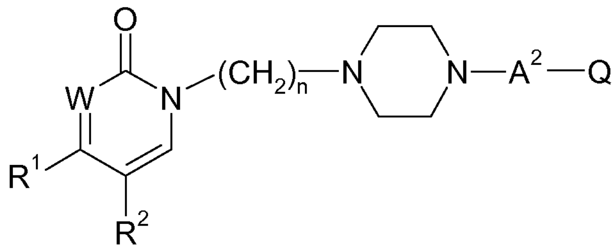

(I.A)

in which

W is CH or N;
$R^1$ is hydrogen or a group $R^a$ or $R^{a1}$;
$R^2$ is hydrogen or a group $R^a$;
each $R^a$ is selected independently from CN, $NO_2$, halogen, $OR^3$, $NR^4R^5$, $C(O)NR^4R^5$, $O-C(O)NR^4R^5$, $SR^6$, $SOR^6$, $SO_2R^6$, $SO_2NR^4R^5$, $COOR^7$, $O-C(O)R^8$, $COR^8$, $C_1$-$C_6$-alkyl, $C_2$-$C_6$-alkenyl, $C_2$-$C_6$-alkynyl, $C_3$-$C_6$-cycloalkyl; where $C_1$-$C_6$-alkyl and $C_2$-$C_6$-alkenyl are optionally substituted by 1, 2 or 3 radicals which are selected independently of one another from halogen, $OR^3$, $NR^4R^5$, $C(O)NR^4R^5$, $O-C(O)NR^4R^5$, $SR^6$, $SOR^6$, $SO_2R^6$, $SO_2NR^4R^5$, $COOR^7$, $O-C(O)R^8$, $COR^8$, $C_3$-$C_6$-cycloalkyl, phenyl and 4- to 6-membered heterocyclyl having 1, 2 or 3 heteroatoms selected from 0, S and N; where phenyl and heterocyclyl in turn may be substituted by one or two radicals which are selected independently of one another from $C_1$-$C_4$-alkyl, $C_1$-$C_4$-alkoxy, $NR^4R^5$, OH, CN, $C_1$-$C_2$-fluoroalkyl and halogen;
$R^{a1}$ is a phenyl radical or a 4- to 6-membered heterocyclyl radical which has 1, 2, 3 or 4 heteroatoms selected independently of one another from 0, S and N as ring members; where the phenyl radical and the heterocyclyl radical is optionally substituted by 1, 2, 3 or 4 groups $R^a$ selected independently of one another;
n is 4, 5, or 6, in particular 4;
$A^2$ is a 1- to 2-membered hydrocarbon chain which may have 1 or 2 methyl groups as substituents, in which 1 carbon atom may be replaced by a carbonyl group;
Q is 5- or 6-membered carbocyclyl or heterocyclyl having 1, 2 or 3 heteroatoms selected from 0, N and S; where carbocyclyl and heterocyclyl may each be completely saturated, partly unsaturated or aromatic, and may have 1, 2 or 3 substituents which are selected independently of one another from
$C_1$-$C_6$-alkyl which is optionally substituted one or more times by OH, $C_1$-$C_4$-alkoxy, halogen or phenyl which may in turn carry 1, 2 or 3 substituents selected from $C_1$-$C_4$-alkyl, $C_1$-$C_4$-alkoxy, $NR^4R^5$, OH, CN, $C_1$-$C_2$-fluoroalkyl and halogen;
$C_2$-$C_6$-alkenyl, $C_2$-$C_6$-alkynyl, $C_3$-$C_6$-cycloalkyl, $C_4$-$C_{10}$-bicycloalkyl, $C_6$-$C_{10}$-tricycloalkyl, where the last five groups mentioned may optionally be substituted by halogen or $C_1$-$C_4$-alkyl;
halogen, CN, $OR^3$, $NR^4R^5$, $NO_2$, $SR^6$, $SO_2R^6$, $SO_2NR^4R^5$, $COOR^7$, $COR^8$;

phenyl, 5- or 6-membered heterocyclyl having 1, 2 or 3 heteroatoms selected from 0, S and N, where phenyl and heterocyclyl optionally carry 1 or 2 substituents which are selected independently of one another from $C_1$-$C_4$-alkyl, $C_1$-$C_4$-alkoxy, $NR^4R^5$, OH, CN, $C_1$-$C_2$-fluoroalkyl and halogen; and
where 2 substituents bonded to adjacent C atoms of the 5- or 6-membered carbocyclyl or heterocyclyl may together be $C_3$- or $C_4$-alkylene, or together with the C atoms to which they are bonded may be a fused, unsaturated 4-, 5- or 6-membered carbocycle or a 4-, 5- or 6-membered heterocycle having 1 or 2 heteroatoms selected from 0, N and S as ring members;
$R^3$, $R^4$, $R^5$, $R^6$, $R^7$, $R^8$ are independently of one another H, $C_1$-$C_6$-alkyl which is optionally substituted by OH, $C_1$-$C_4$-alkoxy or phenyl which in turn may have 1, 2 or 3 substituents selected from $C_1$-$C_4$-alkyl, $C_1$-$C_4$-alkoxy, $NR^4R^5$, OH, CN, $C_1$-$C_2$-fluoroalkyl or halogen, or $COR^{11}$, $C_1$-$C_6$-haloalkyl or phenyl which in turn may have 1, 2 or 3 substituents selected from $C_1$-$C_4$-alkyl, $C_1$-$C_4$-alkoxy, $NR^4R^5$, OH, CN, $C_1$-$C_2$-fluoroalkyl or halogen; where $R^5$ may also be a group $COR^9$; and where
$R^4$ with $R^5$ also together with the nitrogen atom to which they are bonded may form a 4-, 5- or 6-membered, saturated or unsaturated heterocycle which may have a further heteroatom selected from 0, S and $NR^{10}$ as ring member, where the heterocycle is unsubstituted or carries one or two $C_1$-$C_4$-alkyl groups;
$R^9$ is hydrogen, $C_1$-$C_4$-alkyl or phenyl which is optionally substituted by 1, 2 or 3 radicals which are selected independently of one another from $C_1$-$C_4$-alkyl, $C_1$-$C_4$-alkoxy, $NR^4R^5$, CN, $C_1$-$C_2$-fluoroalkyl or halogen;
$R^{10}$ is hydrogen or $C_1$-$C_4$-alkyl, and
$R^{11}$ is H, $C_1$-$C_6$-alkyl which is optionally substituted by OH, $C_1$-$C_4$-alkoxy or phenyl which in turn may have 1, 2 or 3 substituents selected from $C_1$-$C_4$-alkyl, $C_1$-$C_4$-alkoxy, $NR^4R^5$, OH, CN, $C_1$-$C_2$-fluoroalkyl or halogen, or $C_1$-$C_6$-haloalkyl or phenyl which in turn may have 1, 2 or 3 substituents selected from $C_1$-$C_4$-alkyl, $C_1$-$C_4$-alkoxy, $NR^4R^5$, OH, CN, $C_1$-$C_2$-fluoroalkyl or halogen;
and the tautomers of the compounds I.A, the physiologically acceptable salts of the compounds I.A and the physiologically acceptable salts of the tautomers of the compounds I.A.

**2.** Compounds according to Claim 1, in which W is N.

**3.** Compounds according to Claim 1 or 2, in which $R^1$ is selected from $OR^3$, $NR^4R^5$, $SR^6$, $C_3$-$C_6$-cycloalkyl, $C_1$-$C_6$-alkyl which is optionally substituted by 1, 2, 3 or 4 radicals OH, $C_1$-$C_4$-alkoxy, halogen or phenyl, which are selected independently of one another, or phenyl and 5-or 6-membered aromatic heterocyclyl having 1, 2 or 3 heteroatoms selected from 0, S and N, where phenyl and heterocyclyl may be substituted by one or two radicals which are selected independently of one another from $C_1$-$C_4$-alkyl, $C_1$-$C_4$-alkoxy, $NR^4R^5$, CN, OH, $C_1$-$C_2$-fluoroalkyl or halogen.

**4.** Compounds according to Claim 3, in which $R^1$ is $OR^3$, $C_1$-$C_6$-alkyl, $C_1$-$C_2$-fluoroalkyl, $C_3$-$C_6$-cycloalkyl, 5- or 6-membered aromatic heterocyclyl having 1 or 2 heteroatoms selected from 0, S and N, or phenyl, where heterocyclyl and phenyl may be substituted by one or two radicals which are selected independently of one another from $C_1$-$C_4$-alkyl, $C_1$-$C_4$-alkoxy, $NR^4R^5$, OH, CN, $C_1$-$C_2$-fluoroalkyl or halogen.

**5.** Compounds according to Claim 4, in which $R^1$ is OH, methyl, isopropyl, tert-butyl, $CF_3$, cyclobutyl, cyclohexyl, phenyl, p-fluorophenyl, m-fluorophenyl, o-fluorophenyl, p-methylphenyl, m-methylphenyl, o-methylphenyl or 2-furyl.

**6.** Compounds according to any of Claims 1 to 5, in which $R^2$ is selected from H, $C_1$-$C_4$-alkyl, $CF_3$, halogen or cyano.

**7.** Compounds according to any of Claims 1 to 6, in which $R^1$ is OH, methyl, isopropyl, tert-butyl, $CF_3$, cyclobutyl, cyclohexyl, phenyl, p-fluorophenyl, m-fluorophenyl, o-fluorophenyl, p-methylphenyl, m-methylphenyl, o-methylphenyl or 2-furyl, and $R^2$ is H, $C_1$-$C_4$-alkyl, $CF_3$, halogen or cyano.

**8.** Compounds according to any of the preceding claims, in which is $A^2$ is $CH_2$, $CH_2CH_2$, CO, $CH_2CO$ or $COCH_2$, in particular is $CH_2$.

**9.** Compounds according to Claim 8, in which $A^2$ is $CH_2$.

**10.** Compounds according to any of the preceding claims, in which Q is phenyl which optionally has 1, 2 or 3 substituents $R^Q$ which are selected independently of one another from OH, $C_1$-$C_6$-alkyl which is optionally completely or partly substituted by halogen, or halogen, cyano, $C_1$-$C_4$-alkoxy, $C_1$-$C_2$-haloalkoxy, $NR^4R^5$, $C_3$-$C_6$-cycloalkyl, or where 2 substituents linked to adjacent C atoms of the phenyl are, together with the C atoms to which they are bonded, a fused, unsaturated 4-, 5- or 6-membered carbocycle or are a 4-, 5- or 6-membered heterocycle having 1 or 2 heteroatoms selected from 0, N and S, as ring members.

**11.** Compounds according to Claim 10, in which the substituents $R^Q$ are selected independently of one another from methyl, ethyl, n-propyl, isopropyl, tert-butyl, cyclopropyl, cyclobutyl, hydroxyl, methoxy, ethoxy, trifluoromethyl, trifluoromethoxy, fluorine, chlorine, cyano and dimethylamino.

**12.** Compounds according to Claim 10 or 11, in which Q is phenyl which has two substituents $R^Q$.

**13.** Compounds according to Claim 12, in which the two substituents $R^Q$ are located in position 2,3 on the phenyl ring.

**14.** Compounds according to Claim 12, in which the two substituents $R^Q$ are located in position 2,4 on the phenyl ring.

**15.** Compounds according to Claim 12, in which the two substituents $R^Q$ are located in position 3,4 on the phenyl ring.

**16.** Compounds according to Claim 1, selected from 1-{4-[4-(2,4-dichlorobenzyl)piperazin-1-yl]butyl}-5-methyl-1H-pyrimidine-2,4-dione; 1-{4-[4-(2,4-dichlorobenzyl)piperazin-1-yl]butyl}-1H-pyrimidine-2,4-dione; 1-{4-[4-(2,4-dichlorobenzyl)piperazin-1-yl]butyl}-4-phenyl-1H-pyrimidine-2-one; 1-{4-[4-(2,4-dichlorobenzyl)piperazin-1-yl]butyl}-4-methyl-1H-pyrimidine-2-one; 1-{4-[4-(2,4-dichlorobenzyl)piperazin-1-yl]butyl}-5-fluoro-1H-pyrimidine-2,4-dione; 1-{4-[4-(2-fluorobenzyl)piperazin-1-yl]butyl}-5-methyl-1H-pyrimidine-2,4-dione; 1-{4-[4-(2-methoxybenzyl)piperazin-1-yl]butyl}-5-methyl-1H-pyrimidine-2,4-dione; 1-{4-[4-(2-chlorobenzyl)piperazin-1-yl]butyl}-5-methyl-1H-pyrimidine-2,4-dione; 5-methyl-1-{4-[4-(2-methylbenzyl)piperazin-1-yl]butyl}-1H-pyrimidine-2,4-dione; 1-{4-[4-(3,4-dichlorobenzyl)piperazin-1-yl]butyl}-5-methyl-1H-pyrimidine-2,4-dione; 1-{4-[4-(2-chloro-4-fluorobenzyl)piperazin-1-yl]butyl}-5-methyl-1H-pyrimidine-2,4-dione; 1-[4-(4-benzylpiperazin-1-yl)butyl]-5-methyl-1H-pyrimidine-2,4-dione; 5-methyl-1-{4-[4-(4-methylbenzyl)piperazin-1-yl]butyl}-1H-pyrimidine-2,4-dione; 5-methyl-1-{4-[4-(3-methylbenzyl)piperazin-1-yl]butyl}-1H-pyrimidine-2,4-dione; 1-{4-[4-(4-fluorobenzyl)piperazin-1-yl]butyl}-5-methyl-1H-pyrimidine-2,4-dione; 1-{4-[4-(3,4-dimethylbenzyl)piperazin-1-yl]butyl}-5-methyl-1H-pyrimidine-2,4-dione; 1-{4-[4-(4-methoxybenzyl)piperazin-1-yl]butyl}-5-methyl-1H-pyrimidine-2,4-dione; 5-methyl-1-{4-[4-(2,4,6-trimethylbenzyl)piperazin-1-yl]butyl}-1H-pyrimidine-2,4-dione; 1-[4-(4-benzo[1,3]dioxol-5-ylmethylpiperazin-1-yl)-butyl]-5-methyl-1H-pyrimidine-2,4-dione; 5-methyl-1-[4-(4-naphthalin-2-ylmethylpiperazin-1-yl]butyl)-1H-pyrimidine-2,4-dione; 1-{4-[4-(2-chloro-6-fluorobenzyl)piperazin-1-yl]butyl}-5-methyl-1H-pyrimidine-2,4-dione; 1-{4-[4-(4-tert-butylbenzyl)piperazin-1-yl]butyl}-5-methyl-1H-pyrimidine-2,4-dione; 5-methyl-1-[4-(4-pyridin-4-ylmethylpiperazin-1-yl)butyl]-1H-pyrimidine-2,4-dione; 5-methyl-1-[4-(4-pyridin-2-ylmethylpiperazin-1-yl)butyl]-1H-pyrimidine-2,4-dione; 5-methyl-1-[4-(4-pyridin-3-ylmethylpiperazin-1-yl)butyl]-1H-pyrimidine-2,4-dione; 1-[4-(4-benzylpiperidin-1-yl)butyl]-5-methyl-1H-pyrimidine-2,4-dione; 5-methyl-1-{4-[4-(tetrahydrofuran-2-ylmethyl)piperazin-1-yl]butyl}-1H-pyrimidine-2,4-dione; 5-methyl-1-{4-[4-(2-pyrrol-1-ylethyl)piperazin-1-yl]butyl}-1H-pyrimidine-2,4-dione; 1-{4-[4-(furan-2-carbonyl)piperazin-1-yl]butyl}-5-methyl-1H-pyrimidine-2,4-dione; 1-{4-[4-(2-imidazol-1-ylethyl)piperazin-1-yl]butyl}-5-methyl-1H-pyrimidine-2,4-dione; 1-[4-(4-cyclohexylmethylpiperazin-1-yl)butyl]-5-methyl-1H-pyrimidine-2,4-dione; 5-methyl-1-{4-[4-(tetrahydrofuran-2-carbonyl)piperazin-1-yl]butyl}-1H-pyrimidine-2,4-dione; 5-methyl-1-{4-[4-(2-thiophen-2-ylethyl)piperazin-1-yl]butyl}-1H-pyrimidine-2,4-dione; 1-{4-[4-(2-cyclohexylethyl)piperazin-1-yl]butyl}-5-methyl-1H-pyrimidine-2,4-dione; 5-methyl-1-{4-[4-(2-oxo-2-pyrrolidin-1-ylethyl)-piperazin-1-yl]butyl}-1H-pyrimidine-2,4-dione; 5-methyl-1-{4-[4-(2-oxo-2-piperidin-1-ylethyl)-piperazin-1-yl]butyl}-1H-pyrimidine-2,4-dione; and the tautomers, the physiologically acceptable salts and the physiologically acceptable salts of the tautomers thereof.

**17.** Pharmaceutical composition comprising at least one compound according to any of Claims 1 to 16 and/or salt thereof, where appropriate together with physiologically acceptable carriers and/or excipients.

**18.** Use of compounds according to any of Claims 1 to 16 and of the pharmacologically acceptable salts thereof for producing a pharmaceutical composition for the treatment of disorders which respond to influencing by dopamine $D_3$ receptor ligands.

**19.** Use of compounds according to any of Claims 1 to 16 and of the pharmacologically acceptable salts thereof for producing a pharmaceutical composition for the treatment of disorders of the central nervous system.

**20.** Use according to Claim 19 for the treatment of schizophrenia and/or depression.

## Revendications

**1.** Composés N-hétérocycliques substitués de formule générale (I.A)

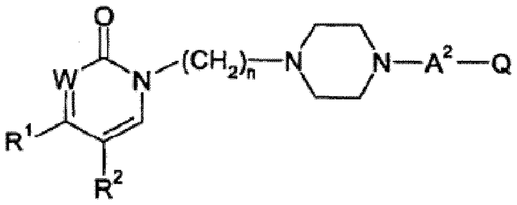

(I.A)

dans laquelle

W représente CH ou N ;
$R^1$ représente l' hydrogène ou un groupe $R^a$ ou $R^{a1}$ ;
$R^2$ représente l'hydrogène ou un groupe $R^a$ ;
chaque $R^a$ est choisi indépendamment parmi CN, $NO_2$, halogène, $OR^3$, $NR^4R^5$, $C(O)NR^4R^5$, $O\text{-}C(O)NR^4R^5$, $SR^6$, $SOR^6$, $SO_2R^6$, $SO_2NR^4R^5$, $COOR^7$, $O\text{-}C(O)R^8$, $COR^8$, alkyle en $C_1$-$C_6$, alcényle en $C_2$-$C_6$, alcynyle en $C_2$-$C_6$, cycloalkyle en $C_3$-$C_6$ ; alkyle en $C_1$-$C_6$ et alcényle en $C_2$-$C_6$ étant éventuellement substitués par 1, 2 ou 3 radicaux choisis indépendamment les uns des autres parmi halogène, $OR^3$, $NR^4R^5$, $C(O)NR^4R^5$, $O\text{-}C(O)NR^4R^5$, $SR^6$, $SOR^6$, $SO_2R^6$, $SO_2NR^4R^5$, $COOR^7$, $O\text{-}C(O)R^8$, $COR^8$, cycloalkyle en $C_3$-$C_6$, phényle et hétérocyclyle de 4 à 6 éléments contenant 1, 2 ou 3 hétéroatomes choisis parmi 0, S et N ; phényle et hétérocyclyle pouvant de leur côté être substitués par un ou deux radicaux choisis indépendamment l'un de l'autre parmi alkyle en $C_1$-$C_4$, alcoxy en $C_1$-$C_4$, $NR^4R^5$, OH, CN, fluoroalkyle en $C_1$-$C_2$ et halogène ;
$R^{a1}$ représente un radical phényle ou un radical hétérocyclyle de 4 à 6 éléments, qui comprend 1, 2, 3 ou 4 hétéroatomes choisis indépendamment les uns des autres parmi 0, S et N en tant qu'éléments de cycle ; le radical phényle et le radical hétérocyclyle étant éventuellement substitués par 1, 2, 3 ou 4 groupes $R^a$ choisis indépendamment les uns des autres ;
n représente 4, 5 ou 6, notamment 4 ;
$A^2$ représente une chaîne hydrocarbonée de 1 à 2 éléments, qui peut comprendre 1 ou 2 groupes méthyle en tant que substituants, dans laquelle 1 atome de carbone peut être remplacé par un groupe carbonyle ;
Q représente un carbocyclyle ou hétérocyclyle à 5 ou 6 éléments contenant 1, 2 ou 3 hétéroatomes choisis parmi 0, N et S ;
le carbocyclyle et l'hétérocyclyle pouvant chacun être entièrement saturés, partiellement insaturés ou aromatiques, et pouvant comprendre 1, 2 ou 3 substituants choisis indépendamment les uns des autres parmi
alkyle en $C_1$-$C_6$, qui est éventuellement substitué une ou plusieurs fois par OH, alcoxy en $C_1$-$C_4$, halogène ou phényle, qui peut de son côté porter 1, 2 ou 3 substituants choisis parmi alkyle en $C_1$-$C_4$, alcoxy en $C_1$-$C_4$, $NR^4R^5$, OH, CN, fluoroalkyle en $C_1$-$C_2$ et halogène ; alcényle en $C_2$-$C_6$, alcynyle en $C_2$-$C_6$, cycloalkyle en $C_3$-$C_6$, bicycloalkyle en $C_4$-$C_{10}$, tricycloalkyle en $C_6$-$C_{10}$, les cinq derniers groupes cités pouvant éventuellement être substitués par halogène ou alkyle en $C_1$-$C_4$;
halogène, CN, $OR^3$, $NR^4R^5$, $NO_2$, $SR^6$, $SO_2R^6$, $SO_2NR^4R^5$, $COOR^7$, $COR^8$ ;
phényle, hétérocyclyle à 5 ou 6 éléments contenant 1, 2 ou 3 hétéroatomes choisis parmi 0, S et N, phényle et hétérocyclyle portant éventuellement 1 ou 2 substituants choisis indépendamment l'un de l'autre parmi alkyle en $C_1$-$C_4$, alcoxy en $C_1$-$C_4$, $NR^4R^5$, OH, CN, fluoroalkyle en $C_1$-$C_2$ et halogène ; et
2 substituants reliés à des atomes C voisins du carbocyclyle ou de l'hétérocyclyle à 5 ou 6 éléments pouvant représenter ensemble alkylène en $C_3$ ou $C_4$, ou pouvant représenter ensemble avec les atomes C auxquels ils sont reliés un carbocycle annelé, insaturé, à 4, 5 ou 6 éléments, ou un hétérocycle à 4, 5 ou 6 éléments, contenant 1 ou 2 hétéroatomes choisis parmi 0, N et S, en tant qu'éléments de cycle ;
$R^3$, $R^4$, $R^5$, $R^6$, $R^7$, $R^8$ représentent indépendamment les uns des autres H, alkyle en $C_1$-$C_6$, qui est éventuellement substitué par OH, alcoxy en $C_1$-$C_4$ ou phényle, qui peut de son côté porter 1, 2 ou 3 substituants choisis parmi alkyle en $C_1$-$C_4$, alcoxy en $C_1$-$C_4$, $NR^4R^5$, OH, CN, fluoroalkyle en $C_1$-$C_2$ ou halogène, $COR^{11}$, halogénoalkyle en $C_1$-$C_6$ ou phényle, qui peut de son côté porter 1, 2 ou 3 substituants choisis parmi alkyle en $C_1$-$C_4$, alcoxy en $C_1$-$C_4$, $NR^4R^5$, OH, CN, fluoroalkyle en $C_1$-$C_2$ ou halogène ;
$R^5$ pouvant également signifier un groupe $COR^9$; et
$R^4$ et $R^5$ pouvant également former ensemble avec l'atome d'azote auquel ils sont reliés un hétérocycle saturé ou insaturé à 4, 5 ou 6 éléments, qui peut comprendre un hétéroatome supplémentaire choisi parmi O, S et $NR^{10}$ en tant qu'élément de cycle, l'hétérocycle étant non substitué ou portant un ou deux groupes alkyle en $C_1$-$C_4$ ;
$R^9$ représente hydrogène, alkyle en $C_1$-$C_4$ ou phényle, qui est éventuellement substitué par 1, 2 ou 3 radicaux choisis indépendamment les uns des autres parmi alkyle en $C_1$-$C_4$, alcoxy en $C_1$-$C_4$, $NR^4R^5$, CN, fluoroalkyle en $C_1$-$C_2$ ou halogène ;

$R^{10}$ représente hydrogène ou alkyle en $C_1$-$C_4$ ; et
$R^{11}$ représente H, alkyle en $C_1$-$C_6$, qui est éventuellement substitué par OH, alcoxy en $C_1$-$C_4$ ou phényle, qui peut de son côté porter 1, 2 ou 3 substituants choisis parmi alkyle en $C_1$-$C_4$, alcoxy en $C_1$-$C_4$, $NR^4R^5$, OH, CN, fluoroalkyle en $C_1$-$C_2$ ou halogène, halogénoalkyle en $C_1$-$C_6$ ou phényle, qui peut de son côté porter 1, 2 ou 3 substituants choisis parmi alkyle en $C_1$-$C_4$, alcoxy en $C_1$-$C_4$, $NR^4R^5$, OH, CN, fluoroalkyle en $C_1$-$C_2$ ou halogène ;
ainsi que les tautomères des composés I.A, les sels physiologiquement acceptables des composés I.A et les sels physiologiquement acceptables des tautomères des composés I.A.

2. Composés selon la revendication 1, dans lesquels W représente N.

3. Composés selon la revendication 1 ou 2, dans lesquels $R^1$ est choisi parmi $OR^3$, $NR^4R^5$, $SR^6$, cycloalkyle en $C_3$-$C_6$, alkyle en $C_1$-$C_6$, qui est éventuellement substitué par 1, 2, 3 ou 4 radicaux OH, alcoxy en $C_1$-$C_4$, halogène ou phényle choisis indépendamment les uns des autres, phényle et hétérocyclyle aromatique à 5 ou 6 éléments contenant 1, 2 ou 3 hétéroatomes choisis parmi 0, S et N, phényle et hétérocyclyle pouvant être substitués par un ou deux radicaux choisis indépendamment l'un de l'autre parmi alkyle en $C_1$-$C_4$, alcoxy en $C_1$-$C_4$, $NR^4R^5$, CN, OH, fluoroalkyle en $C_1$-$C_2$ ou halogène.

4. Composés selon la revendication 3, dans lesquels $R^1$ représente $OR^3$, alkyle en $C_1$-$C_6$, fluoroalkyle en $C_1$-$C_2$, cycloalkyle en $C_3$-$C_6$, hétérocyclyle aromatique à 5 ou 6 éléments contenant 1 ou 2 hétéroatomes choisis parmi 0, S et N, ou phényle, hétérocyclyle et phényle pouvant être substitués par un ou deux radicaux choisis indépendamment l'un de l'autre parmi alkyle en $C_1$-$C_4$, alcoxy en $C_1$-$C_4$, $NR^4R^5$, OH, CN, fluoroalkyle en $C_1$-$C_2$ ou halogène.

5. Composés selon la revendication 4, dans lesquels $R^1$ représente OH, méthyle, iso-propyle, tert-butyle, $CF_3$, cyclobutyle, cyclohexyle, phényle, p-fluorophényle, m-fluorophényle, o-fluorophényle, p-méthylphényle, m-méthylphényle, o-méthylphényle ou 2-furyle.

6. Composés selon l'une quelconque des revendications 1 à 5, dans lesquels $R^2$ est choisi parmi H, alkyle en $C_1$-$C_4$, $CF_3$, halogène ou cyano.

7. Composés selon l'une quelconque des revendications 1 à 6, dans lesquels $R^1$ représente OH, méthyle, iso-propyle, tert-butyle, $CF_3$, cyclobutyle, cyclohexyle, phényle, p-fluorophényle, m-fluorophényle, o-fluorophényle, p-méthylphényle, m-méthylphényle, o-méthylphényle ou 2-furyle, et $R^2$ représente H, alkyle en $C_1$-$C_4$, $CF_3$, halogène ou cyano.

8. Composés selon l'une quelconque des revendications précédentes, dans lesquels $A^2$ représente $CH_2$, $CH_2CH_2$, CO, $CH_2CO$ ou $COCH_2$, notamment $CH_2$.

9. Composés selon la revendication 8, dans lesquels $A^2$ représente $CH_2$.

10. Composés selon l'une quelconque des revendications précédentes, dans lesquels Q représente phényle, qui comprend éventuellement 1, 2 ou 3 substituants $R^Q$ choisis indépendamment les uns des autres parmi OH, alkyle en $C_1$-$C_6$, qui est éventuellement substitué en totalité ou en partie par halogène, halogène, cyano, alcoxy en $C_1$-$C_4$, halogénoalcoxy en $C_1$-$C_2$, $NR^4R^5$, cycloalkyle en $C_3$-$C_6$, ou 2 substituants reliés à des atomes C voisins du phényle pouvant représenter ensemble avec les atomes C auxquels ils sont reliés un carbocycle annelé, insaturé, à 4, 5 ou 6 éléments, ou un hétérocycle à 4, 5 ou 6 éléments, contenant 1 ou 2 hétéroatomes choisis parmi 0, N et S, en tant qu'éléments de cycle.

11. Composés selon la revendication 10, dans lesquels les substituants $R^Q$ sont choisis indépendamment les uns des autres parmi méthyle, éthyle, n-propyle, iso-propyle, tert-butyle, cyclopropyle, cyclobutyle, hydroxyle, méthoxy, éthoxy, trifluorométhyle, trifluorométhoxy, fluor, chlore, cyano et diméthylamino.

12. Composés selon la revendication 10 ou 11, dans lesquels Q représente phényle qui comprend deux substituants $R^Q$.

13. Composés selon la revendication 12, dans lesquels les deux substituants $R^Q$ se trouvent en position 2,3 sur le cycle phényle.

14. Composés selon la revendication 12, dans lesquels les deux substituants $R^Q$ se trouvent en position 2,4 sur le cycle phényle.

**15.** Composés selon la revendication 12, dans lesquels les deux substituants $R^Q$ se trouvent en position 3,4 sur le cycle phényle.

**16.** Composés selon la revendication 1, choisis parmi la 1-{4-[4-(2,4-dichlorobenzyl)-pipérazin-1-yl]-butyl}-5-méthyl-1H-pyrimidine-2,4-dione ;
la 1-{4-[4-(2,4-dichlorobenzyl)-pipérazin-1-yl]-butyl}-1H-pyrimidine-2,4-dione ;
la 1-{4-[4-(2,4-dichlorobenzyl)-pipérazin-1-yl]-butyl}-4-phényl-1H-pyrimidin-2-one ;
la 1-{4-[4-(2,4-dichlorobenzyl)-pipérazin-1-yl]-butyl}-4-méthyl-1H-pyrimidin-2-one ;
la 1-{4-[4-(2,4-dichlorobenzyl)-pipérazin-1-yl]-butyl}-5-fluoro-1H-pyrimidine-2,4-dione ;
la 1-{4-[4-(2-fluorobenzyl)-pipérazin-1-yl]-butyl}-5-méthyl-1H-pyrimidine-2,4-dione ;
la 1-{4-[4-(2-méthoxybenzyl)-pipérazin-1-yl]-butyl}-5-méthyl-1H-pyrimidine-2,4-dione ;
la 1-{4-[4-(2-chlorobenzyl)-pipérazin-1-yl]-butyl}-5-méthyl-1H-pyrimidine-2,4-dione ;
la 5-méthyl-1-{4-[4-(2-méthylbenzyl)-pipérazin-1-yl]-butyl}-1H-pyrimidine-2,4-dione ;
la 1-{4-[4-(3,4-dichlorobenzyl)-pipérazin-1-yl]-butyl}-5-méthyl-1H-pyrimidine-2,4-dione ;
la 1-{4-[4-(2-chloro-4-fluorobenzyl)-pipérazin-1-yl]-butyl}-5-méthyl-1H-pyrimidine-2,4-dione ;
la 1-[4-(4-benzylpipérazin-1-yl)-butyl]-5-méthyl-1H-pyrimidine-2,4-dione ;
la 5-méthyl-1-{4-[4-(4-méthylbenzyl)-pipérazin-1-yl]-butyl}-1H-pyrimidine-2,4-dione ;
la 5-méthyl-1-{4-[4-(3-méthylbenzyl)-pipérazin-1-yl]-butyl}-1H-pyrimidine-2,4-dione ;
la 1-{4-[4-(4-fluorobenzyl)-pipérazin-1-yl]-butyl}-5-méthyl-1H-pyrimidine-2,4-dione ;
la 1-{4-[4-(3,4-diméthylbenzyl)-pipérazin-1-yl]-butyl}-5-méthyl-1H-pyrimidine-2,4-dione ;
la 1-{4-[4-(4-méthoxybenzyl)-pipérazin-1-yl]-butyl}-5-méthyl-1H-pyrimidine-2,4-dione ;
la 5-méthyl-1-{4-[4-(2,4,6-triméthylbenzyl)-pipérazin-1-yl]-butyl}-1H-pyrimidine-2,4-dione ;
la 1-[4-(4-benzo[1,3]dioxol-5-ylméthyl-pipérazin-1-yl)-butyl]-5-méthyl-1H-pyrimidine-2,4-dione ;
la 5-méthyl-1-[4-(4-naphtalin-2-ylméthyl-pipérazin-1-yl)-butyl]-1H-pyrimidine-2,4-dione ;
la 1-{4-[4-(2-chloro-6-fluorobenzyl)-pipérazin-1-yl]-butyl}-5-méthyl-1H-pyrimidine-2,4-dione ;
la 1-{4-[4-(4-tert-butylbenzyl)-pipérazin-1-yl]-butyl}-5-méthyl-1H-pyrimidine-2,4-dione ;
la 5-méthyl-1-[4-(4-pyridin-4-ylméthyl-pipérazin-1-yl)-butyl]-1H-pyrimidine-2,4-dione ;
la 5-méthyl-1-[4-(4-pyridin-2-ylméthyl-pipérazin-1-yl)-butyl]-1H-pyrimidine-2,4-dione ;
la 5-méthyl-1-[4-(4-pyridin-3-ylméthyl-pipérazin-1-yl)-butyl]-1H-pyrimidine-2,4-dione ;
la 1-[4-(4-benzylpipéridin-1-yl)-butyl]-5-méthyl-1H-pyrimidine-2,4-dione ;
la 5-méthyl-l-{4-[4-(tétrahydrofuran-2-ylméthyl)-pipérazin-1-yl]-butyl}-1H-pyrimidine-2,4-dione ;
la 5-méthyl-1-{4-[4-(2-pyrrol-1-yl-éthyl)-pipérazin-1-yl]-butyl}-1H-pyrimidine-2,4-dione ;
la 1-{4-[4-(furane-2-carbonyl)-pipérazin-1-yl]-butyl}-5-méthyl-1H-pyrimidine-2,4-dione ;
la 1-{4-[4-(2-imidazol-1-yl-éthyl)-pipérazin-1-yl]-butyl}-5-méthyl-1H-pyrimidine-2,4-dione ;
la 1-[4-(4-cyclohexylméthyl-pipérazin-1-yl)-butyl]-5-méthyl-1H-pyrimidine-2,4-dione ;
la 5-méthyl-1-{4-[4-(tétrahydrofurane-2-carbonyl)-pipérazin-1-yl]-butyl}-1H-pyrimidine-2,4-dione ;
la 5-méthyl-1-{4-[4-(2-thiophén-2-yl-éthyl)-pipérazin-1-yl]-butyl}-1H-pyrimidine-2,4-dione ;
la 1-{4-[4-(2-cyclohexyléthyl)-pipérazin-1-yl]-butyl}-5-méthyl-1H-pyrimidine-2,4-dione ;
la 5-méthyl-1-{4-[4-(2-oxo-2-pyrrolidin-1-yl-éthyl)-pipérazin-1-yl]-butyl}-1H-pyrimidine-2,4-dione ;
la 5-méthyl-1-{4-[4-(2-oxo-2-pipéridin-1-yl-éthyl)-pipérazin-1-yl]-butyl}-1H-pyrimidine-2,4-dione ;
et les tautomères, les sels physiologiquement acceptables et les sels physiologiquement acceptables des tautomères de ceux-ci.

**17.** Agent pharmaceutique, contenant au moins un composé selon l'une quelconque des revendications 1 à 16 et/ou son sel, éventuellement conjointement avec des véhicules et/ou adjuvants physiologiquement acceptables.

**18.** Utilisation de composés selon l'une quelconque des revendications 1 à 16 et de leurs sels pharmacologiquement acceptables pour la fabrication d'un agent pharmaceutique pour le traitement de maladies réagissant à l'effet de ligands des récepteurs de dopamine $D_3$.

**19.** Utilisation de composés selon l'une quelconque des revendications 1 à 16 et de leurs sels pharmacologiquement acceptables pour la fabrication d'un agent pharmaceutique pour le traitement de maladies du système nerveux central.

**20.** Utilisation selon la revendication 19 pour le traitement de la schizophrénie et/ou de la dépression.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 03002543 A
- WO 9602519 A
- EP 04002609 W
- DE 102004027359
- WO 0067847 A


### In der Beschreibung aufgeführte Nicht-Patentliteratur


- The Dopamine D3 Receptor as a Target for Antipsychotics. **J.C. SCHWARTZ et al.** Novel Antipsychotic Drugs. Raven Press, 1992, 135-144
- **M. DOOLEY et al.** *Drugs and Aging,* 1998, vol. 12, 495-514
- **J.N. JOYCE.** The Dopamine D3 Receptor as a Therapeutic Target for Antipsychotic and Antiparkinsonian Drugs. *Pharmacology and Therapeutics,* 2001, vol. 90, 231-59
- **P. SOKOLOFF et al.** Localization and Function of the D3 Dopamine Receptor. *Arzneim. Forsch./Drug Res.,* 1992, vol. 42 (1), 224
- **P. SOKOLOFF et al.** Molecular Cloning and Characterization of a Novel Dopamine Receptor (D3) as a Target for Neuroleptics. *Nature,* 1990, vol. 347, 146
- Arzneimittelforschung. Birkhäuser Verlag, 1966, vol. 10, 224 ff
- *Chem. Pharm. Bull.,* 1987, vol. 35 (7), 2782-91
- *Bioorganical and Medicinal Chemistry Letters,* 2002, vol. 12 (8), 1149-52
- *J. Med. Chem.,* 1998, vol. 33 (5), 339-47
- *J. Med. Chem.,* 1989, vol. 32 (3), 593-7
- *JCS. Perkin Trans 1: Org. and Bioorg. Chem.,* 1998, vol. 15, 2239-42
- *Chem. Pharm. Bull.,* 1988, vol. 36 (12), 4825-33
- *Austr. J. Chem.,* 1968, vol. 221, 243-255
- *J. Med. Chem.,* 1978, vol. 21, 623-628
- *Tetrahedron Lett.,* 1986, vol. 27, 2611-2612
- *Chemiker Ztg.,* 1977, vol. 6, 305
- *J. Med. Chem.,* 1973, vol. 16 (5), 524-528
- *J. Org. Chem.,* 2002, vol. 67, 4304-4308
- *Bioorg. Med. Chem. Lett,* 2002, vol. 12, 3537-3541
- **A. SUZUKI et al.** *Chem. Rev.,* 1995, vol. 95, 2457-2483
- *J. Org. Chem.,* 2001, vol. 66 (21), 7124-7128
- *Acta Chem. Scand. B,* 1984, vol. 38, 505-508
- *Helv. Chim. Acta,* 2002, vol. 85, 2926-2929
- *Aust. J. Chem.,* 1968, vol. 21, 243-55
- *Tetrahedron,* 1997, vol. 53, 14437-50
- *J. Med. Chem.,* 1984, vol. 27, 1470-80
- *Tetrahedron,* 2001, vol. 57, 9199-9225
- Microwaves in Organic Synthesis. Wiley-VCH, 2002
- Lexikon der Hilfsstoffe für Pharmazie. **FIEDLER, H.P.** Kosmetik und angrenzende Gebiete. ECV-Editio-Kantor-Verlag, 1996
- *J. Am. Chem. Soc.,* 1993, vol. 115, 7636